Europäisches Patentamt

European Patent Office

Office européen des brevets

(19)

(11) Publication number: **0 294 659 B1**

# EUROPEAN PATENT SPECIFICATION

(12)

(45) Date of publication of patent specification: **22.12.93**

(51) Int. Cl.5: **C12Q 1/70**, C12Q 1/68, C12N 15/37

(21) Application number: **88108418.0**

(22) Date of filing: **26.05.88**

The file contains technical information submitted after the application was filed and not included in this specification

(54) **Human papillomavirus nucleic acid hybridization probes and methods for employing the same.**

(30) Priority: **26.05.87 US 53776**
**09.06.87 US 59897**
**09.06.87 US 59987**
**12.06.87 US 60883**
**30.10.87 US 114985**

(43) Date of publication of application:
**14.12.88 Bulletin 88/50**

(45) Publication of the grant of the patent:
**22.12.93 Bulletin 93/51**

(84) Designated Contracting States:
**AT BE CH DE ES FR GB GR IT LI LU NL SE**

(56) References cited:
**EP-A- 0 192 001**

**VIRAL ETIOLOGY OF CERVICAL CANCER CONFERENCE, Cold Spring Harbor, New York, NY (US), 14-17 April 1985, vol. 0, no. 0; A.T. LORINCZ et al., pp. 225-238&NUM;**

**NATURE, vol. 321, 15 May 1986; S. BEAUDENON et al., pp. 246-249&NUM;**

(73) Proprietor: **Digene Diagnostics, Inc.**
**2301-B Broadbirch Drive**
**Silver Spring, Maryland 20904(US)**

(72) Inventor: **Lorincz, Attila T.**
**19409 Poinsetta Court**
**Gaitherburg, MD 20879(US)**

(74) Representative: **Hansen, Bernd, Dr.rer.nat. et al**
**Hoffmann, Eitle & Partner**
**Patentanwälte**
**Postfach 81 04 20**
**D-81904 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

JOURNAL OF VIROLOGY, vol. 58, no. 1, April 1986, American Society for Microbiology; A.T. LORINCZ et al., pp. 225-229&NUM;

VIROLOGY, vol. 159, 1987, Academic Press Inc.; A.T. LORINCZ et al., pp. 187-190&NUM;

NATURE, vol. 321, 1986; pp. 246-249&NUM;

OBSTETRICS & GYNECOLOGY, vol. 79, no. 3, 1992; pp. 328-337&NUM;

OFFICIAL JOURNAL 1990; pp. 250-259&NUM;

**Description**

FIELD OF THE INVENTION

The present invention relates to nucleic acid hybridization probes for human papillomavirus types and particularly for human papillomavirus type 35 (hereinafter "HPV 35"); human papillomavirus type 43 (hereinafter "HPV 43"); human papillomavirus type 44 (hereinafter "HPV 44"); human papillomavirus type 56 (hereinafter "HPV 56); and methods for employing the same.

BACKGROUND OF THE INVENTION

(A) Human Papillomavirus Types

Human papillomaviruses (hereinafter "HPV") are recognized as a cause of various epithelial lesions such as warts, condylomas and dysplasias (see Gissmann, L., Cancer Surv., 3:161 (1984); Pfister, H., Biochem. Pharmacol., 99:111 (1983); Durst, M. et al, Proc. Natl. Acad. Sci. USA, 80:3812 (1983) and Boshart, M. et al, EMBO J., 3:1151 (1984)). Dysplasias of the cervix (also known as cervical intraepithelial neoplasia (CIN)) are believed to be early events in the progression to cervical cancer; the progression proceeding from mild dysplasia (CIN I), to moderate dysplasia (CIN II), to severe dysplasia, to carcinoma in situ (collectively CIN III), to invasive cancer.

Lorincz et al describe in 'Characterization of Human Papillomaviruses in Cervical Neoplasias and Their Detection in Routine Clinical Screening', published in Viral Ethiology of Cervical Cancer Conference, 14 - 17 April 1982, Cold Spring Harbor, Vol. 0, No. 0, p.225-238, that a sequence, designated as HPV 35, was cloned from a carcinoma. The clone is described as having two non-overlapping BamHI fragments. The afore-mentioned article does not provide an enabling disclosure on how to obtain HPV 35 and does not give any indication for the use thereof.

Studies examining the association of HPV type with dysplasias of the cervix and cancer of the cervix have shown that HPV types 6, 11, 16, 18, 31 and 33 are associated with a high percentage of genital lesions (see Gissmann, L., Cancer Surv., 3:161 (1984); Pfister, H., Biochem. Pharmacol., 99:111 (1983); Durst, M. et al, Proc. Natl. Acad. Sci. USA, 80:3812 (1983); Boshart, M. et al, EMBO J., 3:1151 (1984); de Villiers, E.-M. et al, J. Virol., 40:932 (1981); Gissmann, L. et al, J. Virol., 44:393 (1982); Lorincz, A.T. et al J. Virol., 58:225 (1986) and Beaudenon, S., Nature, 321:246 (1986)).

HPVs are grouped into types based on the similarity of their DNA sequence. Two HPVs are taxonomically classified as being of the same type if their DNAs cross-hybridize to greater than 50%, as measured by hybridization in solution under moderately stringent hybridization conditions, which are defined as approximately 25°C below the melting temperature of a perfectly base-paired double-stranded DNA (conveniently written as $T_m$-25°C), followed by chromatography on hydroxyapatite to separate double-stranded DNA from single-stranded DNA (see Coggin, J.R. et al, Cancer Res., 39:545 (1979)). The melting temperature ($T_m$) of a perfectly base-paired double-stranded DNA can be accurately predicted using the following well established formula:

$$T_m = 16.6 \times \log[Na^+] + 0.41 \times \%G{:}C + 81.5 - 0.72 \times (\%)(v/v) \text{ formamide}$$

The above formula provides a convenient means to set a reference point for determining non-stringent and stringent hybridization conditions for various DNAs in solutions having varying salt and formamide concentrations without the need for empirically measuring the $T_m$ for each individual DNA in each hybridization condition.

If less than 50% of the respective HPV DNAs are able to cross-hybridize in solution under moderately stringent conditions to form fully or partially double-stranded structures, as measured and defined by the ability to bind to hydroxyapatite, then the HPV DNAs are not sufficiently related to be taxonomically classified as being of the same type. A cut-off of 50% cross-hybridization using this method is employed as the consensus criterion for the assignment of novel HPV types for nomenclature purposes. This method for measuring the degree of cross-hybridization between HPV DNAs has been historically adopted as the method to be used to determine whether two HPV DNAs represent different isolates of a common type or represent isolates of different types. The use of this criterion pre-dates the establishment of clinical criterion for determining and defining HPV types. As discussed in more detail below, the clinical criterion for determining and defining HPV types is based upon the epidemiological distribution of HPV types among genital lesions.

The above-described method of measuring the degree of cross-hybridization is based on an assessment of the extent of formation of fully or partially double-stranded DNA molecules after the hybridization reaction. However, it should be noted that conversion of 50% of the DNAs into fully or partially double-stranded DNA molecules does not imply that the nucleotide sequences of the DNAs are 50% homologous.

As discussed above, HPVs can also be grouped into types based on clinical criterion. That is, it has been observed that HPV of different types, as defined by the degree of cross-hybridization criterion described above, show distinct epidemiological distributions among genital lesions of different severities and among different geographic populations.

For example, HPV 6 and HPV 11 are principally associated with benign lesions such as exophytic condylomas and to a lesser extent with flat condylomas (see Gissmann, L. et al, Proc. Natl. Acad. Sci., USA, 80:560 (1983)). HPV 6 and HPV 11 are also detected in certain rare types of malignant epithelial tumors (see Zachow, K.R. et al, Nature, 300:771 (1982) and Rando, R.F., J. Virol., 57:353 (1986)). In contrast, HPV 16, HPV 18, HPV 31 and HPV 33 are detected with varying degrees of frequency in cervical and other anogenital cancers as well as their precursor lesions (see Durst, M. et al, Proc. Natl. Acad. Sci., USA, 80:3812 (1983), Boshart, M. et al, Embo. J., 3:115 (1984), Lorincz, A.T. et al, J. Virol., 58:225 (1986) and Beaudenon, S., Nature, 321:246 (1986)). This distribution of HPV 16, HPV 18, HPV 31 and HPV 33 is believed to reflect a greater risk of, or a more rapid progression to, cervical cancer arising from genital lesions infected with HPV 16, HPV 18, HPV 31 and HPV 33 as compared to lesions infected with HPV 6 and HPV 11. As a result, the determination of HPV types has clinical-diagnostic value, i.e., such is an important factor in the assessment of risk of cancer development in patients who exhibit evidence of HPV infection. Based on the assessed risk of cancer development, appropriate therapeutic treatments can be selected.

In addition, HPV 16 is more prevalent in Europe than in Africa (Durst, M. et al, Proc. Natl. Acad. Sci., USA, 80:3812 (1983)), whereas HPV 18 is more prevalent in Africa than in Europe (Boshart, M. et al, EMBO J., 3:1115 (1984)).

Accordingly, within the context of the present invention, two HPVs are considered to be of the same type if either (1) they meet the criterion for the degree of cross-hybridization discussed above or (2) if they show substantially the same epidemiological distribution of cross-hybridization among genital lesions and they both cross-hybridize with the same genital lesions which comprise the epidemiological distribution.

It has been found that a significant percentage of cervical cancer and genital lesions which have the potential to progress to cervical cancer contain "new" HPV types which do not correspond to any of the known HPV types. Thus, in light of the known association of specific HPV types with genital lesions which have a high risk of progression to cervical cancer, the ability to detect and group these "new" HPV types allows the risk of cervical cancer associated with these "new" HPV types to be ascertained in patients who exhibit evidence of HPV infection and who may be infected with these "new" HPV types.

(B) Cloning of HPV Types

In spite of long standing efforts in the art, it has not been possible to propagate HPV in cell culture in vitro. However, recombinant DNA cloning techniques have made it possible to isolate and purify the DNA of many HPV types such as HPV Types 6, 11, 16, 18, 31 and 33 (see Durst, M. et al, Proc. Natl. Acad. Sci. USA, 80:3812 (1983); Boshart, M. et al, EMBO J., 3:1151 (1984); de Villiers, E.-M. et al, J. Virol., 40:932 (1981); Gissmann, L. et al, J. Virol., 44:393 (1982); Lorincz, A.T. et al J. Virol., 58:225 (1986); and Beaudenon, S., Nature, 321:246 (1986)). Most of the knowledge regarding HPVs has been derived from the study of the DNA sequence in such recombinant DNAs and the use of these DNAs to prepare nucleic acid hybridization probes for detection of HPV in tissue samples.

(C) Hybridization Probes

As discussed above, HPV DNA has been employed as hybridization probes to differentiate HPV types. Two HPV DNAs of different types can be readily distinguished by hybridization under stringent hybridization conditions, which are defined as approximately 10°C below the melting temperature of a perfectly based-paired double-stranded DNA hybrid (conveniently written as $T_m$-10°C), using such hybridization probes. Similarly, an HPV DNA of one type can be readily distinguished from an HPV RNA of another type by hybridization under stringent hybridization conditions which are defined as approximately 10°C below the melting temperature of a perfectly based-paired double-stranded DNA-RNA hybrid (conveniently written as $T_m$-10°C), using such hybridization probes. Further, two HPV RNAs of different types can be readily distinguished by hybridization under stringent hybridization conditions, which are defined as approximately 10°C below the melting temperature of a perfectly based-paired double-stranded RNA-RNA hybrid (conveniently written as $T_m$-10°C), using such hybridization probes. It should be noted that HPV DNAs or RNAs which are designated as different types using the above criterion, may in fact have as much as 80% of their nucleotide sequences in common.

Furthermore, two HPV DNAs of different types are able to cross-hybridize under non-stringent hybridization conditions, which are defined as approximately 35°C or more below the melting temperature of a perfectly base-paired double-stranded DNA-DNA hybrid (conveniently written as $T_m$-35°C or more), using such hybridization probes. Similarly, an HPV DNA of one type is able to cross-hybridize with an HPV RNA of another type by hybridization under non-stringent hybridization conditions which are defined as approximately 35°C or more below the melting temperature of a perfectly based-paired double-stranded DNA-RNA hybrid (conveniently written as $T_m$-35°C or more), using such hybridization probes. Further, two HPV RNAs of different types are able to cross-hybridize under non-stringent hybridization conditions, which are defined as approximately 35°C or more below the melting temperature of a perfectly based-paired double-stranded RNA-RNA hybrid (conveniently written as $T_m$-35°C or more), using such hybridization probes (see Anderson, L.M. et al, Nucleic Acid Hybridization, pages 73-111, Eds. B.D. Hames and S.J. Higgins, I.R.L. Press, Oxford, England and Washington, D.C., USA (1985)).

The melting temperatures of DNA-DNA, DNA-RNA and RNA-RNA hybrids of the same nucleotide sequences may be different in various chemical environments. The effect of various compounds on the relative melting temperatures of these various hybrids has been studied for several agents. For example, it is well known that increasing the concentration of formamide differentially destabilizes DNA-DNA hybrids more than DNA-RNA hybrids so that at high concentrations of formamide, such as 80% (v/v), a DNA-RNA hybrid may have a significantly higher melting temperature than a DNA-DNA hybrid of the same nucleotide sequence.

As discussed above, the melting temperature of a DNA-DNA hybrid can be predicted as described in Anderson, L.M. et al, Nucleic Acid Hybridization, pages 73-111, Eds. B.D. Hames and S.J. Higgins, I.R.L. Press, Oxford, England and Washington, D.C., USA (1985). Further, the melting temperature of a DNA-DNA hybrid can be empirically determined as described in Howley, P. et al, J. Biochem., 254:4876 (1979). The melting temperature of a DNA-RNA hybrid and a RNA-RNA hybrid can also be determined by means well known in the art.

Thus, it is possible to test a tissue sample for the presence of HPV DNA or RNA in general and/or a particular HPV DNA or RNA type by nucleic acid hybridization depending upon what conditions, i.e., stringent or non-stringent, are employed for hybridization.

## SUMMARY OF THE INVENTION

Accordingly, an object of the present invention is to ascertain whether cervical cancers and genital lesions, which have the potential to progress to cervical cancer, contain a "new" HPV type(s) and if so, to clone the hypothesized "new" HPV type(s).

Another object of the present invention is to provide nucleic acid hybridization probes which are specific for HPV types in general and for the "new" HPV type(s) in particular.

Still another object of the present invention is to provide a method for detecting HPV DNA or RNA in general and the "new" HPV type(s) DNA or RNA in particular, in an unknown sample of DNA or RNA, particularly an unknown sample of DNA or RNA derived from a genital lesion, so as to determine the risk of cervical cancer development.

These and other objects of the present invention will be apparent from the detailed description of the invention provided hereinafter.

It has been found in the present invention that "new" HPV types cloned in the present invention are novel HPV types, designated HPV 35, HPV 43, HPV 44 and HPV 56.

Thus, in one embodiment, the above-described objects of the present invention have been met by a recombinant DNA of HPV 35, HPV 43, HPV 44 or HPV 56 comprising a cloning vector and substantially all of HPV 35 DNA or fragments thereof; substantially all of HPV 43 DNA or fragments thereof; substantially all of HPV 44 DNA or fragments thereof; or substantially all of HPV 56 DNA or fragments thereof, respectively.

In other embodiments, the above-described objects of the present invention have been met by essentially pure HPV 35 DNA or RNA or fragments thereof; essentially pure HPV 43 DNA or RNA or fragments thereof; essentially pure HPV 44 DNA or RNA or fragments thereof; and essentially pure HPV 56 DNA or RNA or fragments thereof, and by nucleic acid hybridization probes for HPV DNA or RNA in general and HPV 35 DNA or RNA, HPV 43 DNA or RNA, HPV 44 DNA or RNA and HPV 56 DNA or RNA, respectively, in particular which comprise the above-described DNAs or RNAs which have been labelled with a detectable marker.

In still another embodiment, the above-described objects of the present invention have been met by a method for detecting HPV DNA or RNA comprising:

(1) carrying out hybridization, under non-stringent conditions, with

(a) a member selected from the group consisting of

(i) HPV 35 DNA or fragments thereof labelled with a marker, HPV 43 DNA or fragments thereof labelled with a marker, HPV 44 DNA or fragments thereof labelled with a marker, HPV 56 DNA or fragments thereof labelled with a marker, and

(ii) HPV 35 RNA or fragments thereof labelled with a marker, HPV 43 RNA or fragments thereof labelled with a marker, HPV 44 RNA or fragments thereof labelled with a marker, HPV 56 RNA or fragments thereof labelled with a marker;

(b) an unknown sample of DNA or RNA, and

(2) assaying for the presence of cross-hybridization so as to detect HPV DNA or RNA in said sample.

In a further embodiment, the above-described objects of the present invention have been met by a method for detecting HPV 35 DNA or RNA; HPV 43 DNA or RNA; HPV 44 DNA or RNA; or HPV 56 DNA or RNA comprising:

(1) carrying out hybridization, under stringent conditions, with

(a) a member selected from the group consisting of

(i) HPV 35 DNA or fragments thereof labelled with a marker, HPV 43 DNA or fragments thereof labelled with a marker, HPV 44 DNA or fragments thereof labelled with a marker, HPV 56 DNA or fragments thereof labelled with a marker, respectively, and

(ii) HPV 35 RNA or fragments thereof labelled with a marker, HPV 43 RNA or fragments thereof labelled with a marker, HPV 44 RNA or fragments thereof labelled with a marker, HPV 56 RNA or fragments thereof labelled with a marker, respectively;

(b) an unknown sample of DNA or RNA, and

(2) assaying for the presence of cross-hybridization so as to detect HPV 35 DNA or RNA; HPV 43 DNA or RNA; HPV 44 DNA or RNA; or HPV 56 DNA or RNA, respectively, in said sample.

In a still further embodiment, the above-described objects of the present invention have been met by a method for detecting HPV 35 DNA or RNA; HPV 43 DNA or RNA; HPV 44 DNA or RNA; or HPV 56 DNA or RNA comprising:

(1) carrying out hybridization, under stringent conditions, with

(a) a first fraction of DNA or RNA derived from each genital lesion of a sampling of genital lesions, which sampling shows an epidemiological progression to cervical cancer, and

(b) a member selected from the group consisting of

(i) HPV 35 DNA or fragments thereof labelled with a marker, HPV 43 DNA or fragments thereof labelled with a marker, HPV 44 DNA or fragments thereof labelled with a marker, HPV 56 DNA or fragments thereof labelled with a marker, respectively, and

(ii) HPV 35 RNA or fragments thereof labelled with a marker, HPV 43 RNA or fragments thereof labelled with a marker, HPV 44 RNA or fragments thereof labelled with a marker, HPV 56 RNA or fragments thereof labelled with a marker, respectively;

(2) carrying out hybridization, under stringent conditions, with

(a) a second fraction of DNA or RNA derived from each genital lesion of said sampling of genital lesions, and

(b) an unknown sample of DNA derived from a genital lesion labelled with a marker;

(3) comparing the epidemiological distribution of cross-hybridization obtained in Step (1) with that obtained in Step (2) and the cross-hybridization of the DNA of each lesion which comprises said epidemiological distribution so as to detect HPV 35 DNA or RNA; HPV 43 DNA or RNA; HPV 44 DNA or RNA; HPV 56 DNA or RNA, respectively, in said sample.

## BRIEF DESCRIPTION OF THE DRAWINGS

Figure 1 illustrates the restriction nuclease map of HPV 35 DNA. The following code is used to represent the restriction enzyme sites: B1, BamHI; E5, EcoRV; H3, HindIII; P1, PstI; P2, PvuII; and SI, SphI.

Figure 2 shows regions of partial homology between HPV 6 and HPV 35 DNA as determined by nucleic acid hybridization under non-stringent hybridization conditions. The arrows connect regions which exhibit homology. The dotted arrow indicates a region which has only weak homology. The smallest BamHI to PstI fragment of HPV 6 did not hybridize to HPV 35. Each of the maps in Figure 2 is arranged so that the ends of the linear map correspond to the relative position of the HpaI site of HPV 6. The positions of the open reading frames deduced for HPV 6 are shown above the homology map.

Figure 3 graphically illustrates the distribution of HPV types in various lesions based on the data in Table 1. Since the data for HPV 31 and HPV 33 are similar, only the data for HPV 31 was included in Fig. 3

Figure 4 illustrates the restriction nuclease map of HPV 43 DNA.

Figure 5 shows regions of partial homology between HPV 6 and HPV 43 DNA as determined by nucleic acid hybridization under non-stringent hybridization conditions. The arrows connect regions which exhibit homology. Each of the maps in Figure 5 is arranged so that the ends of the linear map correspond to the relative position of the HpaI site of HPV 6. The positions of the open reading frames deduced for HPV 6 are shown above the homology map.

Figure 6 graphically illustrates the distribution of HPV types in various lesions based on the data in Table 2. Since the data for HPV 31 and HPV 33 are similar, only the data for HPV 31 was included in Fig. 6.

Figure 7 illustrates the restriction nuclease map of HPV 44 DNA.

Figure 8 shows regions of partial homology between HPV 6 and HPV 44 DNA as determined by nucleic acid hybridization under non-stringent hybridization conditions. The arrows connect regions which exhibit homology. The dotted arrows indicate regions which have only weak homology. Each of the maps in Figure 8 is arranged so that the ends of the linear map correspond to the relative position of the HpaI site of HPV 6. The positions of the open reading frames deduced for HPV 6 are shown above the homology map.

Figure 9 graphically illustrates the distribution of HPV types in various lesions based on the data in Table 3. *

Figure 10 illustrates the restriction nuclease map of HPV 56 DNA.

Figure 11 shows regions of partial homology between HPV 6 and HPV 56 DNA as determined by nucleic acid hybridization under non-stringent hybridization conditions. The arrows connect regions which exhibit homology. Each of the maps in Figure 11 is arranged so that the ends of the linear map correspond to the relative position of the HpaI site of HPV 6. The positions of the open reading frames deduced for HPV 6 are shown above the homology map.

Figure 12 graphically illustrates the distribution of HPV types in various lesions based on the data in Table 4. Since the data for HPV 31 and HPV 33 are similar, only the data for HPV 31 was included in Fig. 12.

## DETAILED DESCRIPTION OF THE INVENTION

Previously unknown HPV types have been found in the present invention, and designated HPV 35, HPV 43, HPV 44 and HPV C57. HPV 35, HPV 43, HPV 44 and HPV 56 have been cloned for the first time in the present invention, thus enabling the preparation of nucleic acid hybridization probes for the detection of HPV DNA or RNA in general and HPV 35 DNA or RNA; HPV 43 DNA or RNA; HPV 44 DNA or RNA; or HPV 56 DNA or RNA, respectively, in particular in an unknown sample of DNA or RNA, particularly an unknown sample of DNA or RNA derived from genital lesions.

HPV 35 was isolated and cloned from a cervical adenocarcinoma biopsy obtained in Washington, D.C.

HPV 43 was isolated and cloned from a vulvar tissue biopsy obtained from Michigan which exhibited only hyperkeratosis upon histopathological examination.

HPV 44 was isolated and cloned from a vulvar condyloma biopsy obtained from Michigan.

HPV 56 was isolated and cloned from a vulvar condyloma biopsy obtained from the Washington, D.C. metropolitan area.

The specific cloning vector employed in the examples provided herein to initially clone HPV 35, HPV 43, HPV 44 and HPV C57 to prepare HPV 35 clones 1A and 1B HPV 43 clones 1A and 1B, HPV 44 clone 1 and HPV 56 clones 1A and 1B was λ L47.

The HPV 35 DNA from HPV 35 clones 1A and 1B was subcloned in pBR322 (ATCC No. 37017) to prepare HPV 35 clones 2A and 2B. HPV 35 clones 2A and 2B have been deposited at the American Type Culture Collection under ATCC Nos. 40330 and 40331, respectively.

The HPV 43 DNA from HPV 43 clones 1A and 1B was subcloned in pT713 (GIBCO/BRL, Gaithersburg, MD) to prepare HPV 43 clones 2A and 2B. HPV 43 clones 2A and 2B have been deposited at the American Type Culture Collection under ATCC Nos. 40338 and 40339, respectively.

The HPV 44 DNA from HPV 44 clone 1 was subcloned in pT713 (GIBCO/BRL, Gaithersburg, MD) to prepare HPV 44 clone 2. HPV 44 clone 2 has been deposited at the American Type Culture Collection under ATCC No. 40353.

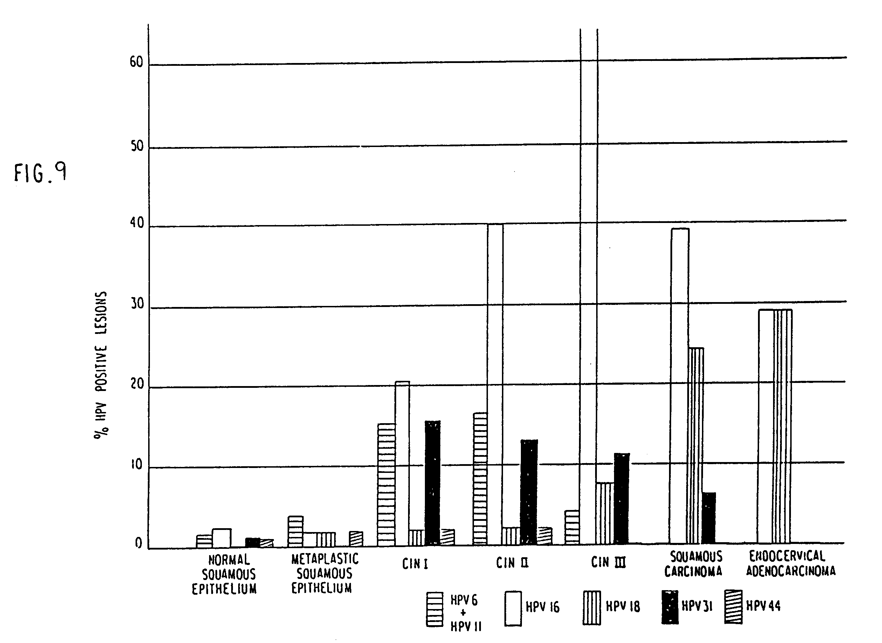

* Since the data for HPV 31 and HPV 33 are similar, only the data for HPV 31 was included in Fig. 9

7

EP 0 294 659 B1

The HPV 56 DNA from HPV 56 clones 1A and 1B was subcloned in pT713 (GIBCO/BRL, Gaithersburg, MD) to prepare HPV 56 clones 2A and 2B. HPV C57 clones 2A and 2B have been deposited at the American Type Culture Collection under ATCC No. 40341 and 40379, respectively.

HPV 35 DNA in its entirety can be excised from HPV 35 clones 2A and 2B using BamHI restriction endonuclease and subcloned in any well known procaryotic and eucaryotic cloning vectors.

HPV 43 DNA in its entirety can be excised from HPV 43 clone 2A using HindIII restriction endonuclease and from clone 2B using BamHI restriction endonuclease, and subcloned in any well known procaryotic and eucaryotic cloning vectors.

HPV 44 DNA in its entirety can be excised from HPV 44 clone 2 using BamHI restriction endonuclease and subcloned in any well known procaryotic and eucaryotic cloning vectors.

HPV 56 DNA in its entirety can be excised from HPV 56 clone 2A using EcoRI restriction endonuclease and from clone 2B using BamHI restriction endonuclease, and subcloned in any well known procaryotic and eucaryotic cloning vectors.

The particular cloning vector employed for subcloning HPV 35, HPV 43, HPV 44 or HPV 56 is not critical and can be any known procaryotic cloning vector such as pUC11, λ derived vectors such as λ charon or M13 derived bacteriophages (see Maniatis, T. et al, Molecular cloning: a laboratory manual, Cold Spring Harbor Laboratory, Cold Spring Harbor, New York (1982) and Loenen, W.A.M. et al, Gene, 20:249 (1980)) or any known eucaryotic cloning vector such as pZIP-Neo SV [X1] or pBKTK-1 (see Poueels, P.H. et al, Cloning Vectors: A Laboratory Manual, Elseiver, Amsterdam (1985)).

Fragments of HPV 35 DNA, HPV 43 DNA, HPV 44 DNA, or HPV 56 DNA can similarly be excised from HPV 35 clones 2A and 2B, HPV 43 clones 2A and 2B, HPV 44 clone 2, or HPV 56 clones 2A and 2B, respectively, using other well known restriction endonucleases and cloned in the above-described cloning vectors. Similarly, the HPV 35 DNA in HPV 35 clones 2A and 2B, the HPV 43 DNA in HPV 43 clones 2A and 2B, the HPV 44 DNA in HPV 44 clone 2, or the HPV 56 DNA in HPV 56 clones 2A and 2B can be excised therefrom and ligated together and cloned in the above-described cloning vectors to obtain a vector containing substantially all of the HPV 35 genome; HPV 43 genome; HPV 44 genome; HPV 56 genome, respectively.

The cloning of HPV 35 DNA or fragments thereof; HPV 43 DNA or fragments thereof; HPV 44 DNA or fragments thereof; or HPV 56 DNA or fragments thereof allows for the relatively simple production of large amounts of HPV 35 DNA or fragments thereof; HPV 43 DNA or fragments thereof; HPV 44 DNA or fragments thereof; or HPV 56 DNA or fragments thereof, respectively, for use in the preparation of nucleic acid hybridization probes for HPV DNA or RNA in general and HPV 35 DNA or RNA; HPV 43 DNA or RNA; HPV 44 DNA or RNA; or HPV 56 DNA or RNA, respectively, in particular.

In addition, HPV 35 DNA or fragments thereof; HPV 43 DNA or fragments thereof; HPV 44 DNA or fragments thereof; or HPV 56 DNA or fragments thereof can be subcloned in other well known cloning vectors to take advantage of special properties of particular cloning vectors which facilitate the synthesis, in vitro, of RNA homologous to the HPV 35 DNA, HPV 43 DNA, HPV 44 DNA or HPV 56 DNA inserted into the cloning vector (see Maniatis, T. et al, Molecular cloning: A laboratory manual, Cold Spring Harbor Laboratory, Cold Spring Harbor, New York (1982)). Examples of these cloning vectors include pT712 and pT713, each of which is commercially available from GIBCO/BRL, Gaithersburg, MD. HPV 35 DNA or fragments thereof; HPV 43 DNA or fragments thereof; HPV 44 DNA or fragments thereof; or HPV 56 DNA or fragments thereof can be subcloned into these cloning vectors so that the HPV 35 DNA or fragments thereof; HPV 43 DNA or fragments thereof; HPV 44 DNA or fragments thereof; or HPV 56 DNA or fragments thereof, respectively, can serve as an efficient template for phage encoded RNA polymerases, e.g., T7, T3 or SP6. Using such cloning vectors and such RNA polymerases, HPV 35 RNA, HPV 43 RNA, HPV 44 RNA or HPV 56 RNA complementary to either one of the strands of HPV 35 DNA or fragments thereof; HPV 43 DNA or fragments thereof; HPV 44 DNA or fragments thereof; or HPV 56 DNA or fragments thereof, respectively, can be synthesized by in vitro transcription using methods well known in the art.

The specific bacterial or eucaryotic hosts for growing the cloning vectors containing HPV 35 DNA or fragments thereof; HPV 43 DNA or fragments thereof; HPV 44 DNA or fragments thereof; or HPV 56 DNA or fragments thereof will depend upon the cloning vector employed. For example, a typical host for growing HPV 35 DNA, HPV 43 DNA, HPV 44 DNA or HPV 56 DNA cloned in λ L47 includes E. coli NM538 (Frischanf, A.M. et al, J. Mol. Biol., 170:827 (1983)). Other hosts such as E. coli HB101 (Boyer, H.W. et al, J. Mol. Biol., 41:459 (1969)) can be employed when using pBR322 or pUC11 as the cloning vector. A typical host for growing HPV 35 DNA, HPV 43 DNA, HPV 44 DNA or HPV 56 DNA cloned in pZIP-Neo SV [X1] is Monkey Cob cells while a typical host for growing HPV DNA cloned in pBKTK-1 would be any of a number of well known mammalian cell lines (see Poueels, P.H. et al, Cloning Vectors: A Laboratory Manual, Elseiver, Amsterdam (1985)).

8

The hybridization of the probes of the present invention to HPV DNA or RNA in general or to HPV 35 DNA or RNA; HPV 43 DNA or RNA; HPV 44 DNA or RNA; or HPV 56 DNA or RNA in particular will depend upon the hybridization conditions employed. That is, under non-stringent hybridization conditions, HPV 35 DNA or or RNA or fragments thereof; HPV 43 DNA or RNA or fragments thereof; HPV 44 DNA or RNA or fragments thereof; or HPV 56 DNA or RNA or fragments thereof can be employed as hybridization probes for HPV DNA or RNA in general. On the other hand, under stringent hybridization conditions, HPV 35 DNA or RNA or fragments thereof; HPV 43 DNA or RNA or fragments thereof; HPV 44 DNA or RNA or fragments thereof; or HPV 56 DNA or RNA or fragments thereof can be employed as hybridization probes for HPV 35 DNA or RNA; HPV 43 DNA or RNA; HPV 44 DNA or RNA; or HPV 56 DNA or RNA, respectively, in particular.

As discussed above, the DNAs and RNAs of different types of HPV are able to cross-hybridize under non-stringent hybridization conditions, i.e., approximately 35°C or more below the melting temperature of a perfectly base-paired double-stranded DNA having a base composition equal to that of HPV DNAs or RNAs as a general group.

Furthermore, it is possible to test an unknown sample of DNA or RNA for the presence of a particular HPV type and to identify that type by carrying out hybridization under stringent hybridization conditions, i.e., approximately 10°C below the melting temperature of a perfectly base-paired double-stranded DNA having a base composition equal to that of HPV DNAs or RNAs as a general group.

In the methods of the present invention, hybridization under non-stringent conditions is carried out by first hybridizing under non-stringent hybridization conditions followed by washing under non-stringent hybridization conditions.

In addition, in the methods of the present invention, hybridization under stringent conditions is carried out by either first hybridizing under non-stringent hybridization conditions followed by washing under stringent hybridization conditions or by first hybridizing under stringent hybridization conditions followed by washing under stringent hybridization conditions. In the first method, i.e., first hybridizing under non-stringent hybridization conditions followed by washing under stringent hybridization conditions, hybrids which form between DNAs or RNAs of different types are unstable but hybrids which form between DNAs and RNAs of the same type are stable.

To determine if an unknown sample of DNA or RNA is of the same or different HPV type as the hybridization probe employed, hybridization is preferably carried out under non-stringent hybridization conditions followed by washing under non-stringent hybridization conditions. After assaying for the presence of hybrids, the detected hybrids are washed under stringent hybridization conditions. In this method, the amount of hybrids which remain after hybridizing under non-stringent hybridization conditions are determined and compared with the amount of hybrids present after washing under stringent hybridization conditions. If the washing under stringent hybridization conditions results in no or minimal reduction in the amount of hybrids formed, then this indicates that the hybrids originally formed, i.e., the ones which formed under non-stringent conditions, were between DNAs or RNAs of the same type. Conversely, abolition of hybrids or a severe reduction of the amount of hybrids which remain after washing under stringent hybridization conditions indicates that the hybrids originally formed, i.e., ones which formed under non-stringent hybridization conditions, were between DNAs or RNAs of different types.

The ability of the HPV DNA or RNA to bind to the unknown sample of DNA or RNA under stringent hybridization conditions is indicative of a high degree of nucleotide sequence homology. On the other hand, the ability of the HPV DNA or RNA to bind to the unknown sample of DNA or RNA only under non-stringent hybridization conditions is indicative of a low or intermediate degree of nucleotide sequence homology. The exact degree of nucleotide sequence homology can only be determined by directly sequencing the unknown DNA and comparing that with the known sequence of the HPV DNA.

In situations in which the detection of HPV DNA or RNA in general in an unknown sample of DNA or RNA, particularly in an unknown sample of DNA or RNA derived from a genital lesion, is being carried out, it is, as a practical matter, advantageous to utilize a hybridization probe composition comprising a mixture of hybridization probes. These hybridization probes comprise probes with sequences representative of all or most of the types suspected of being present in the unknown sample of DNA or RNA. A hybridization probe mixture of DNA or RNA sequences representative of HPV Types 6, 11, 16, 18, 31, 33, 35, 43, 44 and 56 is particularly advantageous when the unknown sample of DNA or RNA is derived from a genital lesion because these HPV types are most likely to be found in genital lesions. Other known HPV types are seldom or never found in genital lesions. For example, HPV Types 1, 2 and 4 are generally found in other types of lesions, i.e., cutaneous warts (see Heilman, C.A. et al, J. Virol., 360:395 (1980)) and thus a hybridization probe mixture of DNA or RNA sequences containing HPV Types 1, 2 and 4 may be advantageous when the unknown sample of DNA or RNA is derived from cutaneous warts but is not as useful when the unknown sample of DNA or RNA is derived from genital lesions.

Examples of sequences of HPV Types 6, 11, 16, 18, 31 and 33 which can be employed in the hybridization probe mixture are described in Gissmann, L., Cancer Surv., 3:161 (1984); Pfister, H., Biochem. Pharmacol., 99:111 (1983); Durst, M. et al, Proc. Natl. Acad. Sci. USA, 80:3812 (1983); Boshart, M. et al, EMBO J., 3:1151 (1984); Lorincz, A.T. et al J. Virol., 58:225 (1986) and Beaudenon, S., Nature, 321:246 (1986). Further, examples of sequences of HPV Types 1, 2 and 4 which can be employed in the hybridization probe mixture are well known in the art (see Heilman, C.A. et al, J. Virol., 360:395 (1980)). Thus, with the disclosure herein as to HPV 35, HPV 43, HPV 44 and HPV 56 and with the knowledge of one skilled in the art as to HPV Types 6, 11, 16, 18, 31 and 33 and to other HPV types such as HPV Types 1, 2 and 4, hybridization probe mixtures can be readily prepared.

In the hybridization probe mixtures, the particular percentage of DNAs or RNAs of each HPV type is not critical in the present invention. Generally, roughly equal molar amounts of DNAs or RNAs of each HPV type are employed in the mixture.

Nucleic acid hybridization as a means of detecting and typing HPV can be carried out in solution as described above (see Loggins, J.R. et al, Cancer Res., 39:545 (1979)) or on a solid support (see Maniatis, T. et al, Molecular cloning: a laboratory manual, Cold Spring Harbor Laboratory, Cold Spring Harbor, New York (1982) or in situ (see Brigati, D.J. et al, Virol., 126:32 (1983) and Beckmann, A.M. et al, J. Med. Virol., 16:265 (1985)).

Hybridization on a solid support can be carried out using a number of different procedures. One such procedure involves purifying all of the unknown DNAs or RNAs, immobilizing such to a solid support in single-stranded form, followed by hybridization with labelled HPV 35 DNA or RNA or fragments thereof; labelled HPV 43 DNA or RNA or fragments thereof; labelled HPV 44 DNA or RNA or fragments thereof; or labelled HPV 56 DNA or RNA or fragments thereof.

Alternatively, the purified unknown DNAs can be digested with one or more restriction endonucleases and the resulting DNA fragments in the samples can be separated electrophoretically. The DNA fragments can then be transferred to a solid support and hybridized with labelled HPV 35 DNA or RNA or fragments thereof; labelled HPV 43 DNA or RNA or fragments thereof; labelled HPV 44 DNA or RNA or fragments thereof; or labelled HPV 56 DNA or RNA fragments thereof.

Hybridization in situ is performed on glass slides and the end result of the procedure is viewed through a microscope. In this procedure, the DNA or RNA is not purified from the cells but is left with all of the other cellular components.

HPV 35 RNA or fragments thereof; HPV 43 RNA or fragments thereof; HPV 44 RNA or fragments thereof; or HPV 56 RNA or fragments thereof are preferably used as nucleic acid hybridization probes for HPV DNA in general and HPV 35 DNA, HPV 43 DNA, HPV 44 DNA, or HPV 56 DNA, respectively, in particular when using crude extracts, particularly crude genital lesion extracts rather than purified DNA, e.g., from such genital lesions.

When employing HPV 35 RNA, HPV 43 RNA, HPV 44 RNA or HPV 56 RNA as a hybridization probe for detecting HPV 35 DNA, HPV 43 DNA, HPV 44 DNA or HPV 56 DNA, respectively, in an unknown sample of DNA, it is preferable that the DNA-RNA hybrids formed after first hybridizing under stringent hybridization conditions, are treated with pancreatic RNaseA (about 20 mg/ml in 50 mM NaCl (pH 7.0)) at room temperature, followed by washing under stringent hybridization conditions.

The HPV 35 DNA or fragments thereof; HPV 43 DNA or fragments thereof; HPV 44 DNA or fragments thereof; or HPV 56 DNA or fragments thereof or HPV 35 RNA or fragments thereof; HPV 43 RNA or fragments thereof; HPV 44 RNA or fragments thereof; or HPV 56 RNA or fragments thereof are useful as nucleic acid hybridization probes for HPV DNA or RNA in general and HPV 35 DNA or RNA; HPV 43 DNA or RNA; HPV 44 DNA or RNA; or HPV 56 DNA or RNA, respectively, in particular when labelled with a radioactive marker such as $^{32}$P, $^{14}$C, $^{3}$H, $^{125}$I or $^{35}$S.

HPV 35 DNA or fragments thereof; HPV 43 DNA or fragments thereof; HPV 44 DNA or fragments thereof; or HPV 56 DNA or fragments thereof can be radioactively labelled, for example, by "nick-translation" by well known means, as described in, for example, Rigby, P.J.W. et al, J. Mol. Biol., 113:237 (1977) and by T4 DNA polymerase replacement synthesis as described in, for examples Deen, K.C. et al, Anal. Biochem., 135:456 (1983)).

HPV 35 RNA or fragments thereof; HPV 43 RNA or fragments thereof; HPV 44 RNA or fragments thereof; or HPV 56 RNA or fragments thereof can be labelled with a radioactive marker by in vitro transcription as described in, for example, Davanloo, P. et al, Proc. Natl. Acad. Sci., USA, 81:2035 (1984)). Since RNA polymerases can utilize labelled precursors, it is possible to synthesize labelled RNA by this method so as to prepare HPV 35 RNA probes; HPV 43 RNA probes; HPV 44 RNA probes; or HPV 56 RNA probes, respectively, for the detection of HPV DNA or RNA in general or HPV 35 DNA or RNA; HPV 43 DNA or RNA; HPV 44 DNA or RNA; or HPV 56 DNA or RNA, respectively, in particular. The labelled precursors which can be used to synthesize labelled RNA include precursors containing radioactive markers such as $^{32}$P, $^{14}$C, $^{3}$H, $^{125}$I or $^{35}$S.

HPV 35 DNA or fragments thereof; HPV 43 DNA or fragments thereof; HPV 44 DNA or fragments thereof; or HPV 56 DNA or fragments thereof or HPV 35 RNA or fragments thereof; HPV 43 RNA or fragments thereof; HPV 44 RNA or fragments thereof; or HPV 56 RNA or fragments thereof are also useful as nucleic acid hybridization probes for HPV DNA or RNA in general and HPV 35 DNA or RNA; HPV 43 DNA or RNA; HPV 44 DNA or RNA; or HPV 56 DNA or RNA, respectively, in particular when labelled with a non-radioactive marker such as biotin, an enzyme or fluorescent group. Biotin acts as a hapten-like group and can be bound to the DNA or RNA and detected by binding an avidin-conjugated enzyme or streptavidin-conjugated enzyme to the biotin followed by washing to remove non-specifically bound enzyme. Upon addition of appropriate substrates for the enzyme, the conversion of the substrate to a colored product can be detected (see Leary, J.J. et al, Proc. Natl. Acad. Sci., USA, 80:4045 (1983)). Examples of such enzymes include alkaline phosphatase and horseradish peroxidase. In addition, fluorescent molecules such as fluorescein and rhodamine can be chemically conjugated to avidin or streptavidin and employed as the non-radioactive marker.

Alternatively, the above-described enzymes or fluorescent molecules can be chemically conjugated directly to the HPV 35 DNA or fragments thereof; HPV 43 DNA or fragments thereof; HPV 44 DNA or fragments thereof; or HPV 56 DNA or fragments thereof or HPV 35 RNA or fragments thereof; HPV 43 RNA or fragments thereof; HPV 44 RNA or fragments thereof; or HPV 56 RNA or fragments thereof as described in, for example, Renz, M, EMBO J., 6:817 (1983), and used in this manner as hybridization probes.

The thus labelled HPV 35 DNA or HPV 35 RNA or fragments thereof; HPV 43 DNA or HPV 43 RNA or fragments thereof; HPV 44 DNA or HPV 44 RNA or fragments thereof; or HPV 56 DNA or HPV 56 RNA or fragments thereof can be used as described above in hybridization studies with an unknown sample of DNA or RNA, particularly an unknown sample of DNA or RNA derived from a genital lesion, to determine if the sample contains HPV DNA or RNA in general and HPV 35 DNA, HPV 43 DNA, HPV 44 DNA or HPV 56 DNA, respectively, in particular.

The unknown sample of DNA, in addition to being derived from a genital lesion, can be derived from other lesions such as throat, oral or skin lesions.

The unknown sample of DNA or RNA can be obtained by, for example, biopsying an epithelial lesion, scraping the cervix or swabbing the cervix to obtain exfoliated cells. In addition, the unknown sample of DNA or RNA can be obtained from bacterial cells in which DNA from a lesion has been cloned using well known means as described in Maniatis, T. et al, Molecular cloning: A laboratory manual, Cold Spring Harbor Laboratory, Cold Spring Harbor, New York (1982) and Gissmann, L., Cancer Surv., 3:161-181 (1984).

In the methods of the present invention, assaying for cross-hybridization can be carried out by assaying for the presence of the radioactive or non-radioactive marker associated with double-stranded nucleic acid hybrids. The methods for determining whether a specific marker is present will depend upon the marker employed and are well known in the art.

In the embodiment of the present invention wherein the detection of HPV 35 DNA or RNA; HPV 43 DNA or RNA; HPV 44 DNA or RNA; or HPV 56 DNA or RNA is based upon a comparison of the epidemiological distribution of cross-hybridization of an unknown sample of DNA or RNA derived from a genital lesion with that of HPV 35 DNA, HPV 43 DNA, HPV 44 DNA or HPV 56 DNA, respectively, the unknown sample of DNA or RNA may exhibit less than 50% cross-hybridization with HPV 35 DNA, HPV 43 DNA, HPV 44 DNA or HPV 56 DNA, respectively, under moderately stringent hybridization conditions, i.e., using hydroxyapatite chromatography for determining whether two HPVs represent different isolates of a common type or represent isolates of a different type, yet, may still be considered HPV 35 DNA or RNA; HPV 43 DNA or RNA; HPV 44 DNA or RNA; or HPV 56 DNA or RNA, respectively, by the definitions herein. As a result, it is necessary to also compare the cross-hybridization of each lesion which comprises the epidemiological distribution in order to detect HPV 35 DNA or RNA; HPV 43 DNA or RNA; HPV 44 DNA or RNA; or HPV 56 DNA or RNA, respectively, in the sample. This is because it may be possible for different HPV types to show the same or similar epidemiological distributions. However, by demonstrating that the same lesions which comprise the epidemiological distribution cross-hybridize both to HPV 35 DNA; HPV 43 DNA; HPV 44 DNA; or HPV 56 DNA, respectively, and the unknown sample of DNA or RNA derived from a genital lesion it is possible to definitively conclude that the sample of unknown DNA or RNA derived from a genital lesion is HPV 35 DNA or RNA; HPV 43 DNA or RNA; HPV 44 DNA or RNA; or HPV 56 DNA or RNA, respectively.

As discussed in more detail below, HPV 35 DNA or RNA has been found to be present in approximately 1% to 4% of cervical lesions from mild dysplasias to invasive cancer. Thus, HPV 35 DNA or RNA is rather uniformly distributed in cervical lesions of various grades.

HPV 43 DNA or RNA has been found to be present in approximately 1% to 4% of benign cervical lesions (such as mild dysplasias) but has not been found in invasive cancer. Thus, HPV 43 DNA or RNA appears to be present only in low grade cervical lesions.

HPV 44 DNA or RNA has been found to be present in approximately 1% to 4% of benign cervical lesions but has not been found in any invasive cancers. Thus, HPV 44 DNA or RNA appears to be present in only low grade cervical lesions.

HPV 56 DNA or RNA has been found to be present in approximately 1% to 4% of benign cervical lesions and has been found in 4% of invasive cancers. Thus, HPV C57 DNA or RNA appears to be present in low grade cervical lesions and cancers. On the other hand, other HPV types, such as HPV Types 6, 11, 16, 18 and 31 exhibit a different distribution in cervical lesions of various grades.

Thus, in the embodiment of the present invention wherein the detection of HPV 35 DNA or RNA; HPV 43 DNA or RNA; HPV 44 DNA or RNA; or HPV 56 DNA or RNA is based upon a comparison of the epidemiological distribution of cross-hybridization of an unknown sample of DNA or RNA derived from a genital lesion with that of HPV 35 DNA, HPV 43 DNA, HPV 44 DNA or HPV 56 DNA, respectively, the unknown sample of DNA or RNA derived from a genital lesion would cross-hybridize with cervical lesions. If such an epidemiological distribution of cross-hybridization is found with the unknown sample of DNA or RNA derived from a genital lesion, then this unknown sample of DNA or RNA may be an HPV 35 DNA or RNA; HPV 43 DNA or RNA; HPV 44 DNA or RNA; or HPV 56 DNA or RNA, respectively. By demonstrating that the same lesions which comprise the epidemiological distribution also cross-hybridize with the unknown sample of DNA or RNA derived from a genital lesion, it can be concluded that the unknown sample of DNA or RNA derived from a genital lesion is HPV 35 DNA or RNA; HPV 43 DNA or RNA; HPV 44 DNA or RNA; or HPV 56 DNA or RNA, respectively.

The particular size of the HPV 35 DNA or HPV 35 RNA fragments; HPV 43 DNA or HPV 43 RNA fragments; HPV 44 DNA or HPV 44 RNA fragments; or HPV 56 DNA or HPV 56 RNA fragments which can be employed as hybridization probes in the present invention is not critical. The size of the HPV 35 DNA or HPV 35 RNA fragments; HPV 43 DNA or HPV 43 RNA fragments; HPV 44 DNA or HPV 44 RNA fragments; or HPV 56 DNA or HPV 56 RNA fragments can be, for example, from about 15 to about 8000 bases or base pairs, depending on whether single stranded or double stranded probes are employed, preferably about 300 to about 800 bases or base pairs. When carrying out hybridization in situ, it is preferable that the size of the HPV 35 DNA or HPV 35 RNA fragments; HPV 43 DNA or HPV 43 RNA fragments; HPV 44 DNA or HPV 44 RNA fragments; or HPV 56 DNA or HPV 56 RNA fragments is smaller than about 500 bases or base pairs since fragments of this size hybridize in situ more efficiently than HPV DNA or HPV RNA fragments larger than about 1000 bases or base pairs. When using double-stranded DNA or RNA, the DNA or RNA must be denatured prior to carrying out hybridization.

The HPV 35 DNA fragments; HPV 43 DNA fragments; HPV 44 DNA fragments; or HPV 56 DNA fragments can be obtained by restriction endonuclease digestion of HPV 35 clones 2A and 2B; HPV 43 clones 2A and 2B; HPV 44 clone 2; or HPV 56 clones 2A or 2B, respectively, or by synthetically manufacturing such using any of the commercially available DNA synthesizing apparatus or by well known chemical methods using the HPV 35 DNA sequence; HPV 43 DNA sequence; HPV 44 DNA sequence; HPV 56 DNA sequence which can be determined by well known means (Sanger, S. et al, Proc. Natl. Acad. Sci. USA, 74:5363 (1977)).

When detecting HPV 35 DNA or RNA; HPV 43 DNA or RNA; HPV 44 DNA or RNA; or HPV 56 DNA or RNA it is preferable to use substantially all of the HPV 35 genome, HPV 43 genome, HPV 44 genome or HPV 56 genome, respectively, as a hybridization probe.

The following examples are given to further illustrate the present invention and are no way intended to limit the scope of the present invention. Unless otherwise indicated, all parts, percents, ratios and the like are by weight.

Example 1

(A) Cloning of HPV 35 DNA

The starting material employed was a cervical adenocarcinoma biopsy obtained from Washington, D.C. consisting of a few milligrams of tissue. Total DNA was purified as described in (Maniatis, T. et al, Molecular cloning: A laboratory manual, Cold Spring Harbor Laboratory, Cold Spring Harbor, New York (1982)). More specifically, the tissue was minced, then digested in 1.0 ml of 50 mM Tris-HCl, pH 8.0 containing 0.6% (w/v) sodium dodecyl sulfate and 50 $\mu$g/ml proteinase K at 37°C overnight. The resulting digest was extracted twice with 1.0 ml of phenol:chloroform (1:1 (v/v)). DNA was then precipitated from the aqueous phase by addition of 2 volumes of 90% (v/v) ethanol. The precipitated DNA was redissolved in 10 mM Tris, 1.0 mM EDTA buffer, pH 8.0 (hereinafter "TE buffer") at a concentration of about 1.0 mg/ml.

The DNA was digested to completion with PstI, electrophoresed in 1% (w/v) agarose gels and DNA transferred to nitrocellulose filters as described in Southern, E.M., J. Mol. Biol., 98:503 (1975). The filters were then probed under non-stringent hybridization conditions ($T_m$-35°C) and stringent hybridization conditions ($T_m$-10°C) with DNA from HPV types 6, 11, 16, 18 and 31. Hybridization was performed overnight at 43°C in 1.0 M NaCl, 50 mM sodium phosphate buffer (pH 7.4), 1.0 mM EDTA, 2% (w/v) sodium dodecyl sulfate, 0.1% (w/v) gelatin, 50 $\mu$g/ml tRNA and 30% (v/v) formamide. Four 30 minute washes were performed at 55°C in 1.2X SSC (1X SSC is 0.15 M NaCl plus 0.015 M sodium citrate), 10 mM sodium phosphate (pH 7.4), 1.0 mM EDTA and 0.5% (w/v) sodium dodecyl sulfate. Hybridization was achieved under non-stringent conditions with HPV types 6, 11, 16, 18 and 31 but not under stringent hybridization conditions.

The resulting purified DNA and λ L47 were digested with BamHI restriction endonuclease, which produced fragments of 3.75 kb and 4.1 kb, the sum of which, i.e., 7.85 kb, is the typical size for a papillomavirus genome. Each of these fragments was cloned into the single BamHI site of λ L47. More specifically, 2.0 $\mu$g of the resulting purified DNA, and 2.0 $\mu$g of λ L47 DNA were cut with 10 units of BamHI in a total volume of 50 $\mu$l of TE buffer for 1 hr at 37°C. The resulting reaction mixture was then diluted with 400 $\mu$l of TE buffer and phenol extracted with an equal volume of phenol:chloroform as described above. The aqueous phase was then extracted with chloroform:isoamyl alcohol (24:1 (v/v)) and DNA from the aqueous phase was precipitated with 80% (v/v) ethanol and dried. The dried DNA was then suspended in 10 $\mu$l of 1X ligase buffer comprising 66 mM Tris-HCl, 6.6 mM $MgCl_2$, 10 mM DTT and 1.0 mM ATP and incubated at 42°C for 2 hours to allow the λ arms to anneal. Next, 0.5 $\mu$l of T4 DNA ligase, i.e., about 1 unit, and 0.5 $\mu$l of 10 mM ATP, pH 7.0 was added to the reaction solution and ligation was allowed to proceed at 12°C overnight.

Next, the ligation products were packaged to form infectious phage and used to infect E. coli. NM538. More specifically, a single colony of E. coli NM538 growing on an agarose plate comprising 10 g Tryptone and 5.0 g NaCl per liter (hereinafter "TN medium") was selected and grown overnight at 37°C in 20 ml of TN medium on a shaking platform (250 rpm) to early stationary phase. The cell culture was then diluted four fold with TN medium and grown for 3 hours. Next, the cells were harvested by centrifuging for 5 minutes at 5,000 rpm in a Sorvall HB-4 rotor and the resulting cell pellet was resuspended in 0.25 of the original volume, in 10 mM $MgSO_4$ and stored at 4°C.

The packaged infectious phages were prepared using a commercially available BRL Lambda In Vitro Packaging System (see Maniatis, T. et al, Molecular cloning: A laboratory manual, Cold Spring Harbor Laboratory, Cold Spring Harbor, New York (1982).

100 $\mu$l of an appropriate dilution of packaged phage in phage storage buffer comprising 0.5 M Tris-HCl (pH 8.0), 0.1 M NaCl, 0.01 M $MgSO_4$ and 0.01% (w/v) gelatin (Difco®) to give 1.5 x $10^4$ plaques per 9 cm diameter plates, was added to 100 $\mu$l of E. coli NM538 prepared as described above in a 10-15 ml test tube, gently mixed and incubated at room temperature for 15 minutes. Then, the cell-phage solution was plated on Trypticase soy broth agar plates comprising 10 g of Trypticase soy broth, 5.0 g NaCl and 15 g agar per liter, which had been prepared at least one day in advance and which had been pre-warmed at 37°C. Thereafter, 3-5 ml of an agarose overlayer comprising 0.5% agarose (ultrapure, electrophoresis grade) dissolved in 10 mM $MgSO_4$, which had been heated in a microwave oven until the solution boiled and then cooled to 45°C before use, was placed over the plated cells-phage. After the agarose had solidified, the plates were transferred to a 37°C forced air incubator with good circulation with the lids of the plates cracked for 30 minutes and then the lids were closed and the plates inverted. After 8-12 hours, plaques became apparent.

Infection resulted in confluent lysis of bacteria on the plates. Recombinant phage carrying HPV DNA were localized by performing "plaque lifts" as described by Benton, W.D. et al, Science, 196:180 (1977). More specifically, confluent lysed plates were placed at 4°C for 1 hour to harden the agarose. Then, an appropriately sized piece of nitrocellulose filter was placed onto each plate by bowing it in the middle, touching the center of the plate and working the contact points toward the edge. Then, four assymetric holes were punched through the nitrocellulose filter and the agar with a small gauge needle and the positions of the holes were marked on the bottom of the plate with a permanent marker. This allowed the nitrocellulose filter and any other areas containing positive signals to be referenced to corresponding positions on the plates. After 10 minutes, the nitrocellulose filters were removed and the DNA was denatured by placing the nitrocellulose filters, plaque side facing upwards, into a dish containing 200 ml of 0.5 M NaOH, 2.0 M NaCl for 1 minute. The nitrocellulose filters were then neutralized by immersion in 500 ml of 0.5 M Tris-HCl, 2.0 M NaCl, pH 7.5 for 5 minutes. Next, the filters were rinsed in 6X SSC comprising 0.9 M NaCl, 0.09 M sodium citrate for 1 minute, dried on Whatman® 3 MM paper and then baked for 30 minutes at 80°C under vacuum.

Thereafter, non-stringent hybridization using HPV 16 DNA labelled with $^{32}$P by "nick translation" as a probe was carried out on the DNA isolated from the lifted plaques (see Rigby, P.J.W. et al, J. Mol. Biol., 113:237 (1977) and Maniatis, T. et al, Molecular cloning: A laboratory manual, Cold Spring Harbor Laboratory, Cold Spring Harbor, New York (1982)) followed by washing and autoradiography. More specifically, hybridization was performed at 41°C in a solution comprising 1.0 M NaCl, 28% (v/v) formamide, 50 mM N-Tris (hydroxymethyl)-methyl-2-aminoethane sulfonic acid (hereinafter "TES"), 10X Denhardt solution, 0.1 mM EDTA and 10 mM sodium phosphate (pH 7.4). Then a non-stringent wash was carried out at 52°C using 1.1X SSC (comprising 0.165 M NaCl and 0.0165 M sodium citrate) in 10 mM sodium phosphate (pH 7.4), 0.1 mM EDTA.

By correspondence with the sites of radioactive exposure, a region of the plate containing phage, which contained DNA that hybridized to HPV 16 DNA, was excised and used to reinfect E. coli NM538 as described above. Localization of phage plaques containing HPV DNA was accomplished by repeating the above procedure. Seven plaques were identified from among the 2 x 10$^5$ plaques screened and three types of clones were found. Two clones exhibited a 3.75 kb insert, two clones a 4.1 kb insert and two clones a 4.3 kb insert. All similar size clones had identical restriction maps. The 3.75 kb and 4.1 kb clones did not cross-hybridize. However, the 4.3 kb clones were found to be homologous to the 3.75 kb clones and also to human genomic DNA. This suggests that these clones contained junction fragments between human DNA and integrated copies of HPV 35 DNA and thus were not analyzed further. A clone containing the 3.75 kb fragment was designated HPV 35 clone 1A and a clone containing the 4.1 kb fragment was designated HPV 35 clone 1B.

The HPV DNA of HPV 35 clones 1A and 1B were then digested with BamHI and subcloned in the single BamHI site of pBR322. The resulting recombinant DNA was designated HPV 35 clones 2A and 2B. HPV 35 clones 2A and 2B have been deposited at the American Type Culture Collection under ATCC No. 40330 and ATCC No. 40331, respectively.

(B) Characterization of HPV 35 DNA

1. Hybridization Studies

Hybridization studies were carried out on HPV 35 clones 2A and 2B DNA to demonstrate that HPV 35 clones 2A and 2B were a new HPV type.

More specifically, $^{32}$P "nick translated" DNA prepared from HPV 35 clones 2A and 2B were hybridized by Southern blotting under stringent conditions to 5.0 ng of DNA from HPV Types 1 to 34. DNA from HPV Types 1 to 34 were obtained from Dr. Gerard Orth of the Institut Pasteur, Paris, France, the assignor of HPV type designations, and Dr. Ethel-Michelle de Villiers of the Papilloma Reference Center in Heidelberg, West Germany, in pre-immobilized form on nitrocellulose filters. More specifically, hybridization was performed at 41°C in a solution comprising 1.0 M NaCl, 28% (v/v) formamide, 50 mM N-Tris TES, 10X Denhardt solution, 0.5 mM EDTA and 20 mM sodium phosphate (pH 7.4). Then, a stringent wash was carried out at 65°C using 0.03X SSC (comprising 0.0045 M NaCl and 0.00045 M sodium citrate) in 10 mM sodium phosphate (pH 7.4), 0.1 mM EDTA.

While significant homology was detected between the recombinant DNA of HPV 35 clones 2A and 2B and HPV types 2, 3, 6, 7, 8, 10, 11, 13, 16, 18, 30 and 31 under non-stringent hybridization conditions, no homology was observed with any of HPV types 1-34 under stringent hybridization conditions, thus demonstrating that HPV 35 clones 2A and 2B represent a new HPV type.

2. Restriction Endonuclease Map

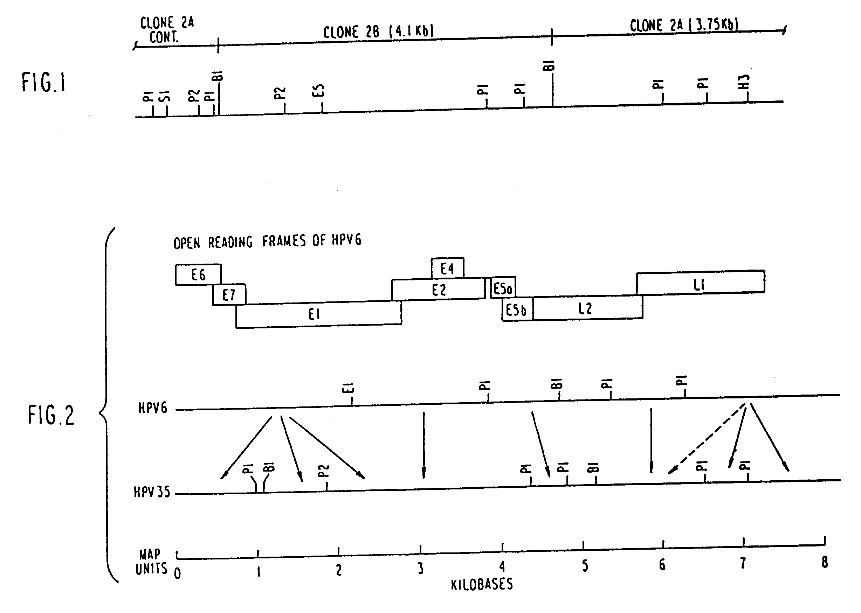

The restriction endonuclease map for HPV 35 is shown in Figure 1. The following restriction enzymes do not cut HPV 35 DNA: SalI, XbaI, NcoI and HpaI.

3. Genomic Organization

In order to demonstrate that the genome of HPV 35 had the same or similar open reading frame organization to HPV 6, the following hybridization studies were carried out. Purified DNA from HPV 35 clones 2A and 2B was subjected to Southern blotting using $^{32}$P "nick translated" fragments of HPV 6 DNA as a probe under non-stringent conditions and under stringent conditions as described above. More specifically, fragments of the BamHI-linearized HPV 6b clone were generated with EcoRI and PstI, then gel purified, nick translated with $^{32}$P and used to probe Southern blots containing: (a) PstI restriction digests of the purified BamHI fragments of HPV 35 clone 2A DNA or (b) PvuII-PstI restriction digests of the purified BamHI fragments of HPV 35 clone 2B DNA. The results, which are shown in Figure 2, demonstrate that the DNA of HPV 35 clones 2A and 2B have the same genomic organizations as HPV 6.

4. Epidemiological Distribution

In order to demonstrate that hybridization probes prepared from HPV 35 clones 2A and 2B hybridize efficiently under stringent conditions only to HPV 35 DNA, and that these hybridization probes can be used to detect genital lesions which contain HPV 35 and to distinguish such genital lesions from genital lesions which contain the DNA of other HPV types, e.g., 6, 11, 16, 18, 31 or 33, the DNA of a collection of cervical biopsies and cervical swabs containing exfoliated cells from the Washington, D.C. metropolitan area (including Maryland and Virginia) and Michigan and surrounding states, were analyzed by nucleic acid hybridization under stringent and non-stringent conditions, for the presence of specific HPV DNAs using probes specific for various HPV types, including probes specific for HPV 35. The biopsies were bisected with half of the specimens being processed for conventional light microscopy and the other half being frozen and stored at -20°C for molecular analysis. The tissues on which Southern blot hybridizations were performed were sectioned on a cryostat in order to obtain material for DNA extraction. Approximately every fifteenth section was stained with hematoxylin and eosin stain and examined microscopically in order to confirm that this tissue sample was comparable to the portion of the specimen analyzed by light microscopy. Exfoliated cervical cells were analyzed by standard cytological methods, e.g., pap smear, on paired samples, the other of which was used for DNA analysis.

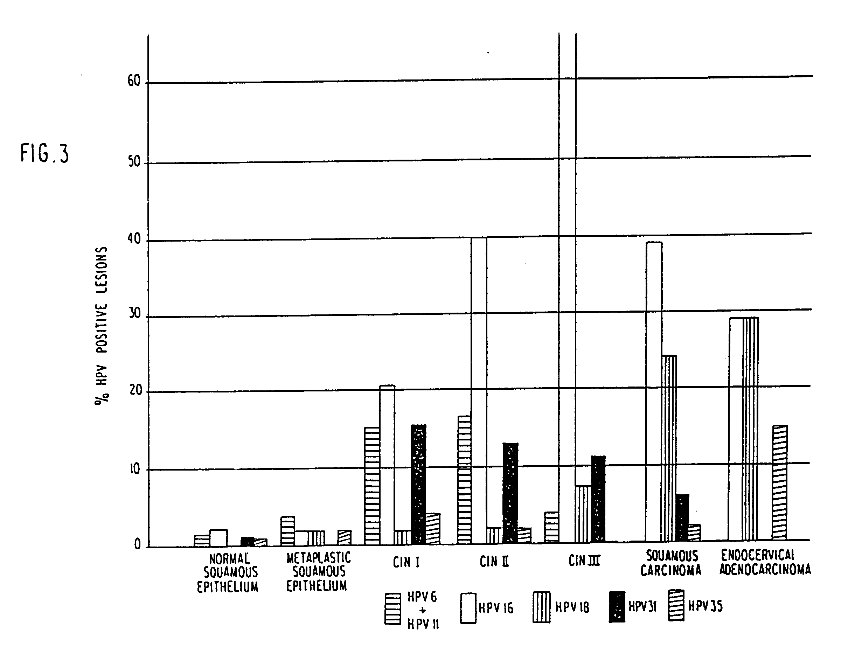

High molecular weight DNA was prepared from the samples as described above. 1 to 10 $\mu$g of purified cellular DNA were digested with either PstI or BamHI and the digested samples were electrophoresed in 1.0% (w/v) agarose gels and transferred to nitrocellulose filters. Thereafter, hybridization was carried out under stringent conditions as described above with nick-translated $^{32}$P-labelled HPV DNAs from the types discussed above. (For HPV 35 DNA, a mixture of HPV DNA from HPV 35 clones 2A and 2B was employed.) Note, since the HPV DNAs were propagated in pBR322 or related vectors, in order to minimize the possibility of reactivity with pBR322 and related vector-like sequences in the tissue samples, all probes were electrophoretically purified to remove most of the associated vector sequences. Additional hybridization was also carried out in the presence of labelled pBR322 and related vectors, to reveal any potential false positives due to the plasmid-like sequences in the tissue samples. The results are shown in Table 1 below. The results in Table 1 are graphically illustrated in Figure 3.

Table 1

Distribution of HPV Types in Cervical Biopsies from the Washington, D.C. Metropolitan Area and Michigan

| | Normal Squamous Epithelium [a] | Metaplastic Squamous Epithelium [b] | Condyloma | CIN I | CIN II | CIN III | Squamous Carcinoma | Endocervical Adenocarcinoma |
|---|---|---|---|---|---|---|---|---|
| HPV 6 | 5 (1.14%) | 1 (1.82%) | 17 (29.31%) | 6 (11.11%) | 9 (16.36%) | 1 (3.57%) | 0 | 0 |
| HPV 11 | 0 | 1 (1.82%) | 7 (12.07%) | 2 (3.70%) | 0 | 0 | 0 | 0 |
| HPV 16 | 10 (2.28%) | 1 (1.82%) | 4 (6.89%) | 11 (20.37%) | 22 (40.0%) | 19 (67.86%) | 19 (38.78%) | 2 (28.57%) |
| HPV 18 | 0 | 1 (1.82%) | 1 (1.72%) | 1 (1.85%) | 1 (1.82%) | 2 (7.14%) | 12 (24.40%) | 2 (28.57%) |
| HPV 31 | 4 (0.91%) | 0 | 1 (1.72%) | 8 (14.81%) | 7 (12.73%) | 3 (10.71%) | 3 (6.12%) | 0 |
| HPV 33 | 3 (0.68%) | 1 (1.82%) | 0 | 3 (5.56%) | 1 (1.82%) | 0 | 1 (2.04%) | 0 |
| HPV 35 | 5 (1.14%) | 1 (1.82%) | 1 (1.72%) | 2 (3.70%) | 1 (1.82%) | 0 | 1 (2.04%) | 1 (14.29%) |
| Total No. of HPV Positive Biopsies | 27 | 6 | 31 | 33 | 41 | 25 | 36 | 5 |
| Total No. of HPV Negative Biopsies | 412 | 49 | 27 | 21 | 14 | 3 | 13 | 2 |
| Total No. of Biopsies | 439 | 55 | 58 | 54 | 55 | 28 | 49 | 7 |

a These biopsies were from the portio of the cervix.

b These biopsies were from the transformation zone.

Table 1 and Figure 3 demonstrate that cervical biopsies containing HPV 35 can be distinguished from biopsies containing other HPV types, e.g., 6, 11, 16, 18, 31 or 33, not only by the criteria of degree of cross-hybridization in solution followed by hydroxyapatite chromatography but, also by the ability of an HPV 35 DNA probe to specifically detect and identify distinct populations of genital lesions, i.e., ones which contain HPV 35 DNA, as compared to ones which contain other HPV types. In addition, the results in Table 1 and Figure 3 show that the epidemiological distribution of HPV 35 DNA among cervical biopsies is distinct from that found for some other HPV types.

Example 2

(A) Cloning of HPV 43 DNA

The starting material employed was a vulvar hyperkeratosis obtained from Michigan. consisting of a few milligrams of tissue. Total DNA was purified as described in (Maniatis, T. et al, Molecular cloning: A laboratory manual, Cold Spring Harbor Laboratory, Cold Spring Harbor, New York (1982)). More specifically, the tissue was minced, then digested in 1.0 ml of 50 mM Tris-HCl, pH 8.0 containing 0.6% (w/v) sodium dodecyl sulfate and 50 $\mu$g/ml proteinase K at 37°C overnight. The resulting digest was extracted twice with 1.0 ml of phenol:chloroform (1:1 (v/v)). DNA was then precipitated from the aqueous phase by addition of 2 volumes of 90% (v/v) ethanol. The precipitated DNA was redissolved in 10 mM Tris, 1.0 mM EDTA buffer, pH 8.0 (hereinafter "TE buffer") at a concentration of about 1.0 mg/ml.

The DNA was digested to completion with PstI, electrophoresed in 1.0% (w/v) agarose gels and DNA transferred to nitrocellulose filters as described in Southern, E.M., J. Mol. Biol., 98:503 (1975). The filters were then probed under non-stringent hybridization conditions ($T_m$-35°C) and stringent hybridization conditions ($T_m$-10°C) with DNA from HPV types 6, 11, 16, 18 and 31. Hybridization was performed overnight at 43°C in 1.0 M NaCl, 50 mM sodium phosphate buffer (pH 7.4), 1.0 mM EDTA, 2% (w/v) sodium dodecyl sulfate, 0.1% (w/v) gelatin, 50 $\mu$g/ml tRNA and 30% (v/v) formamide. Four 30 minute washes were performed at 55°C in 1.2X SSC (1X SSC is 0.15 M NaCl plus 0.015 M sodium citrate), 10 mM sodium phosphate (pH 7.4), 1.0 mM EDTA and 0.5% (w/v) sodium dodecyl sulfate. Hybridization was achieved under non-stringent conditions with HPV types 6, 11, 16, 18 and 31 but not under stringent hybridization conditions.

The resulting purified DNA and λ L47 were digested with BamHI restriction endonuclease, which produced a fragment of 6.3 kb or with HindIII which produced a fragment of 2.85 kb, the sum of which, i.e., 9.15 kb, is larger than the papillomavirus genome. Mapping revealed that the 6.3 kb and 2.85 kb fragments overlapped for 1.55 kb of their lengths. Thus, the amount of non-overlapping HPV sequence represented by the 6.3 kb and the 2.85 kb fragments is 7.6 kb or approximately 96% of the typical size of HPV genome (7.9 kb). Each of these fragments was cloned into the single BamHI or HindIII site of λ L47. More specifically, 2.0 $\mu$g of the resulting purified DNA, and 2.0 $\mu$g of λ L47 DNA were cut with 10 units of BamHI in a total volume of 50 $\mu$l of TE buffer for 1 hr at 37°C. In another reaction 2.0 $\mu$g of λ L47 DNA and 2 $\mu$g of the purified DNA were cut with HindIII in a total volume of 50 $\mu$l of TE buffer for 1 hr at 37°C. The resulting reaction mixtures were then diluted with 400 $\mu$l of TE buffer and phenol extracted with equal volumes of phenol:chloroform as described above. The aqueous phases were then extracted with chloroform:isoamyl alcohol (24:1 (v/v)) and DNA from the aqueous phases were precipitated with 80% (v/v) ethanol and dried. The dried DNAs were then each suspended in 10 $\mu$l of 1X ligase buffer comprising 66 mM Tris-HCl, 6.6 mM $MgCl_2$, 10 mM DTT and 1.0 mM ATP and incubated at 42°C for 2 hours to allow the λ arms to anneal. Next, 0.5 $\mu$l of T4 DNA ligase, i.e., about 1 unit, and 0.5 $\mu$l of 10 mM ATP, pH 7.0 was added to each reaction solution and ligation was allowed to proceed at 12°C overnight.

Next, the ligation products were packaged to form infectious phage and used to infect E. coli. NM538. More specifically, a single colony of E. coli NM538 growing on an agarose plate comprising 10 g Tryptone and 5.0 g NaCl per liter (hereinafter "TN medium") was selected and grown overnight at 37°C in 20 ml of TN medium on a shaking platform (250 rpm) to early stationary phase. The cell culture was then diluted four fold with TN medium and grown for 3 hours. Next, the cells were harvested by centrifuging for 5 minutes at 5,000 rpm in a Sorvall HB-4 rotor and the resulting cell pellet was resuspended in 0.25 of the original volume, in 10 mM $MgSO_4$ and stored at 4°C.

The packaged infectious phages were prepared using a commercially available BRL Lambda In Vitro Packaging System (see Maniatis, T. et al, Molecular cloning: A laboratory manual, Cold Spring Harbor Laboratory, Cold Spring Harbor, New York (1982).

100 $\mu$l of an appropriate dilution of packaged phage in phage storage buffer comprising 0.5 M Tris-HCl (pH 8.0), 0.1 M NaCl, 0.01 M $MgSO_4$ and 0.01% (w/v) gelatin (Difco®) to give 1.5 x $10^4$ plaques per 9 cm diameter plates, was added to 100 $\mu$l of E. coli NM538 prepared as described above in a 10-15 ml test tube, gently mixed and incubated at room temperature for 15 minutes. Then, the cell-phage solution was plated on Trypticase soy broth agar plates comprising 10 g of Trypticase soy broth, 5.0 g NaCl and 15 g agar per liter, which had been prepared at least one day in advance and which had been pre-warmed at 37°C. Thereafter, 3-5 ml of an agarose overlayer comprising 0.5% agarose (ultrapure, electrophoresis grade) dissolved in 10 mM $MgSO_4$, which had been heated in a microwave oven until the solution boiled and then cooled to 45°C before use, was placed over the plated cells-phage. After the agarose had solidified, the plates were transferred to a 37°C forced air incubator with good circulation with the lids of the plates cracked for 30 minutes and then the lids were closed and the plates inverted. After 8-12 hours, plaques became apparent.

Infection resulted in confluent lysis of bacteria on the plates. Recombinant phage carrying HPV DNA were localized by performing "plaque lifts" as described by Benton, W.D. et al, Science, 196:180 (1977). More specifically, confluent lysed plates were placed at 4°C for 1 hour to harden the agarose. Then, an appropriately sized piece of nitrocellulose filter was placed onto each plate by bowing it in the middle, touching the center of the plate and working the contact points toward the edge. Then, four assymetric holes were punched through the nitrocellulose filter and the agar with a small gauge needle and the positions of the holes were marked on the bottom of the plate with a permanent marker. This allowed the nitrocellulose filter and any other areas containing positive signals to be referenced to corresponding positions on the plates. After 10 minutes, the nitrocellulose filters were removed and the DNA was denatured by placing the nitrocellulose filters, plaque side facing upwards, into a dish containing 200 ml of 0.5 M NaOH, 2.0 M NaCl for 1 minute. The nitrocellulose filters were then neutralized by immersion in 500 ml of 0.5 M Tris-HCl, 2.0 M NaCl, pH 7.5 for 5 minutes. Next, the filters were rinsed in 6X SSC comprising 0.9 M NaCl, 0.09 M sodium citrate for 1 minute, dried on Whatman® 3 MM paper and then baked for 30 minutes at 80°C under vacuum.

Thereafter, non-stringent hybridization using HPV 16 DNA labelled with $^{32}$P by "nick translation" as a probe was carried out on the DNA isolated from the lifted plaques (see Rigby, P.J.W. et al, J. Mol. Biol., 113:237 (1977) and Maniatis, T. et al, Molecular cloning: A laboratory manual, Cold Spring Harbor Laboratory, Cold Spring Harbor, New York (1982)) followed by washing and autoradiography. More specifically, hybridization was performed at 41°C in a solution comprising 1.0 M NaCl, 28% (v/v) formamide, 50 mM N-Tris (hydroxymethyl)-methyl-2-aminoethane sulfonic acid (hereinafter "TES"), 10X Denhardt solution, 0.1 mM EDTA and 10 mM sodium phosphate (pH 7.4). Then a non-stringent wash was carried out at 52°C using 1.1X SSC (comprising 0.165 M NaCl and 0.0165 M sodium citrate) in 10 mM sodium phosphate (pH 7.4), 0.1 mM EDTA.

By correspondence with the sites of radioactive exposure, a region of the plate containing phage, which contained DNA that hybridized to HPV 16 DNA, was excised and used to reinfect E. coli NM538 as described above. Localization of phage plaques containing HPV DNA was accomplished by repeating the above procedure. One plaque was identified from among the $1.65 \times 10^5$ plaques screened from the cloning using the BamHI digested DNA. The cloned fragment exhibited a size of 6.3 kb and was designated HPV 43 clone 1A. One plaque was identified from among the $9 \times 10^4$ plaques screened from the cloning using the HindIII digested DNA. The cloned fragment exhibited a size of 2.85 kb and was designated HPV 43 clone 1B.

The HPV DNA of HPV 43 clones 1A and 1B were then digested with BamHI or HindIII, respectively, and subcloned in the single BamHI or HindIII site of pT713. The resulting recombinant DNA was designated HPV 43 clones 2A and 2B. HPV 43 clones 2A and 2B have been deposited at the American Type Culture Collection under ATCC No. 40338 and ATCC No. 40339, respectively.

(B) Characterization of HPV 43 DNA

1. Hybridization Studies

Hybridization studies were carried out on HPV 43 clones 2A and 2B DNA to demonstrate that HPV 43 clones 2A and 2B were a new HPV type.

More specifically, $^{32}$P "nick translated" DNA prepared from HPV 43 clones 2A and 2B were hybridized by Southern blotting under stringent conditions to 5.0 ng of DNA from HPV Types 1 to 42. DNA from HPV Types 1 to 42 were obtained from Dr. Gerard Orth of the Institut Pasteur, Paris, France, the assignor of HPV type designations, Dr. Ethel-Michelle de Villiers of the Papilloma Reference Center in Heidelberg, West Germany, and Life Technologies, Inc. in pre-immobilized form on nitrocellulose filters. More specifically, hybridization was performed at 41°C in a solution comprising 1.0 M NaCl, 28% (v/v) formamide, 50 mM N-Tris TES, 10X Denhardt solution, 0.5 mM EDTA and 20 mM sodium phosphate (pH 7.4). Then, a stringent wash was carried out at 65°C using 0.03X SSC (comprising 0.0045 M NaCl and 0.00045 M sodium citrate) in 10 mM sodium phosphate (pH 7.4), 0.1 mM EDTA.

While significant homology was detected between the recombinant DNA of HPV 43 clones 2A and 2B and most of the other HPV types under non-stringent hybridization conditions, no homology was observed with any of HPV types 1-42 under stringent hybridization conditions, thus demonstrating that HPV 43 clones 2A and 2B represent a new HPV type.

2. Restriction Endonuclease Map

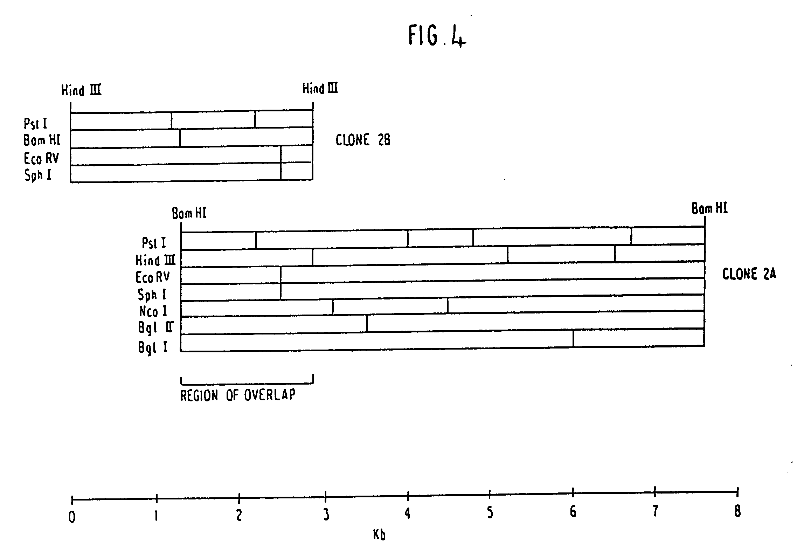

The restriction endonuclease map for HPV 43 clone 2A and clone 2B is shown in Figure 4. The following restriction enzymes do not cut HPV 43 DNA: EcoRI and SalI.

3. Genomic Organization

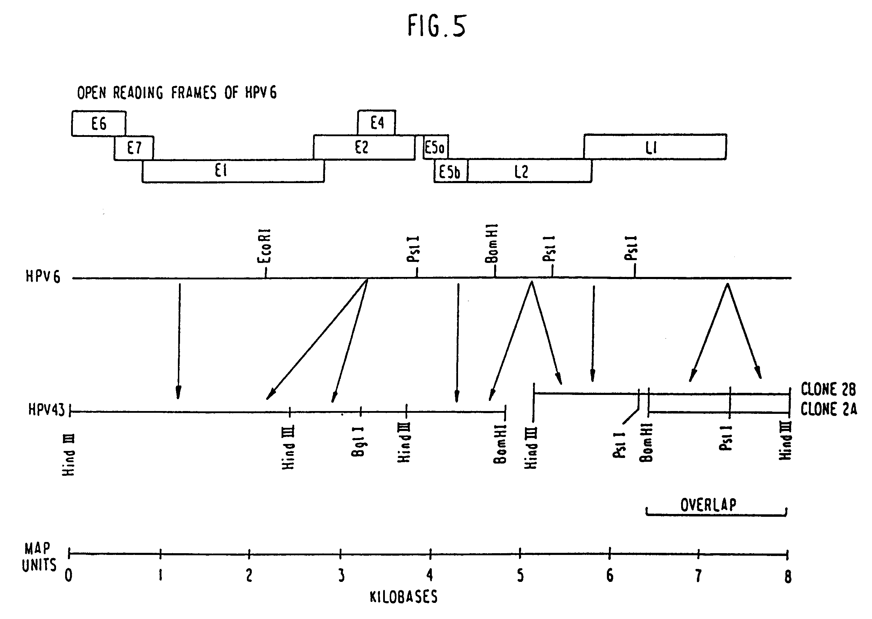

In order to demonstrate that the genome of HPV 43 had the same or similar open reading frame organization to HPV 6, the following hybridization studies were carried out. Purified DNA from HPV 43 clones 2A and 2B was subjected to Southern blotting using $^{32}$P "nick translated" fragments of HPV 6 DNA as a probe under non-stringent conditions and under stringent conditions as described above. More specifically, fragments of the BamHI-linearized HPV 6b clone were generated with EcoRI and PstI, then gel purified, nick translated with $^{32}$P and used to probe Southern blots containing: (a) PstI restriction digests of the purified BamHI fragments of HPV 43 clone 2A DNA or PstI digests of the purified HindIII fragments of HPV 43 clone 2B DNA. The results, which are shown in Figure 5, demonstrate that the DNA of HPV 43 clones 2A and 2B have the same genomic organizations as HPV 6.

4. Epidemiological Distribution

In order to demonstrate that hybridization probes prepared from HPV 43 clones 2A and 2B hybridize efficiently under stringent conditions only to HPV 43 DNA, and that these hybridization probes can be used to detect genital lesions which contain HPV 43 and to distinguish such genital lesions from genital lesions which contain the DNA of other HPV types, e.g., 6, 11, 16, 18, 31 or 33, the DNA of a collection of cervical biopsies and cervical swabs containing exfoliated cells from the Washington, D.C. metropolitan area (including Maryland and Virginia) and Michigan and surrounding states, were analyzed by nucleic acid hybridization under stringent and non-stringent conditions, for the presence of specific HPV DNAs using probes specific for various HPV types, including probes specific for HPV 43. The biopsies were bisected with half of the specimens being processed for conventional light microscopy and the other half being frozen and stored at -20°C for molecular analysis. The tissues on which Southern blot hybridizations were performed were sectioned on a cryostat in order to obtain material for DNA extraction. Approximately every fifteenth section was stained with hematoxylin and eosin stain and examined microscopically in order to confirm that this tissue sample was comparable to the portion of the specimen analyzed by light microscopy. Exfoliated cervical cells were analyzed by standard cytological methods, e.g., pap smear, on paired samples, the other of which was used for DNA analysis.

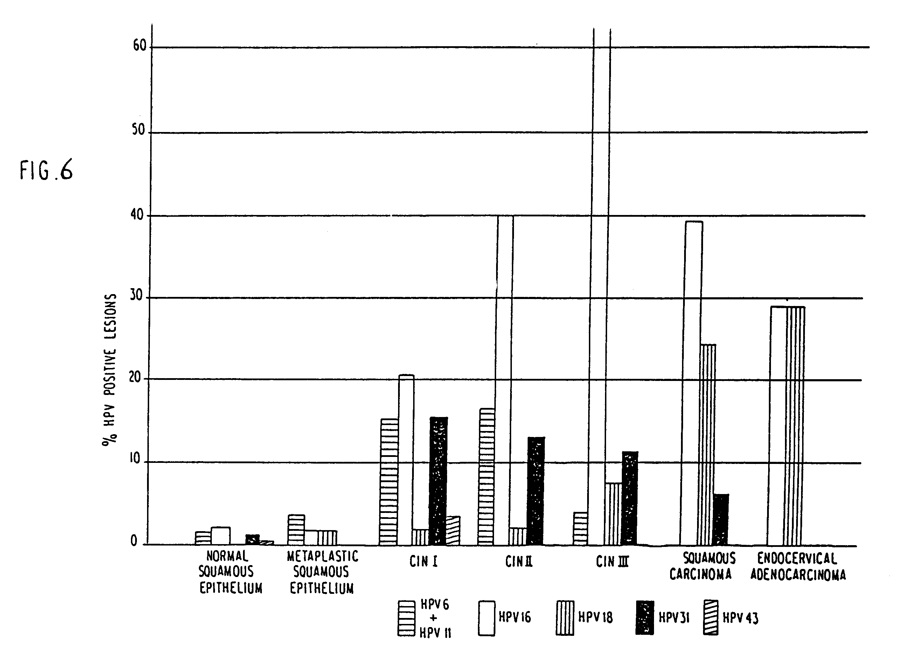

High molecular weight DNA was prepared from the samples as described above. 1 to 10 $\mu$g of purified cellular DNA were digested with either PstI or BamHI and the digested samples were electrophoresed in 1.0% (w/v) agarose gels and transferred to nitrocellulose filters. Thereafter, hybridization was carried out under stringent conditions as described above with nick-translated $^{32}$P-labelled HPV DNAs from the types discussed above. (For HPV 43 DNA, a mixture of HPV DNA from HPV 43 clones 2A and 2B was employed.) Note, since the HPV DNAs were propagated in pT713, pBR322 or related vectors, in order to minimize the possibility of reactivity with pT713, pBR322 and related vector-like sequences in the tissue samples, all probes were electrophoretically purified to remove most of the associated vector sequences. Additional hybridization was also carried out in the presence of labelled pT713, pBR322 and related vectors, to reveal any potential false positives due to the plasmid-like sequences in the tissue samples. The results are shown in Table 2 below. The results in Table 2 are graphically illustrated in Figure 6.

Table 2

Distribution of HPV Types in Cervical Biopsies from the Washington, D.C. Metropolitan Area and Michigan

| | Normal Squamous Epithelium [a] | Metaplastic Squamous Epithelium [b] | Condyloma | CIN I | CIN II | CIN III | Squamous Carcinoma | Endocervical Adenocarcinoma |
|---|---|---|---|---|---|---|---|---|
| HPV 6 | 5 (1.14%) | 1 (1.82%) | 17 (29.31%) | 6 (11.11%) | 9 (16.36%) | 1 (3.57%) | 0 | 0 |
| HPV 11 | 0 | 1 (1.82%) | 7 (12.07%) | 2 (3.70%) | 0 | 0 | 0 | 0 |
| HPV 16 | 10 (2.28%) | 1 (1.82%) | 4 (6.89%) | 11 (20.37%) | 22 (40.0%) | 19 (67.86%) | 19 (38.78%) | 2 (28.57%) |
| HPV 18 | 0 | 1 (1.82%) | 1 (1.72%) | 1 (1.85%) | 1 (1.82%) | 2 (7.14%) | 12 (24.40%) | 2 (28.57%) |
| HPV 31 | 4 (0.91%) | 0 | 1 (1.72%) | 8 (14.81%) | 7 (12.73%) | 3 (10.71%) | 3 (6.12%) | 0 |
| HPV 33 | 3 (0.68%) | 1 (1.82%) | 0 | 3 (5.56%) | 1 (1.82%) | 0 | 1 (2.04%) | 0 |
| HPV 43 | 1 (0.23%) | 0 | 2 (3.45%) | 2 (3.70%) | 0 | 0 | 0 | 0 |
| Total No. of HPV Positive Biopsies | 23 | 5 | 32 | 33 | 40 | 25 | 35 | 4 |
| Total No. of HPV Negative Biopsies | 412 | 49 | 27 | 21 | 14 | 3 | 13 | 2 |
| Total No. of Biopsies | 435 | 54 | 59 | 54 | 54 | 28 | 48 | 6 |

a These biopsies were from the portio of the cervix.

b These biopsies were from the transformation zone.

Table 2 and Figure 6 demonstrate that cervical biopsies containing HPV 43 can be distinguished from biopsies containing other HPV types, e.g., 6, 11, 16, 18, 31 or 33, not only by the criteria of degree of cross-hybridization in solution followed by hydroxyapatite chromatography but, also by the ability of an HPV 43 DNA probe to specifically detect and identify distinct populations of genital lesions, i.e., ones which contain HPV 43 DNA, as compared to ones which contain other HPV types. In addition, the results in Table 2 and Figure 6 show that the epidemiological distribution of HPV 43 DNA among cervical biopsies is distinct from that found for some other HPV types.

Example 3

(A) Cloning of HPV 44 DNA

The starting material employed was a vulvar condyloma biopsy obtained from Michigan consisting of a few milligrams of tissue. Total DNA was purified as described in (Maniatis, T. et al, Molecular cloning: A laboratory manual, Cold Spring Harbor Laboratory, Cold Spring Harbor, New York (1982)). More specifically, the tissue was minced, then digested in 1.0 ml of 50 mM Tris-HCl, pH 8.0 containing 0.6% (w/v) sodium dodecyl sulfate and 50 $\mu$g/ml proteinase K at 37°C overnight. The resulting digest was extracted twice with 1.0 ml of phenol:chloroform (1:1 (v/v)). DNA was then precipitated from the aqueous phase by addition of 2 volumes of 90% (v/v) ethanol. The precipitated DNA was redissolved in 10 mM Tris, 1.0 mM EDTA buffer, pH 8.0 (hereinafter "TE buffer") at a concentration of about 1.0 mg/ml.

The DNA was digested to completion with PstI, electrophoresed in 1.0% (w/v) agarose gels and DNA transferred to nitrocellulose filters as described in Southern, E.M., J. Mol. Biol., 98:503 (1975). The filters were then probed under non-stringent hybridization conditions ($T_m$-35°C) and stringent hybridization conditions ($T_m$-10°C) with DNA from HPV types 6, 11, 16, 18 and 31. Hybridization was performed overnight at 43°C in 1.0 M NaCl, 50 mM sodium phosphate buffer (pH 7.4), 1.0 mM EDTA, 2% (w/v) sodium dodecyl sulfate, 0.1% (w/v) gelatin, 50 $\mu$g/ml tRNA and 30% (v/v) formamide. Four 30 minute washes were performed at 55°C in 1.2X SSC (1X SSC is 0.15 M NaCl plus 0.015 M sodium citrate), 10 mM sodium phosphate (pH 7.4), 1.0 mM EDTA and 0.5% (w/v) sodium dodecyl sulfate. Hybridization was achieved under non-stringent conditions with HPV types 6, 11, 16, 18 and 31 but not under stringent hybridization conditions.

The resulting purified DNA was digested with BamHI restriction endonuclease, which produced a fragment of 7.8 kb (the typical size for a papillomavirus genome). This fragment was cloned into the single BamHI site of λ L47. More specifically, 2.0 $\mu$g of the resulting purified DNA, and 2.0 $\mu$g of λ L47 DNA were cut with 10 units of BamHI in a total volume of 50 $\mu$l of TE buffer for 1 hr at 37°C. The resulting reaction mixture was then diluted with 400 $\mu$l of TE buffer and phenol extracted with an equal volume of phenol:chloroform as described above. The aqueous phase was then extracted with chloroform:isoamyl alcohol (24:1 (v/v)) and DNA from the aqueous phase was precipitated with 80% (v/v) ethanol and dried. The dried DNA was then suspended in 10 $\mu$l of 1X ligase buffer comprising 66 mM Tris-HCl, 6.6 mM MgCl$_2$, 10 mM DTT and 1.0 mM ATP and incubated at 42°C for 2 hours to allow the λ arms to anneal. Next, 0.5 $\mu$l of T4 DNA ligase, i.e., about 1 unit, and 0.5 $\mu$l of 10 mM ATP, pH 7.0 was added to the reaction solution and ligation was allowed to proceed at 12°C overnight.

Next, the ligation products were packaged to form infectious phage and used to infect E. coli. NM538. More specifically, a single colony of E. coli NM538 growing on an agarose plate comprising 10 g Tryptone and 5.0 g NaCl per liter (hereinafter "TN medium") was selected and grown overnight at 37°C in 20 ml of TN medium on a shaking platform (250 rpm) to early stationary phase. The cell culture was then diluted four fold with TN medium and grown for 3 hours. Next, the cells were harvested by centrifuging for 5 minutes at 5,000 rpm in a Sorvall HB-4 rotor and the resulting cell pellet was resuspended in 0.25 of the original volume, in 10 mM MgSO$_4$ and stored at 4°C.

The packaged infectious phages were prepared using a commercially available BRL Lambda In Vitro Packaging System (see Maniatis, T. et al, Molecular cloning: A laboratory manual, Cold Spring Harbor Laboratory, Cold Spring Harbor, New York (1982).

100 $\mu$l of an appropriate dilution of packaged phage in phage storage buffer comprising 0.5 M Tris-HCl (pH 8.0), 0-1 M NaCl, 0.01 M MgSO$_4$ and 0.01% (w/v) gelatin (Difco®) to give 1.5 x 10$^4$ plaques per 9 cm diameter plates, was added to 100 $\mu$l of E. coli NM538 prepared as described above in a 10-15 ml test tube, gently mixed and incubated at room temperature for 15 minutes. Then, the cell-phage solution was plated on Trypticase soy broth agar plates comprising 10 g of Trypticase soy broth, 5.0 g NaCl and 15 g agar per liter, which had been prepared at least one day in advance and which had been pre-warmed at 37°C. Thereafter, 3-5 ml of an agarose overlayer comprising 0.5% agarose (ultrapure, electrophoresis grade) dissolved in 10 mM MgSO$_4$, which had been heated in a microwave oven until the solution boiled and then cooled to 45°C before use, was placed over the plated cells-phage. After the agarose had solidified, the plates were transferred to a 37°C forced air incubator with good circulation with the lids of the plates cracked for 30 minutes and then the lids were closed and the plates inverted. After 8-12 hours, plaques became apparent.

Infection resulted in confluent lysis of bacteria on the plates. Recombinant phage carrying HPV DNA were localized by performing "plaque lifts" as described by Benton, W.D. et al, Science, 196:180 (1977). More specifically, confluent lysed plates were placed at 4°C for 1 hour to harden the agarose. Then, an appropriately sized piece of nitrocellulose filter was placed onto each plate by bowing it in the middle, touching the center of the plate and working the contact points toward the edge. Then, four assymetric holes were punched through the nitrocellulose filter and the agar with a small gauge needle and the positions of the holes were marked on the bottom of the plate with a permanent marker. This allowed the nitrocellulose filter and any other areas containing positive signals to be referenced to corresponding positions on the plates. After 10 minutes, the nitrocellulose filters were removed and the DNA was denatured by placing the nitrocellulose filters, plaque side facing upwards, into a dish containing 200 ml of 0.5 M NaOH, 2.0 M NaCl for 1 minute. The nitrocellulose filters were then neutralized by immersion in 500 ml of 0.5 M Tris-HCl, 2.0 M NaCl, pH 7.5 for 5 minutes. Next, the filters were rinsed in 6X SSC comprising 0.9 M NaCl, 0.09 M sodium citrate for 1 minute, dried on Whatman® 3 MM paper and then baked for 30 minutes at 80°C under vacuum.

Thereafter, non-stringent hybridization using HPV 16 DNA labelled with $^{32}$P by "nick translation" as a probe was carried out on the DNA isolated from the lifted plaques (see Rigby, P.J.W. et al, J. Mol. Biol., 113:237 (1977) and Maniatis, T. et al, Molecular cloning: A laboratory manual, Cold Spring Harbor Laboratory, Cold Spring Harbor, New York (1982)) followed by washing and autoradiography. More specifically, hybridization was performed at 41°C in a solution comprising 1.0 M NaCl, 28% (v/v) formamide, 50 mM N-Tris (hydroxymethyl)-methyl-2-aminoethane sulfonic acid (hereinafter "TES"), 10X Denhardt solution, 0.1 mM EDTA and 10 mM sodium phosphate (pH 7.4). Then a non-stringent wash was carried out at 52°C using 1.1X SSC (comprising 0.165 M NaCl and 0.0165 M sodium citrate) in 10 mM sodium phosphate (pH 7.4), 0.1 mM EDTA.

By correspondence with the sites of radioactive exposure, a region of the plate containing phage, which contained DNA that hybridized to HPV 16 DNA, was excised and used to reinfect E. coli NM538 as described above. Localization of phage plaques containing HPV DNA was accomplished by repeating the above procedure. Six plaques were identified from among the $2 \times 10^5$ plaques screened. All clones had identical restriction maps. One of the clones was chosen for further studies and was designated HPV 44 clone 1.

The HPV DNA of HPV 44 clone 1 was then digested with BamHI and subcloned in the single BamHI site of pT713. The resulting recombinant DNA was designated HPV 44 clone 2. HPV 44 clone 2 has been deposited at the American Type Culture Collection under ATCC No. 40353.

(B) Characterization of HPV 44 DNA

1. Hybridization Studies

Hybridization studies were carried out on HPV 44 clone 2 DNA to demonstrate that HPV 44 clone 2 was a new HPV type.

More specifically, $^{32}$P "nick translated" DNA prepared from HPV 44 clone 2 was hybridized by Southern blotting under stringent conditions to 5.0 ng of DNA from HPV Types 1 to 43. DNA from HPV Types 1 to 43 were obtained from Dr. Gerard Orth of the Institut Pasteur, Paris, France, the assignor of HPV type designations, Dr. Ethel-Michelle de Villiers of the Papilloma Reference Center in Heidelberg, West Germany, and from Life Technologies, Inc., in pre-immobilized form on nitrocellulose filters. More specifically, hybridization was performed at 41°C in a solution comprising 1.0 M NaCl, 28% (v/v) formamide, 50 mM N-Tris TES, 10X Denhardt solution, 0.5 mM EDTA and 20 mM sodium phosphate (pH 7.4). Then, a stringent wash was carried out at 65°C using 0.03X SSC (comprising 0.0045 M NaCl and 0.00045 M sodium citrate) in 10 mM sodium phosphate (pH 7.4), 0.1 mM EDTA.

While significant homology was detected between the recombinant DNA of HPV 44 clone 2 and most of HPV types 1 to 43 under non-stringent hybridization conditions, no homology was observed with any of HPV types 1-43 under stringent hybridization conditions, thus demonstrating that HPV 44 clone 2 represents a new HPV type.

2. Restriction Endonuclease Map

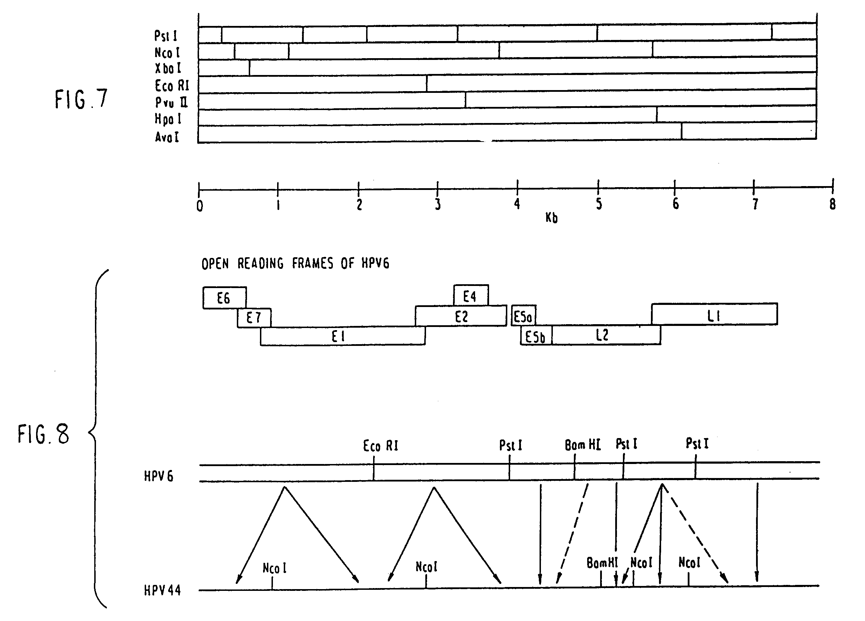

The restriction endonuclease map for HPV 44 is shown in Figure 7. The following restriction enzymes do not cut HPV 44 DNA: BglI, BglII, ClaI, EcoRV, HindIII, NruI, SalI, SphI, SstI and XhoI.

3. Genomic Organization

In order to demonstrate that the genome of HPV 44 had the same or similar open reading frame organization to HPV 6, the following hybridization studies were carried out. Purified DNA from HPV 44 clone 2 was subjected to Southern blotting using $^{32}$P "nick translated" fragments of HPV 6 DNA as a probe under non-stringent conditions and under stringent conditions as described above. More specifically, fragments of the BamHI-linearized HPV 6b clone were generated with EcoRI and PstI, then gel purified, nick translated with $^{32}$P and used to probe Southern blots containing NcoI restriction digests of the purified BamHI fragment of HPV 44 clone 2 DNA. The results, which are shown in Figure 8, demonstrate that the DNA of HPV 44 clone 2 has the same genomic organization as HPV 6.

## 4. Epidemiological Distribution

In order to demonstrate that hybridization probes prepared from HPV 44 clone 2 hybridizes efficiently under stringent conditions only to HPV 44 DNA, and that these hybridization probes can be used to detect genital lesions which contain HPV 44 and to distinguish such genital lesions from genital lesions which contain the DNA of other HPV types, e.g., 6, 11, 16, 18, 31 or 33, the DNA of a collection of cervical biopsies and cervical swabs containing exfoliated cells from the Washington, D.C. metropolitan area (including Maryland and Virginia) and Michigan and surrounding states, were analyzed by nucleic acid hybridization under stringent and non-stringent conditions, for the presence of specific HPV DNAs using probes specific for various HPV types, including probes specific for HPV 44. The biopsies were bisected with half of the specimens being processed for conventional light microscopy and the other half being frozen and stored at -20°C for molecular analysis. The tissues on which Southern blot hybridizations were performed were sectioned on a cryostat in order to obtain material for DNA extraction. Approximately every fifteenth section was stained with hematoxylin and eosin stain and examined microscopically in order to confirm that this tissue sample was comparable to the portion of the specimen analyzed by light microscopy. Exfoliated cervical cells were analyzed by standard cytological methods, e.g., pap smear, on paired samples, the other of which was used for DNA analysis.

High molecular weight DNA was prepared from the samples as described above. 1 to 10 $\mu$g of purified cellular DNA were digested with either PstI or BamHI and the digested samples were electrophoresed in 1.0% (w/v) agarose gels and transferred to nitrocellulose filters. Thereafter, hybridization was carried out under stringent conditions as described above with nick-translated $^{32}$P-labelled HPV DNAs from the types discussed above. Note, since the HPV DNAs were propagated in pT713 or related vectors, in order to minimize the possibility of reactivity with pT713 and related vector-like sequences in the tissue samples, all probes were electrophoretically purified to remove most of the associated vector sequences. Additional hybridization was also carried out in the presence of labelled pT713 or pBR322 and related vectors, to reveal any potential false positives due to the plasmid-like sequences in the tissue samples. The results are shown in Table 3 below. The results in Table 3 are graphically illustrated in Figure 9.

Table 3

Distribution of HPV Types in Cervical Biopsies from the Washington, D.C. Metropolitan Area and Michigan

| | Normal Squamous Epithelium[a] | Metaplastic Squamous Epithelium[b] | Condyloma | CIN I | CIN II | CIN III | Squamous Carcinoma | Endocervical Adenocarcinoma |
|---|---|---|---|---|---|---|---|---|
| HPV 6 | 5 (1.14%) | 1 (1.82%) | 17 (29.31%) | 6 (11.11%) | 9 (16.36%) | 1 (3.57%) | 0 | 0 |
| HPV 11 | 0 | 1 (1.82%) | 7 (12.07%) | 2 (3.70%) | 0 | 0 | 0 | 0 |
| HPV 16 | 10 (2.28%) | 1 (1.82%) | 4 (6.89%) | 11 (20.37%) | 22 (40.0%) | 19 (67.86%) | 19 (38.78%) | 2 (28.57%) |
| HPV 18 | 0 | 1 (1.82%) | 1 (1.72%) | 1 (1.85%) | 1 (1.82%) | 2 (7.14%) | 12 (24.40%) | 2 (28.57%) |
| HPV 31 | 4 (0.91%) | 0 | 1 (1.72%) | 8 (14.81%) | 7 (12.73%) | 3 (10.71%) | 3 (6.12%) | 0 |
| HPV 33 | 3 (0.68%) | 1 (1.82%) | 0 | 3 (5.56%) | 1 (1.82%) | 0 | 1 (2.04%) | 0 |
| HPV 44 | 5 (1.14%) | 1 (1.82%) | 2 (3.44%) | 1 (1.85%) | 1 (1.82%) | 0 | 0 | 0 |
| Total No. of HPV Positive Biopsies | 27 | 6 | 32 | 32 | 41 | 25 | 35 | 4 |
| Total No. of HPV Negative Biopsies | 412 | 49 | 27 | 21 | 14 | 3 | 13 | 2 |
| Total No. of Biopsies | 439 | 55 | 59 | 53 | 55 | 28 | 48 | 7 |

[a] These biopsies were from the portio of the cervix.

[b] These biopsies were from the transformation zone.

Table 3 and Figure 9 demonstrate that cervical biopsies containing HPV 44 can be distinguished from biopsies containing other HPV types, e.g., 6, 11, 16, 18, 31 or 33, not only by the criteria of degree of cross-hybridization in solution followed by hydroxyapatite chromatography but, also by the ability of an HPV 44 DNA probe to specifically detect and identify distinct populations of genital lesions, i.e., ones which contain HPV 44 DNA, as compared to ones which contain other HPV types. In addition, the results in Table 3 and Figure 9 show that the epidemiological distribution of HPV 44 DNA among cervical biopsies is distinct from that found for some other HPV types.

Example 4

(A) Cloning of HPV 56 DNA

The starting material employed was a vulvar condyloma biopsy obtained from the Washington, D.C. metropolitan area consisting of a few milligrams of tissue. Total DNA was purified as described in (Maniatis, T. et al, Molecular cloning: A laboratory manual, Cold Spring Harbor Laboratory, Cold Spring Harbor, New York (1982)). More specifically, the tissue was minced, then digested in 1.0 ml of 50 mM Tris-HCl, pH 8.0 containing 0.6% (w/v) sodium dodecyl sulfate and 50 μg/ml proteinase K at 37°C overnight. The resulting digest was extracted twice with 1.0 ml of phenol:chloroform (1:1 (v/v)). DNA was then precipitated from the

24

aqueous phase by addition of 2 volumes of 90% (v/v) ethanol. The precipitated DNA was redissolved in 10 mM Tris, 1.0 mM EDTA buffer, pH 8.0 (hereinafter "TE buffer") at a concentration of about 1.0 mg/ml.

The DNA was digested to completion with PstI, electrophoresed in 1.0% (w/v) agarose gels and DNA transferred to nitrocellulose filters as described in Southern, E.M., J. Mol. Biol., 98:503 (1975). The filters were then probed under non-stringent hybridization conditions ($T_m$-35°C) and stringent hybridization conditions ($T_m$-10°C) with DNA from HPV types 6, 11, 16, 18 and 31. Hybridization was performed overnight at 43°C in 1.0 M NaCl, 50 mM sodium phosphate buffer (pH 7.4), 1.0 mM EDTA, 2% (w/v) sodium dodecyl sulfate, 0.1% (w/v) gelatin, 50 $\mu$g/ml tRNA and 30% (v/v) formamide. Four 30 minute washes were performed at 55°C in 1.2X SSC (1X SSC is 0.15 M NaCl plus 0.015 M sodium citrate), 10 mM sodium phosphate (pH 7.4), 1.0 mM EDTA and 0.5% (w/v) sodium dodecyl sulfate. Hybridization was achieved under non-stringent conditions with HPV types 6, 11, 16, 18 and 31 but not under stringent hybridization conditions.

A portion of the resulting purified DNA was digested with EcoRI restriction endonuclease, which produced a fragment of 5.1 kb (which represents approximately 65% of the typical papillomavirus genome size). This fragment was cloned into the single EcoRI site of λ L47. Another portion of the resulting purified DNA was digested with BamHI restriction endonuclease, which produced a fragment of 5.9 kb (which represents approximately 75% of the typical papillomavirus genome size). This fragment was cloned into the single BamHI site of λ L47. More specifically, 2.0 $\mu$g of the resulting purified DNAs, and 2.0 $\mu$g of λ L47 DNA were cut with 10 units of EcoRI or 10 units of BamHI in a total volume of 50 $\mu$l of TE buffer for 1 hr at 37°C. The resulting reaction mixtures were then diluted with 400 $\mu$l of TE buffer and phenol extracted with an equal volume of phenol:chloroform as described above. The aqueous phases were then extracted with chloroform:isoamyl alcohol (24:1 (v/v)) and DNA from the aqueous phases was precipitated with 80% (v/v) ethanol and dried. The dried DNAs were then suspended in 10 $\mu$l of 1X ligase buffer comprising 66 mM Tris-HCl, 6.6 mM MgCl$_2$, 10 mM DTT and 1.0 mM ATP and incubated at 42°C for 2 hours to allow the λ arms to anneal. Next, 0.5 $\mu$l of T4 DNA ligase, i.e., about 1 unit, and 0.5 $\mu$l of 10 mM ATP, pH 7.0 was added to the reaction solutions and ligation was allowed to proceed at 12°C overnight.

Next, the ligation products were packaged to form infectious phage and used to infect E. coli. NM538. More specifically, a single colony of E. coli NM538 growing on an agarose plate comprising 10 g Tryptone and 5.0 g NaCl per liter (hereinafter "TN medium") was selected and grown overnight at 37°C in 20 ml of TN medium on a shaking platform (250 rpm) to early stationary phase. The cell culture was then diluted four fold with TN medium and grown for 3 hours. Next, the cells were harvested by centrifuging for 5 minutes at 5,000 rpm in a Sorvall HB-4 rotor and the resulting cell pellet was resuspended in 0.25 of the original volume, in 10 mM MgSO$_4$ and stored at 4°C.

The packaged infectious phages were prepared using a commercially available BRL Lambda In Vitro Packaging System (see Maniatis, T. et al, Molecular cloning: A laboratory manual, Cold Spring Harbor Laboratory, Cold Spring Harbor, New York (1982).

100 $\mu$l of an appropriate dilution of packaged phage in phage storage buffer comprising 0.5 M Tris-HCl (pH 8.0), 0.1 M NaCl, 0.01 M MgSO$_4$ and 0.01% (w/v) gelatin (Difco®) to give 1.5 x 10$^4$ plaques per 9 cm diameter plates, was added to 100 $\mu$l of E. coli NM538 prepared as described above in a 10-15 ml test tube, gently mixed and incubated at room temperature for 15 minutes. Then, the cell-phage solution was plated on Trypticase soy broth agar plates comprising 10 g of Trypticase soy broth, 5.0 g NaCl and 15 g agar per liter, which had been prepared at least one day in advance and which had been pre-warmed at 37°C. Thereafter, 3-5 ml of an agarose overlayer comprising 0.5% agarose (ultrapure, electrophoresis grade) dissolved in 10 mM MgSO$_4$, which had been heated in a microwave oven until the solution boiled and then cooled to 45°C before use, was placed over the plated cells-phage. After the agarose had solidified, the plates were transferred to a 37°C forced air incubator with good circulation with the lids of the plates cracked for 30 minutes and then the lids were closed and the plates inverted. After 8-12 hours, plaques became apparent.

Infection resulted in confluent lysis of bacteria on the plates. Recombinant phage carrying HPV DNA were localized by performing "plaque lifts" as described by Benton, W.D. et al, Science, 196:180 (1977). More specifically, confluent lysed plates were placed at 4°C for 1 hour to harden the agarose. Then, an appropriately sized piece of nitrocellulose filter was placed onto each plate by bowing it in the middle, touching the center of the plate and working the contact points toward the edge. Then, four assymetric holes were punched through the nitrocellulose filter and the agar with a small gauge needle and the positions of the holes were marked on the bottom of the plate with a permanent marker. This allowed the nitrocellulose filter and any other areas containing positive signals to be referenced to corresponding positions on the plates. After 10 minutes, the nitrocellulose filters were removed and the DNA was denatured by placing the nitrocellulose filters, plaque side facing upwards, into a dish containing 200 ml of 0.5 M NaOH, 2.0 M NaCl for 1 minute. The nitrocellulose filters were then neutralized by immersion in 500 ml of 0.5 M Tris-HCl, 2.0 M NaCl, pH 7.5 for 5 minutes. Next, the filters were rinsed in 6X SSC comprising 0.9 M NaCl, 0.09 M sodium citrate for 1 minute, dried on Whatman® 3 MM paper and then baked for 30 minutes at 80°C under vacuum.

Thereafter, non-stringent hybridization using HPV 16 DNA labelled with $^{32}$P by "nick translation" as a probe was carried out on the DNA isolated from the lifted plaques (see Rigby, P.J.W. et al, J. Mol. Biol., 113:237 (1977) and Maniatis, T. et al, Molecular cloning: A laboratory manual, Cold Spring Harbor Laboratory, Cold Spring Harbor, New York (1982)) followed by washing and autoradiography. More specifically, hybridization was performed at 41°C in a solution comprising 1.0 M NaCl, 28% (v/v) formamide, 50 mM N-Tris (hydroxymethyl)-methyl-2-aminoethane sulfonic acid (hereinafter "TES"), 10X Denhardt solution, 0.1 mM EDTA and 10 mM sodium phosphate (pH 7.4). Then a non-stringent wash was carried out at 52°C using 1.1X SSC (comprising 0.165 M NaCl and 0.0165 M sodium citrate) in 10 mM sodium phosphate (pH 7.4), 0.1 mM EDTA.

By correspondence with the sites of radioactive exposure, a region of the plate containing phage, which contained DNA that hybridized to HPV 16 DNA, was excised and used to reinfect E. coli NM538 as described above. Localization of phage plaques containing HPV DNA was accomplished by repeating the above procedure. Two plaques were identified from among the 1.5 x 10$^5$ plaques screened using the EcoRI digested DNA. Both clones had identical restriction maps. One of the clones was chosen for further studies and was designated HPV 56 clone 1A. One plaque was identified from among the 5 x 10$^4$ plaques screened using the BamHI digested DNA and was designated HPV 56 clone 1B.

The HPV DNAs of HPV 56 clones 1A and 1B were then digested with EcoRI or BamHI, respectively, and subcloned in the single EcoRI or BamHI site of pT713. The resulting recombinant DNAs were designated HPV 56 clones 2A and 2B. HPV 56 clones 2A and 2B have been deposited at the American Type Culture Collection under ATCC Nos. 40341 and 40379, respectively.

(B) Characterization of HPV C57 DNA

1. Hybridization Studies

Hybridization studies were carried out on HPV 56 clones 2A and 2B DNA to demonstrate that HPV 56 clones 2A and 2B represented a new HPV type.

More specifically, $^{32}$P "nick translated" DNA prepared from HPV 56 clones 2A and 2B was hybridized by Southern blotting under stringent conditions to 5.0 ng of DNA from HPV Types 1 to 45. DNAs from HPV Types 1 to 45 were obtained from Dr. Gerard Orth of the Institut Pasteur, Paris, France, the assignor of HPV type designations, Dr. Ethel-Michelle de Villiers of the Papilloma Reference Center in Heidelberg, West Germany, and from Life Technologies, Inc., in pre-immobilized form on nitrocellulose filters. More specifically, hybridization was performed at 41°C in a solution comprising 1.0 M NaCl, 28% (v/v) formamide, 50 mM N-Tris TES, 10X Denhardt solution, 0.5 mM EDTA and 20 mM sodium phosphate (pH 7.4). Then, a stringent wash was carried out at 65°C using 0.03X SSC (comprising 0.0045 M NaCl and 0.00045 M sodium citrate) in 10 mM sodium phosphate (pH 7.4), 0.1 mM EDTA.

While significant homology was detected between the recombinant DNA of HPV 56 clones 2A and 2B and most of HPV types 1 to 45 under non-stringent hybridization conditions, no homology was observed with any of HPV types 1-45 under stringent hybridization conditions, thus demonstrating that HPV 56 clones 2A and 2B represent a new HPV type.

2. Restriction Endonuclease Map

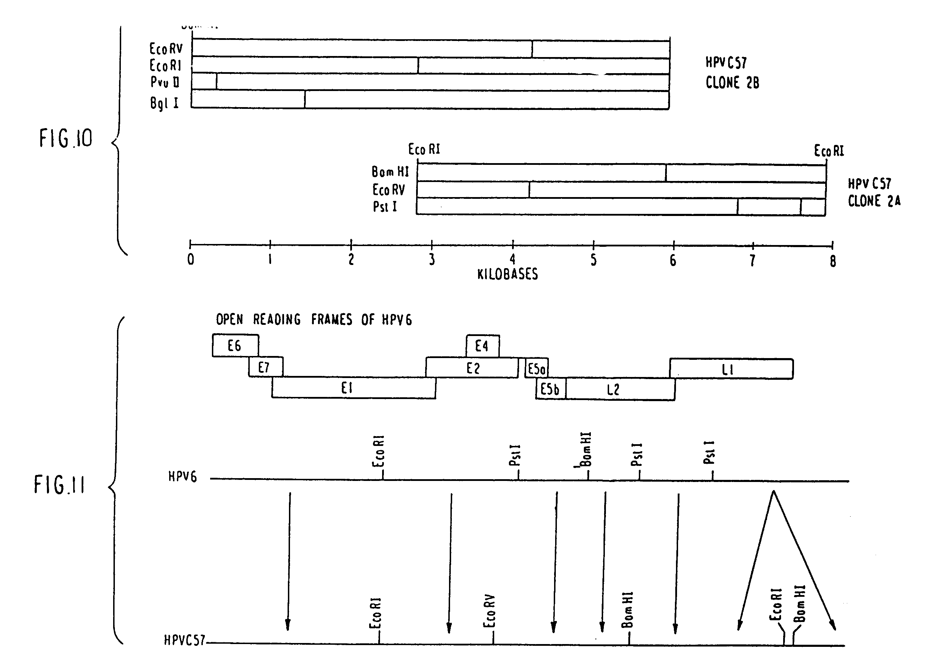

The restriction endonuclease maps for HPV 56 clones 2A and 2B are shown in Figure 10. The following restriction enzymes do not cut HPV 56 DNA: BglII, NcoI, HindIII, SalI, SstI and XbaI.

3. Genomic Organization

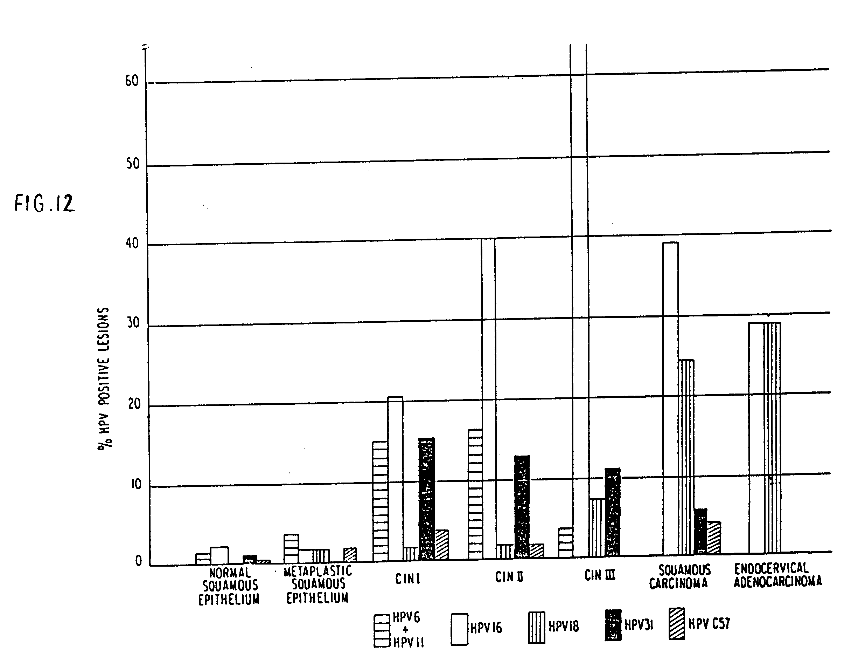

In order to demonstrate that the genome of HPV 56 had the same or similar open reading frame organization to HPV 6, the following hybridization studies were carried out. Purified DNA from HPV 56 clones 2A and 2B was subjected to Southern blotting using $^{32}$P "nick translated" fragments of HPV 6 DNA as a probe under non-stringent conditions and under stringent conditions as described above. More specifically, fragments of the BamHI-linearized HPV 6b clone were generated with EcoRI and PstI, then gel purified, nick translated with $^{32}$P and used to probe Southern blots containing BamHI plus EcoRV restriction digests of the purified EcoRI fragment of HPV 56 clone 2A DNA and EcoRI plus EcoRV restriction digests of the purified BamHI fragment of HPV 56 clone 2B. The results, which are shown in Figure 11, demonstrate that the DNA of HPV 56 clones 2A and 2B has the same genomic organization as HPV 6.

4. Epidemiological Distribution

In order to demonstrate that hybridization probes prepared from HPV 56 clones 2A and 2B hybridize efficiently under stringent conditions only to HPV 56 DNA, and that these hybridization probes can be used to detect genital lesions which contain HPV 56 and to distinguish such genital lesions from genital lesions which contain the DNA of other HPV types, e.g., 6, 11, 16, 18, 31 or 33, the DNA of a collection of cervical biopsies and cervical swabs containing exfoliated cells from the Washington, D.C. metropolitan area (including Maryland and Virginia) and Michigan and surrounding states, were analyzed by nucleic acid hybridization under stringent and non-stringent conditions, for the presence of specific HPV DNAs using probes specific for various HPV types, including probes specific for HPV 56. The biopsies were bisected with half of the specimens being processed for conventional light microscopy and the other half being frozen and stored at -20°C for molecular analysis. The tissues on which Southern blot hybridizations were performed were sectioned on a cryostat in order to obtain material for DNA extraction. Approximately every fifteenth section was stained with hematoxylin and eosin stain and examined microscopically in order to confirm that this tissue sample was comparable to the portion of the specimen analyzed by light microscopy. Exfoliated cervical cells were analyzed by standard cytological methods, e.g., pap smear, on paired samples, the other of which was used for DNA analysis.

High molecular weight DNA was prepared from the samples as described above. 1 to 10 $\mu$g of purified cellular DNA were digested with either PstI or BamHI and the digested samples were electrophoresed in 1.0% (w/v) agarose gels and transferred to nitrocellulose filters. Thereafter, hybridization was carried out under stringent conditions as described above with nick-translated $^{32}$P-labelled HPV DNAs from the types discussed above (for HPV C57 DNA, a mixture of HPV DNA from HPV 56 clones 2A and 2B was employed). Note, since the HPV DNAs were propagated in pT713 or related vectors, in order to minimize the possibility of reactivity with pT713 and related vector-like sequences in the tissue samples, all probes were electrophoretically purified to remove most of the associated vector sequences. Additional hybridization was also carried out in the presence of labelled pT713 or pBR322 and related vectors, to reveal any potential false positives due to the plasmid-like sequences in the tissue samples. The results are shown in Table 4 below. The results in Table 4 are graphically illustrated in Figure 12.

**Table 4**

Distribution of HPV Types in Cervical Biopsies from the Washington, D.C. Metropolitan Area and Michigan

| | Normal Squamous Epithelium[a] | Metaplastic Squamous Epithelium[b] | Condyloma | CIN I | CIN II | CIN III | Squamous Carcinoma | Endocervical Adenocarcinoma |
|---|---|---|---|---|---|---|---|---|
| HPV 6 | 5 (1.14%) | 1 (1.82%) | 17 (29.31%) | 6 (11.11%) | 9 (16.36%) | 1 (3.57%) | 0 | 0 |
| HPV 11 | 0 | 1 (1.82%) | 7 (12.07%) | 2 (3.70%) | 0 | 0 | 0 | 0 |
| HPV 16 | 10 (2.28%) | 1 (1.82%) | 4 (6.89%) | 11 (20.37%) | 22 (40.0%) | 19 (67.86%) | 19 (38.78%) | 2 (28.57%) |
| HPV 18 | 0 | 1 (1.82%) | 1 (1.72%) | 1 (1.85%) | 1 (1.82%) | 2 (7.14%) | 12 (24.40%) | 2 (28.57%) |
| HPV 31 | 4 (0.91%) | 0 | 1 (1.72%) | 8 (14.81%) | 7 (12.75%) | 3 (10.71%) | 3 (6.12%) | 0 |
| HPV 33 | 3 (0.68%) | 1 (1.82%) | 0 | 3 (5.56%) | 1 (1.82%) | 0 | 1 (2.04%) | 0 |
| HPV 56 | 2 (0.46%) | 1 (1.82%) | 2 (3.39%) | 2 (3.70%) | 1 (1.82%) | 0 | 2 (4.08%) | 0 |
| Total No. of HPV Positive Biopsies | 24 | 6 | 32 | 33 | 41 | 25 | 37 | 4 |
| Total No. of HPV Negative Biopsies | 412 | 49 | 27 | 21 | 14 | 3 | 13 | 2 |
| Total No. of Biopsies | 436 | 55 | 59 | 54 | 55 | 28 | 50 | 6 |

a These biopsies were from the portio of the cervix.

b These biopsies were from the transformation zone.

Table 4 and Figure 12 demonstrate that cervical biopsies containing HPV 56 can be distinguished from biopsies containing other HPV types, e.g., 6, 11, 16, 18, 31 or 33, not only by the criteria of degree of cross-hybridization in solution followed by hydroxyapatite chromatography but, also by the ability of an HPV 56 DNA probe to specifically detect and identify distinct populations of genital lesions, i.e., ones which contain HPV 56 DNA, as compared to ones which contain other HPV types. In addition, the results in Table 4 and Figure 12 show that the epidemiological distribution of HPV 56 DNA among cervical biopsies is distinct from that found for some other HPV types.

**Claims**

**Claims for the following Contracting States : AT, BE, CH, DE, FR, GB, GR, IT, LI, LU, NL, SE**

1. A recombinant DNA of HPV 35, HPV 43, HPV 44 or HPV 56 comprising a cloning vector and HPV 35 DNA or fragments thereof; HPV 43 DNA or fragments thereof; HPV 44 DNA or fragments thereof; or HPV 56 DNA or fragments thereof, respectively.

2. The recombinant DNA as claimed in Claim 1, wherein said cloning vector is selected from the group consisting of pBR322, pUC11, λ charon, λ L47, M13 derived bacteriophage, pZIP-Neo SV [X1], pBKTK-1, pT712 and pT713.

3. The recombinant DNA as claimed in Claim 1, wherein said fragments are about 15 to about 8000 base pairs in size.

4. The recombinant DNA as claimed in Claim 3, wherein said fragments are about 300 to about 800 base pairs in size.

5. The recombinant DNA as claimed in Claim 1, wherein said recombinant DNA has the identifying characteristics of HPV 35 clones 2A and 2B (ATCC No. 40330 and ATCC No. 40331, respectively); HPV 43 clones 2A and 2B (ATCC No. 40338 and ATCC No. 40339, respectively); HPV 44 clone 2 (ATCC No. 40353); or HPV 56 clones 2A and 2B (ATCC No. 40341 and ATCC No. 40379, respectively).

6. The recombinant DNA as claimed in Claim 1, wherein the HPV DNA of said recombinant DNA is labelled with a marker.

7. The recombinant DNA as claimed in Claim 6, wherein said marker is a radioactive marker.

8. The recombinant DNA as claimed in Claim 7, wherein said marker is a radioactive marker selected from the group consisting of $^{32}$P, $^{14}$C, $^{3}$H, $^{125}$I and $^{35}$S.

9. The recombinant DNA as claimed in Claim 6, wherein said marker is a non-radioactive marker selected from the group consisting of biotin, an enzyme and a fluorescent molecule.

10. The recombinant DNA as claimed in Claim 9, wherein said enzyme is selected from the group consisting of alkaline phosphatase and horseradish peroxidase.

11. The recombinant DNA as claimed in Claim 9, wherein said fluorescent molecule is selected from the group consisting of fluorescein and rhodamine.

12. Pure HPV 35 DNA or fragments thereof; pure HPV 43 DNA or fragments thereof; pure HPV 44 DNA or fragments thereof; or pure HPV 56 DNA or fragments thereof.

13. The pure HPV DNA as claimed in Claim 12, wherein said HPV DNA or fragments thereof are produced biologically.

14. The pure HPV DNA as claimed in Claim 12, wherein said HPV DNA or fragments thereof are produced chemically.

15. The pure HPV DNA as claimed in Claim 12, wherein said fragments are about 15 to about 8000 bases or base pairs in size.

16. The pure HPV DNA as claimed in Claim 15, wherein said fragments are about 300 to about 800 bases or base pairs in size.

17. Pure HPV 35 RNA or fragments thereof; pure HPV 43 RNA or fragments thereof; pure HPV 44 RNA or fragments thereof; or pure HPV 56 RNA or fragments thereof.

18. The pure HPV RNA as claimed in Claim 17, wherein said HPV RNA or fragments thereof are produced biologically.

19. The pure HPV RNA as claimed in Claim 17, wherein said HPV RNA or fragments thereof are produced chemically.

20. The pure HPV RNA as claimed in Claim 17, wherein said fragments are about 15 to about 8000 bases or base pairs in size.

21. The pure HPV RNA as claimed in Claim 20, wherein said fragments are about 300 to about 800 bases or base pairs in size.

22. An HPV hybridization probe comprising a member selected from the group consisting of (i) HPV 35 DNA or fragments thereof labelled with a marker, (ii) HPV 35 RNA or fragments thereof labelled with a marker; (iii) HPV 43 DNA or fragments thereof labelled with a marker, (iv) HPV 43 RNA or fragments thereof labelled with a marker; (v) HPV 44 DNA or fragments thereof labelled with a marker, (vi) HPV 44 RNA or fragments thereof labelled with a marker; (vii) HPV 56 DNA or fragments thereof labelled with a marker and (viii) HPV 56 RNA or fragments thereof labelled with a marker.

23. The HPV hybridization probe as claimed in Claim 22, wherein said fragments are about 15 to about 8000 bases or base pairs in size.

24. The HPV hybridization probe as claimed in Claim 23, wherein said fragments are about 300 to about 800 bases or base pairs in size.

25. The HPV hybridization probe as claimed in Claim 24, wherein said marker is a radioactive marker.

26. The HPV hybridization probe as claimed in Claim 25, wherein said marker is a radioactive marker selected from the group consisting of $^{32}$P, $^{14}$C, $^{3}$H, $^{125}$I and $^{35}$S.

27. The HPV hybridization probe as claimed in Claim 22, wherein said marker is a non-radioactive marker selected from the group consisting of biotin, an enzyme and a fluorescent molecule.

28. The HPV hybridization probe as claimed in Claim 27, wherein said enzyme is selected from the group consisting of alkaline phosphatase and horseradish peroxidase.

29. The HPV hybridization probe as claimed in Claim 27, wherein said fluorescent molecule is selected from the group consisting of fluorescein and rhodamine.

30. An HPV hybridization probe composition comprising
    (a) a member selected from the group consisting of (i) HPV 35 DNA or fragments thereof labelled with a marker and (ii) HPV 35 RNA or fragments thereof labelled with a marker and,
    (b) DNA or RNA or fragments thereof of at least one other HPV type labelled with a marker; or
    (a') a member selected from the group consisting of (i) HPV 43 DNA or fragments thereof labelled with a marker and (ii) HPV 43 RNA or fragments thereof labelled with a marker and,
    (b') DNA or RNA or fragments thereof of at least one other HPV type labelled with a marker; or
    (a'') a member selected from the group consisting of (i) HPV 44 DNA or fragments thereof labelled with a marker and (ii) HPV 44 RNA or fragments thereof labelled with a marker and,
    (b'') DNA or RNA or fragments thereof of at least one other HPV type labelled with a marker; or
    (a''') a member selected from the group consisting of (i) HPV 56 DNA or fragments thereof labelled with a marker and (ii) HPV 56 RNA or fragments thereof labelled with a marker and,
    (b''') DNA or RNA or fragments thereof of at least one other HPV type labelled with a marker.

31. The HPV hybridization probe composition as claimed in Claim 30, wherein said fragments are about 15 to about 8000 bases or base pairs in size.

32. The HPV hybridization probe composition as claimed in Claim 31, wherein said fragments are about 300 to about 800 bases or base pairs in size.

33. The HPV hybridization probe composition as claimed in Claim 30, wherein said marker is a radioactive marker.

34. The HPV hybridization probe composition as claimed in Claim 33, wherein said marker is a radioactive marker selected from the group consisting of $^{32}$P, $^{14}$C, $^{3}$H, $^{125}$I and $^{35}$S.

35. The HPV hybridization probe composition as claimed in Claim 30, wherein said marker is a non-radioactive marker selected from the group consisting of biotin, an enzyme and a fluorescent molecule.

36. The HPV hybridization probe composition as claimed in Claim 35, wherein said enzyme is selected from the group consisting of alkaline phosphatase and horseradish peroxidase.

37. The HPV hybridization probe composition as claimed in Claim 35, wherein said fluorescent molecule is selected from the group consisting of fluorescein and rhodamine.

38. The HPV hybridization probe composition as claimed in Claim 30, wherein said other HPV type is at least one member selected from the group consisting of HPV 6, HPV 11, HPV 16, HPV 18 and HPV 31.

39. The HPV hybridization probe composition as claimed in Claim 38, wherein said other HPV type is at least one member selected from the group consisting of HPV 16, HPV 18 and HPV 31.

40. The HPV hybridization probe composition as claimed in Claim 39, wherein said other HPV type is HPV 16, HPV 18 and HPV 31.

41. A method for detecting HPV DNA or RNA comprising:
    (1) carrying out hybridization, under non-stringent conditions, with
        (a) a member selected from the group consisting of
            (i) HPV 35 DNA or fragments thereof labelled with a marker; HPV 43 DNA or fragments thereof labelled with a marker; HPV 44 DNA or fragments thereof labelled with a marker; or HPV 56 DNA or fragments thereof labelled with a marker, and
            (ii) HPV 35 RNA or fragments thereof labelled with a marker; HPV 43 RNA or fragments thereof labelled with a marker; HPV 44 RNA or fragments thereof labelled with a marker; or HPV 56 RNA or fragments thereof labelled with a marker;
        (b) an unknown sample of DNA or RNA, and
    (2) assaying for the presence of cross-hybridization so as to detect HPV DNA or RNA in said sample.

42. The method as claimed in Claim 41, wherein said unknown sample of DNA or RNA is derived from a genital, throat, oral or skin lesion.

43. The method as claimed in Claim 42, wherein said unknown sample of DNA or RNA is derived from a genital lesion.

44. The method as claimed in Claim 43, wherein said unknown sample of DNA or RNA derived from a genital lesion is obtained by biopsying an epithelial lesion, scraping the cervix or by swabbing the cervix to obtain exfoliated cells or is DNA derived from a genital lesion which has been cloned in a cloning vector.

45. The method as claimed in Claim 41, wherein said fragments are about 15 to about 8000 bases or base pairs in size.

46. The method as claimed in Claim 45, wherein said fragments are about 300 to about 800 bases or base pairs in size.

47. The method as claimed in Claim 41, wherein hybridization is also carried out with
    (c) at least one of: a member selected from the group consisting of (i) HPV 6 DNA or fragments thereof labelled with a marker and (ii) HPV 6 RNA or fragments thereof labelled with a marker; a member selected from the group consisting of (i) HPV 11 DNA or fragments thereof labelled with a marker and (ii) HPV 11 RNA or fragments thereof labelled with a marker; a member selected from the group consisting of (i) HPV 16 DNA or fragments thereof labelled with a marker and (ii) HPV 16 RNA or fragments thereof labelled with a marker; and a member selected from the group consisting of (i) HPV 18 DNA or fragments thereof labelled with a marker and (ii) HPV 18 RNA or fragments thereof labelled with a marker.

**48.** The method as claimed in Claim 47, wherein said other type is at least one member selected from the group consisting of HPV 16 and HPV 18.

**49.** The method as claimed in Claim 48, wherein said other HPV type is HPV 16 and HPV 18.

**50.** The method as claimed in Claim 41, wherein said marker is a radioactive marker.

**51.** The method as claimed in Claim 50, wherein said marker is a radioactive marker selected from the group consisting of $^{32}$P, $^{14}$C, $^{3}$H, $^{125}$I and $^{35}$S.

**52.** The method as claimed in Claim 41, wherein said marker is a non-radioactive marker selected from the group consisting of biotin, an enzyme and a fluorescent molecule.

**53.** The method as claimed in Claim 52, wherein said enzyme is selected from the group consisting of alkaline phosphatase and horseradish peroxidase.

**54.** The method as claimed in Claim 52, wherein said fluorescent molecule is selected from the group consisting of fluorescein and rhodamine.

**55.** The method as claimed in Claim 41, wherein said cross-hybridization produces DNA-DNA hybrids.

**56.** The method as claimed in Claim 41, wherein said cross-hybridization produces DNA-RNA hybrids.

**57.** A method for detecting HPV 35 DNA or RNA; HPV 43 DNA or RNA; HPV 44 DNA or RNA; or HPV 56 DNA or RNA comprising:
  (1) carrying out hybridization, under stringent conditions, with
    (a) a member selected from the group consisting of
      (i) HPV 35 DNA or fragments thereof labelled with a marker; HPV 43 DNA or fragments thereof labelled with a marker; HPV 44 DNA or fragments thereof labelled with a marker; or HPV 56 DNA or fragments thereof labelled with a marker, respectively, and
      (ii) HPV 35 RNA or fragments thereof labelled with a marker; HPV 43 RNA or fragments thereof labelled with a marker; HPV 44 RNA or fragments thereof labelled with a marker; or HPV 56 RNA or fragments thereof labelled with a marker, respectively;
    (b) an unknown sample of DNA or RNA, and
  (2) assaying for the presence of cross-hybridization so as to detect HPV 35 DNA or RNA; HPV 43 DNA or RNA; HPV 44 DNA or RNA; or HPV 56 DNA or RNA, respectively, in said sample.

**58.** The method as claimed in Claim 57, wherein said unknown sample of DNA or RNA is derived from a genital, throat, oral or skin lesion.

**59.** The method as claimed in Claim 58, wherein said unknown sample of DNA or RNA is derived from a genital lesion.

**60.** The method as claimed in Claim 59, wherein said unknown sample of DNA or RNA derived from a genital lesion is obtained by biopsying an epithelial lesion, scraping the cervix or by swabbing the cervix to obtain exfoliated cells or is DNA derived from a genital lesion which has been cloned in a cloning vector.

**61.** The method as claimed in Claim 57, wherein said fragments are about 15 to about 8000 bases or base pairs in size.

**62.** The method as claimed in Claim 61, wherein said fragments are about 300 to about 800 bases or base pairs in size.

**63.** The method as claimed in Claim 57, wherein said HPV 35 DNA, HPV 43 DNA, HPV 44 DNA or HPV 56 DNA comprise the HPV 35 genome; the HPV 43 genome; the HPV 44 genome; or the HPV 56 genome, respectively.

64. The method as claimed in Claim 57, wherein said marker is a radioactive marker.

65. The method as claimed in Claim 64, wherein said marker is a radioactive marker selected from the group consisting of $^{32}$P, $^{14}$C, $^3$H, $^{125}$I and $^{35}$S.

66. The method as claimed in Claim 57, wherein said marker is a non-radioactive marker selected from the group consisting of biotin, an enzyme and a fluorescent molecule.

67. The method as claimed in Claim 66, wherein said enzyme is selected from the group consisting of alkaline phosphatase and horseradish peroxidase.

68. The method as claimed in Claim 66, wherein said fluorescent molecule is selected from the group consisting of fluorescein and rhodamine.

69. The method as claimed in Claim 57, wherein said cross-hybridization produces DNA-DNA hybrids.

70. The method as claimed in Claim 57, wherein said cross-hybridization produces DNA-RNA hybrids.

71. A method for detecting HPV 35 DNA or RNA; HPV 43 DNA or RNA; HPV 44 DNA or RNA; or HPV 56 DNA or RNA comprising:
    (1) carrying out hybridization, under stringent conditions, with
        (a) a first fraction of DNA or RNA derived from each genital lesion of a sampling of genital lesions, which sampling shows an epidemiological progression to cervical cancer, and
        (b) a member selected from the group consisting of
            (i) HPV 35 DNA or fragments thereof labelled with a marker; HPV 43 DNA or fragments thereof labelled with a marker; HPV 44 DNA or fragments thereof labelled with a marker; or HPV 56 DNA or fragments thereof labelled with a marker, respectively, and
            (ii) HPV 35 RNA or fragments thereof labelled with a marker; HPV 43 RNA or fragments thereof labelled with a marker; HPV 44 RNA or fragments thereof labelled with a marker; or HPV 56 RNA or fragments thereof labelled with a marker, respectively;
    (2) carrying out hybridization, under stringent conditions, with
        (a) a second fraction of DNA or RNA derived from each genital lesion of said sampling of genital lesions, and
        (b) an unknown sample of DNA or RNA derived from a genital lesion which has been labelled with a marker;
    (3) comparing the epidemiological distribution of cross-hybridization obtained in Step (1) with that obtained in Step (2) and the cross-hybridization of the DNA from each lesion which comprises said epidemiological distribution so as to detect HPV 35 DNA or RNA; HPV 43 DNA or RNA; HPV 44 DNA or RNA; or HPV 56 DNA or RNA, respectively, in said sample.

72. The method as claimed in Claim 71, wherein said fragments are about 15 to about 8000 bases or base pairs in size.

73. The method as claimed in Claim 72, wherein said fragments are about 300 to about 800 bases or base pairs in size.

74. The method as claimed in Claim 71, wherein said HPV 35 DNA, HPV 43 DNA, HPV 44 DNA, or HPV 56 DNA comprise substantially all of the HPV 35 genome; the HPV 43 genome; the HPV 44 genome; or the HPV 56 genome, respectively.

75. The method as claimed in Claim 71, wherein said marker is a radioactive marker.

76. The method as claimed in Claim 75, wherein said marker is a radioactive marker selected from the group consisting of $^{32}$P, $^{14}$C, $^3$H, $^{125}$I and $^{35}$S.

77. The method as claimed in Claim 71, wherein said marker is a non-radioactive marker selected from the group consisting of biotin, an enzyme and a fluorescent molecule.

**78.** The method as claimed in Claim 77, wherein said enzyme is selected from the group consisting of alkaline phosphatase and horseradish peroxidase.

**79.** The method as claimed in Claim 77, wherein said fluorescent molecule is selected from the group consisting of fluorescein and rhodamine.

**80.** The method as claimed in Claim 71, wherein said unknown sample of DNA or RNA derived from a genital lesion is obtained by biopsying an epithelial lesion, scraping the cervix or by swabbing the cervix to obtain exfoliated cells or is DNA derived from a genital lesion which has been cloned in a cloning vector.

**81.** The method as claimed in Claim 71, wherein said cross-hybridization produces DNA-DNA hybrids.

**82.** The method as claimed in Claim 71, wherein said cross-hybridization produces DNA-RNA hybrids.

**Claims for the following Contracting State : ES**

**1.** A method for detecting HPV DNA or RNA comprising:
(1) carrying out hybridization, under non-stringent conditions, with
(a) a member selected from the group consisting of
(i) HPV 35 DNA or fragments thereof labelled with a marker; HPV 43 DNA or fragments thereof labelled with a marker; HPV 44 DNA or fragments thereof labelled with a marker; or HPV 56 DNA or fragments thereof labelled with a marker, and
(ii) HPV 35 RNA or fragments thereof labelled with a marker; HPV 43 RNA or fragments thereof labelled with a marker; HPV 44 RNA or fragments thereof labelled with a marker; or HPV 56 RNA or fragments thereof labelled with a marker;
(b) an unknown sample of DNA or RNA, and
(2) assaying for the presence of cross-hybridization so as to detect HPV DNA or RNA in said sample.

**2.** A method according to claim 1, wherein said HPV DNA has the identifying characteristics of HPV 35 clones 2A and 2B (ATCC No. 40330 and ATCC No. 40331, respectively); HPV 43 clones 2A and 2B (ATCC No. 40338 and ATCC No. 40339, respectively); HPV 44 clone 2 (ATCC No. 40353); or HPV 56 clones 2A and 2B (ATCC No. 40341 and ATCC No. 40379, respectively).

**3.** A method of claim 1, wherein said marker is a radioactive marker.

**4.** A method of claim 3, wherein said marker is a radioactive marker selected from the group consisting of $^{32}$P, $^{14}$C, $^{3}$H, $^{125}$I and $^{35}$S.

**5.** A method of claim 1, wherein said marker is a non-radioaktive marker selected from the group consisting of biotin, an enzyme and a fluorescent molecule.

**6.** A method of claim 5, wherein said enzyme is selected from the group consisting of alkaline phosphatase and horseradish peroxidase.

**7.** A method of claim 5, wherein said fluorescent molecule is selected from the group consisting of fluorescein and rhodamine.

**8.** A method of claim 1, wherein said HPV DNA or fragments thereof are produced biologically.

**9.** A method of claim 1, wherein said HPV DNA or fragments thereof are produced chemically.

**10.** A method of claim 1, wherein said unknown sample of DNA or RNA is derived from a genital, throat, oral or skin lesion.

**11.** A method of claim 10, wherein said unknown sample of DNA or RNA is derived from a genital lesion.

**12.** A method of claim 11, wherein said unknown sample of DNA or RNA derived from a genital lesion is obtained by biopsying an epithelial lesion, scraping the cervix or by swabbing the cervix to obtain exfoliated cells or is DNA derived from a genital lesion which has been cloned in a cloning vector.

**13.** A method of claim 1, wherein hybridization is also carried out with

(c) at least one of: a member selected from the group consisting of (i) HPV 6 DNA or fragments thereof labelled with a marker and (ii) HPV 6 RNA or fragments thereof labelled with a marker; a member selected from the group consisting of (i) HPV 11 DNA or fragments thereof labelled with a marker and (ii) HPV 11 RNA or fragments thereof labelled with a marker; a member selected from the group consisting of (i) HPV 16 DNA or fragments thereof labelled with a marker and (ii) HPV 16 RNA or fragments thereof labelled with a marker; and a member selected from the group consisting of (i) HPV 18 DNA or fragments thereof labelled with a marker and (ii) HPV 18 RNA or fragments thereof labelled with a marker.

**14.** A method of claim 13, wherein said other type is at least one member selected from the group consisting of HPV 16 and HPV 18.

**15.** A method of claim 14, wherein said other type is HPV 16 and HPV 18.

**16.** A method of claim 1, wherein said cross-hybridization produces DNA-DNA hybrids.

**17.** A method of claim 1, wherein said cross-hybridization produces DNA-RNA hybrids.

**18.** A method for detecting HPV 35 DNA or RNA; HPV 43 DNA or RNA; HPV 44 DNA or RNA; or HPV 56 DNA or RNA comprising:

(1) carrying out hybridization, under stringent conditions, with

(a) a member selected from the group consisting of

(i) HPV 35 DNA or fragments thereof labelled with a marker; HPV 43 DNA or fragments thereof labelled with a marker; HPV 44 DNA or fragments thereof labelled with a marker; or HPV 56 DNA or fragments thereof labelled with a marker, respectively, and

(ii) HPV 35 RNA or fragments thereof labelled with a marker; HPV 43 RNA or fragments thereof labelled with a marker; HPV 44 RNA or fragments thereof labelled with a marker; or HPV 56 RNA or fragments thereof labelled with a marker, respectively;

(b) an unknown sample of DNA or RNA, and

(2) assaying for the presence of cross-hybridization so as to detect HPV 35 DNA or RNA; HPV 43 DNA or RNA; HPV 44 DNA or RNA; or HPV 56 DNA or RNA, respectively, in said sample.

**19.** A method as claimed in claim 18, wherein said unknown sample of DNA or RNA is derived from a genital, throat, oral or skin lesion.

**20.** A method of claim 19, wherein said unknown sample of DNA or RNA is derived from a genital lesion.

**21.** A method of claim 20, wherein said unknown sample of DNA or RNA derived from a genital lesion is obtained by biopsying an epithelial lesion, scraping the cervix or by swabbing the cervix to obtain exfoliated cells or is DNA derived from a genital lesion which has been cloned in a cloning vector.

**22.** A method as claimed in claim 18, wherein said fragments are about 15 to about 8000 bases or base pairs in size.

**23.** A method as claimed in claim 22, wherein said fragments are about 300 to about 800 bases or base pairs in size.

**24.** A method as claimed in claim 18, wherein said HPV 35 DNA, HPV 43 DNA, HPV 44 DNA or HPV 56 DNA comprise the HPV 35 genome, the HPV 43 genome, the HPV 44 genome, or the HPV 56 genome, respectively.

**25.** A method of claim 18, wherein said marker is a radioactive marker.

26. A method of claim 25, wherein said marker is a radioactive marker selected from the group consisting of $^{32}$P, $^{14}$C, $^{3}$H, $^{125}$I, and $^{35}$S.

27. A method as claimed in claim 18, wherein said marker is a non-radioactive marker selected from the group consisting of biotin, an enzyme and a fluorescent molecule.

28. A method of claim 27, wherein said enzyme is selected from the group consisting of alkaline phosphatase and horseradish peroxidase.

29. A method as claimed in claim 27, wherein said fluorescent molecule is selected from the group consisting of fluorescein and rhodamine.

30. A method as claimed in claim 18, wherein said cross-hybridization produces DNA-DNA hybrids.

31. A method as claimed in claim 18, wherein said cross-hybridization produces DNA-RNA hybrids.

32. A method for detecting HPV 35 DNA or RNA; HPV 43 DNA or RNA; HPV 44 DNA or RNA; or HPV 56 DNA or RNA comprising:
   (1) carrying out hybridization, under stringent conditions, with
      (a) a first fraction of DNA or RNA derived from each genital lesion of a sampling of genital lesions, which sampling shows an epidemiological progression to cervical cancer, and
      (b) a member selected from the group consisting of
         (i) HPV 35 DNA or fragments thereof labelled with a marker; HPV 43 DNA or fragments thereof labelled with a marker; HPV 44 DNA or fragments thereof labelled with a marker; or HPV 56 DNA or fragments thereof labelled with a marker, respectively, and
         (ii) HPV 35 RNA or fragments thereof labelled with a marker; HPV 43 RNA or fragments thereof labelled with a marker; HPV 44 RNA or fragments thereof labelled with a marker; or HPV 56 RNA or fragments thereof labelled with a marker, respectively;
   (2) carrying out hybridization, under stringent conditions, with
      (a) a second fraction of DNA or RNA derived from each genital lesion of said sampling of genital lesions, and
      (b) an unknown sample of DNA or RNA derived from a genital lesion which has been labelled with a marker;
   (3) comparing the epidemiological distribution of cross-hybridization obtained in Step (1) with that obtained in Step (2) and the cross-hybridization of the DNA from each lesion which comprises said epidemiological distribution so as to detect HPV 35 DNA or RNA; HPV 43 DNA or RNA; HPV 44 DNA or RNA; or HPV 56 DNA or RNA, respectively, in said sample.

33. A method as claimed in claim 32, wherein said fragments are about 15 to about 8000 bases or base pairs in size.

34. A method as claimed in claim 33, wherein said fragments are about 300 to about 800 bases or base pairs in size.

35. A method as claimed in claim 32, wherein said HPV 35 DNA, HPV 43 DNA, HPV 44 DNA, or HPV 56 DNA comprise substantially all of the HPV 35 genome; the HPV 43 genome; the HPV 44 genome; or the HPV 56 genome, respectively.

36. A method as claimed in claim 32, wherein said marker is a radioactive marker.

37. A method as claimed in claim 36, wherein said marker is a radioactive marker selected from the group consisting of $^{32}$P, $^{14}$C, $^{3}$H, $^{125}$I and $^{35}$S.

38. A method as claimed in claim 32, wherein said marker is a non-radioactive marker selected from the group consisting of biotin, an enzyme and a fluorescent molecule.

39. A method as claimed in claim 38, wherein said enzyme is selected from the group consisting of alkaline phosphatase and horseradish peroxidase.

**40.** A method as claimed in claim 38, wherein said fluorescent molecule is selected from the group consisting of fluorescein and rhodamine.

**41.** A method as claimed in claim 32, wherein said unknown sample of DNA or RNA derived from a genital lesion is obtained by biopsying an epithelial lesion, scraping the cervix or by swabbing the cervix to obtain exfoliated cells or is DNA derived from a genital lesion which has been cloned in a cloning vector.

**42.** A method as claimed in claim 32, wherein said cross-hybridization produces DNA-DNA hybrids.

**43.** A method as claimed in claim 32, wherein said cross-hybridization produces DNA-RNA hybrids.

**Patentansprüche**

**Patentansprüche für folgende Vertragsstaaten : AT, BE, CH, DE, FR, GB, GR, IT, LI, LU, NL, SE**

**1.** Rekombinante DNA von HPV 35, HPV 43, HPV 44 oder HPV 56, umfassend einen Klonierungsvektor und HPV 35-DNA oder Fragmente davon; HPV 43-DNA oder Fragmente davon; HPV 44-DNA oder Fragmente davon; bzw. HPV 56-DNA oder Fragmente davon.

**2.** Rekombinante DNA nach Anspruch 1, wobei der Klonierungsvektor aus der aus pBR322, pUC11, lambda charon, lambda L47, aus M13 abgeleiteten Bakteriophagen, pZIP-Neo SV [X1], pBKTK-1, pT712 und pT713 bestehenden Gruppe ausgewählt ist.

**3.** Rekombinante DNA nach Anspruch 1, wobei die Fragmente eine Größe von etwa 15 bis etwa 8000 Basenpaaren aufweisen.

**4.** Rekombinante DNA nach Anspruch 3, wobei die Fragmente eine Größe von etwa 300 bis etwa 800 Basenpaaren aufweisen.

**5.** Rekombinante DNA nach Anspruch 1, wobei die rekombinante DNA die identifizierenden Merkmale der HPV 35-Klone 2A und 2B (ATCC Nr. 40330 bzw. ATCC Nr. 40331); der HPV 43-Klone 2A und 2B (ATCC Nr. 40338 bzw. ATCC Nr. 40339); des HPV 44-Klons 2 (ATCC Nr. 40353) oder der HPV 56-Klone 2A und 2B (ATCC Nr. 40341 bzw. ATCC Nr. 40379) aufweist.

**6.** Rekombinante DNA nach Anspruch 1, wobei die HPV-DNA der rekombinanten DNA mit einem Marker markiert ist.

**7.** Rekombinante DNA nach Anspruch 6, wobei es sich bei dem Marker um einen radioaktiven Marker handelt.

**8.** Rekombinante DNA nach Anspruch 7, wobei es sich bei dem Marker um einen radioaktiven Marker handelt, der aus der aus $^{32}$P, $^{14}$C, $^{3}$H, $^{125}$I und $^{35}$S bestehenden Gruppe ausgewählt ist.

**9.** Rekombinante DNA nach Anspruch 6, wobei es sich bei dem Marker um einen nicht-radioaktiven Marker handelt, der aus der aus Biotin, einem Enzym und einem fluoreszierenden Molekül bestehenden Gruppe ausgewählt ist.

**10.** Rekombinante DNA nach Anspruch 9, wobei das Enzym aus der aus alkalischer Phosphatase und Meerrettich-Peroxidase bestehenden Gruppe ausgewählt ist.

**11.** Rekombinante DNA nach Anspruch 9, wobei das fluoreszierende Molekül aus der aus Fluoreszein und Rhodamin bestehenden Gruppe ausgewählt ist.

**12.** Reine HPV 35-DNA oder Fragmente davon; reine HPV 43-DNA oder Fragmente davon; reine HPV 44-DNA oder Fragmente davon; bzw. reine HPV 56-DNA oder Fragmente davon.

**13.** Reine HPV-DNA nach Anspruch 12, wobei die HPV-DNA oder Fragmente davon biologisch hergestellt wurden.

**14.** Reine HPV-DNA nach Anspruch 12, wobei die HPV-DNA oder Fragmente davon chemisch hergestellt wurden.

**15.** Reine HPV-DNA nach Anspruch 12, wobei die Fragmente eine Größe von etwa 15 bis etwa 8000 Basen oder Basenpaaren aufweisen.

**16.** Reine HPV-DNA nach Anspruch 15, wobei die Fragmente eine Größe von etwa 300 bis etwa 800 Basen oder Basenpaaren aufweisen.

**17.** Reine HPV 35-RNA oder Fragmente davon; reine HPV 43-RNA oder Fragmente davon; reine HPV 44-RNA oder Fragmente davon; oder reine HPV 56-RNA oder Fragmente davon.

**18.** Reine HPV-RNA nach Anspruch 17, wobei die HPV-RNA oder Fragmente davon biologisch hergestellt wurden.

**19.** Reine HPV-RNA nach Anspruch 17, wobei die HPV-RNA oder Fragmente davon chemisch hergestellt wurden.

**20.** Reine HPV-RNA nach Anspruch 17, wobei die Fragmente eine Größe von etwa 15 bis etwa 8000 Basen oder Basenpaaren aufweisen.

**21.** Reine HPV-RNA nach Anspruch 20, wobei die Fragmente eine Größe von etwa 300 bis etwa 800 Basen oder Basenpaaren aufweisen.

**22.** HPV-Hybridisierungssonde, umfassend ein Mitglied der aus (i) mit einem Marker markierter HPV 35-DNA oder Fragmenten davon; (ii) mit einem Marker markierter HPV 35-RNA oder Fragmenten davon; (iii) mit einem Marker markierter HPV 43-DNA oder Fragmenten davon; (iv) mit einem Marker markierter HPV 43-RNA oder Fragmenten davon; (v) mit einem Marker markierter HPV 44-DNA oder Fragmenten davon; (vi) mit einem Marker markierter HPV 44-RNA oder Fragmenten davon; (vii) mit einem Marker markierter HPV 56-DNA oder Fragmenten davon und (viii) mit einem Marker markierter HPV 56-RNA oder Fragmenten davon bestehenden Gruppe.

**23.** HPV-Hybridisierungssonde nach Anspruch 22, wobei die Fragmente eine Größe von etwa 15 bis etwa 8000 Basen oder Basenpaaren aufweisen.

**24.** HPV-Hybridisierungssonde nach Anspruch 23, wobei die Fragmente eine Größe von etwa 300 bis etwa 800 Basen oder Basenpaaren aufweisen.

**25.** HPV-Hybridisierungssonde nach Anspruch 22, wobei es sich bei dem Marker um einen radioaktiven Marker handelt,

**26.** HPV-Hybridisierungssonde nach Anspruch 25, wobei es sich bei dem Marker um einen radioaktiven Marker handelt, der aus der aus $^{32}$P, $^{14}$C, $^{3}$H, $^{125}$I und $^{35}$S bestehenden Gruppe ausgewählt ist.

**27.** HPV-Hybridisierungssonde nach Anspruch 22, wobei es sich bei dem Marker um einen nicht-radioaktiven Marker handelt, der aus der aus Biotin, einem Enzym und einem fluoreszierenden Molekül bestehenden Gruppe ausgewählt ist.

**28.** HPV-Hybridisierungssonde nach Anspruch 27, wobei das Enzym aus der aus alkalischer Phosphatase und Meerrettich-Peroxidase bestehenden Gruppe ausgewählt ist.

**29.** HPV-Hybridisierungssonde nach Anspruch 27, wobei das fluoreszierende Molekül aus der aus Fluoreszein und Rhodamin bestehenden Gruppe ausgewählt ist.

**30.** HPV-Hybridisierungssonden-Zusammensetzung, umfassend
(a) ein Mitglied aus der aus (i) mit einem Marker markierter HPV 35-DNA oder Fragmenten davon und (ii) mit einem Marker markierter HPV 35-RNA oder Fragmenten davon bestehenden Gruppe und

38

(b) mit einem Marker markierte DNA oder RNA oder Fragmente davon mindestens eines anderen HPV-Typs; oder

(a') ein Mitglied aus der aus (i) mit einem Marker markierter HPV 43-DNA oder Fragmenten davon und (ii) mit einem Marker markierter HPV 43-RNA oder Fragmenten davon bestehenden Gruppe und

(b') mit einem Marker markierte DNA oder RNA oder Fragmente davon mindestens eines anderen HPV-Typs; oder

(a'') ein Mitglied aus der aus (i) mit einem Marker markierter HPV 44-DNA oder Fragmenten davon und (ii) mit einem Marker markierter HPV 44-RNA oder Fragmenten davon bestehenden Gruppe und

(b'') mit einem Marker markierte DNA oder RNA oder Fragmente davon mindestens eines anderen HPV-Typs; oder

(a''') ein Mitglied aus der aus (i) mit einem Marker markierter HPV 35-DNA oder Fragmenten davon und (ii) mit einem Marker markierter HPV 35-RNA oder Fragmenten davon bestehenden Gruppe und

(b''') mit einem Marker markierte DNA oder RNA oder Fragmente davon mindestens eines anderen HPV-Typs.

31. HPV-Hybridisierungssonden-Zusammensetzung nach Anspruch 30, wobei die Fragmente eine Größe von etwa 15 bis etwa 8000 Basen oder Basenpaaren aufweisen.

32. HPV-Hybridisierungssonden-Zusammensetzung nach Anspruch 31, wobei die Fragmente eine Größe von etwa 300 bis etwa 800 Basen oder Basenpaaren aufweisen.

33. HPV-Hybridisierungssonden-Zusammensetzung nach Anspruch 30, wobei es sich bei dem Marker um einen radioaktiven Marker handelt.

34. HPV-Hybridisierungssonden-Zusammensetzung nach Anspruch 33, wobei es sich bei dem Marker um einen radioaktiven Marker handelt, der aus der aus $^{32}$P, $^{14}$C, $^{3}$H, $^{125}$I und $^{35}$S bestehenden Gruppe ausgewählt ist.

35. HPV-Hybridisierungssonden-Zusammensetzung nach Anspruch 30, wobei es sich bei dem Marker um einen nicht-radioaktiven Marker handelt, der aus der aus Biotin, einem Enzym und einem fluoreszierenden Molekül bestehenden Gruppe ausgewählt ist.

36. HPV-Hybridisierungssonden-Zusammensetzung nach Anspruch 35, wobei das Enzym aus der aus alkalischer Phosphatase und Meerrettich-Peroxidase bestehenden Gruppe ausgewählt ist.

37. HPV-Hybridisierungssonden-Zusammensetzung nach Anspruch 35, wobei das fluoreszierende Molekül aus der aus Fluoreszein und Rhodamin bestehenden Gruppe ausgewählt ist.

38. HPV-Hybridisierungssonden-Zusammensetzung nach Anspruch 30, wobei es sich bei dem anderen HPV-Typ um mindestens ein Mitglied der aus HPV 6, HPV 11, HPV 16, HPV 18 und HPV 31 bestehenden Gruppe handelt.

39. HPV-Hybridisierungssonden-Zusammensetzung nach Anspruch 38, wobei es sich bei dem anderen HPV-Typ um mindestens ein Mitglied der aus HPV 16, HPV 18 und HPV 31 bestehenden Gruppe handelt.

40. HPV-Hybridisierungssonden-Zusammensetzung nach Anspruch 39, wobei es sich bei dem anderen HPV-Typ um HPV 16, HPV 18 und HPV 31 handelt.

41. Verfahren zum Nachweis von HPV-DNA oder -RNA, umfassend:

(1) Ausführung einer Hybridisierung unter nichtstringenten Bedingungen mit

(a) einem aus der folgenden Gruppe ausgewählten Mitglied:

(i) mit einem Marker markierte HPV 35-DNA oder Fragmente davon; mit einem Marker markierte HPV 43-DNA oder Fragmente davon; mit einem Marker markierte HPV 44-DNA oder Fragmente davon; oder mit einem Marker markierte HPV 56-DNA oder Fragmente davon und

(ii) mit einem Marker markierte HPV 35-RNA oder Fragmente davon; mit einem Marker markierte HPV 43-RNA oder Fragmente davon; mit einem Marker markierte HPV 44-RNA oder Fragmente davon; oder mit einem Marker markierte HPV 56-RNA oder Fragmente davon,
(b) und einer unbekannten Probe von DNA oder RNA, und

(2) Untersuchung auf das Auftreten von Kreuzhybridisierung, um HPV-DNA oder -RNA in der Probe nachzuweisen.

42. Verfahren nach Anspruch 41, wobei die unbekannte Probe von DNA oder RNA aus einer Genitalläsion, einer Läsion der Kehle, einer oralen Läsion oder einer Hautläsion stammt.

43. Verfahren nach Anspruch 42, wobei die unbekannte Probe von DNA oder RNA aus einer Genitalläsion stammt.

44. Verfahren nach Anspruch 43, wobei die unbekannte Probe von DNA oder RNA, die aus einer Genitalläsion stammt, durch Biopsie einer Epithelläsion, Ausschaben des Zervix oder Abtupfen des Zervix, um abgeschilferte Zellen zu erhalten, erhalten wird oder es sich um DNA handelt, die aus einer Genitalläsion stammt und in einen Klonierungsvektor kloniert worden ist.

45. Verfahren nach Anspruch 41, wobei die Fragmente eine Größe von etwa 15 bis etwa 8000 Basen oder Basenpaaren aufweisen.

46. Verfahren nach Anspruch 45, wobei die Fragmente eine Größe von etwa 300 bis etwa 800 Basen oder Basenpaaren aufweisen.

47. Verfahren nach Anspruch 41, wobei die Hybridisierung auch mit
(c) mindestens einem der folgenden Bestandteile durchgeführt wird: einem Mitglied aus der aus (i) mit einem Marker markierter HPV 6-DNA oder Fragmenten davon und (ii) mit einem Marker markierter HPV 6-RNA oder Fragmenten davon bestehenden Gruppe; einem Mitglied aus der aus (i) mit einem Marker markierter HPV 11-DNA oder Fragmenten davon und (ii) mit einem Marker markierter HPV 11-RNA oder Fragmenten davon bestehenden Gruppe; einem Mitglied aus der aus (i) mit einem Marker markierter HPV 16-DNA oder Fragmenten davon und (ii) mit einem Marker markierter HPV 16-RNA oder Fragmenten davon bestehenden Gruppe; und einem Mitglied aus der aus (i) mit einem Marker markierter HPV 18-DNA oder Fragmenten davon und (ii) mit einem Marker markierter HPV 18-RNA oder Fragmenten davon bestehenden Gruppe.

48. Verfahren nach Anspruch 47, wobei es sich bei dem weiteren Typ um mindestens ein Mitglied der aus HPV 16 und HPV 18 bestehenden Gruppe handelt.

49. Verfahren nach Anspruch 48, wobei es sich bei dem weiteren Typ um HPV 16 und HPV 18.

50. Verfahren nach Anspruch 41, wobei es sich bei dem Marker um einen radioaktiven Marker handelt.

51. Verfahren nach Anspruch 50, wobei es sich bei dem Marker um einen radioaktiven Marker handelt, der aus der aus $^{32}$P, $^{14}$C, $^{3}$H, $^{125}$I und $^{35}$S bestehenden Gruppe ausgewählt ist.

52. Verfahren nach Anspruch 41, wobei es sich bei dem Marker um einen nicht-radioaktiven Marker handelt, der aus der aus Biotin, einem Enzym und einem fluoreszierenden Molekül bestehenden Gruppe ausgewählt ist.

53. Verfahren nach Anspruch 52, wobei das Enzym aus der aus alkalischer Phosphatase und Meerrettich-Peroxidase bestehenden Gruppe ausgewählt ist.

54. Verfahren nach Anspruch 52, wobei das fluoreszierende Molekül aus der aus Fluoreszein und Rhodamin bestehenden Gruppe ausgewählt ist.

55. Verfahren nach Anspruch 41, wobei die Kreuzhybridisierung zu DNA-DNA-Hybriden führt.

56. Verfahren nach Anspruch 41, wobei die Kreuzhybridisierung zu DNA-RNA-Hybriden führt.

**57.** Verfahren zum Nachweis von HPV 35-DNA oder -RNA; HPV 43-DNA oder -RNA; HPV 44-DNA oder -RNA; oder HPV 56-DNA oder -RNA, umfassend:

(1) Durchführung einer Hybridisierung unter stringenten Bedingungen mit

(a) einem aus der folgenden Gruppe ausgewählten Mitglied:

(i) mit einem Marker markierte HPV 35-DNA oder Fragmente davon; mit einem Marker markierte HPV 43-DNA oder Fragmente davon; mit einem Marker markierte HPV 44-DNA oder Fragmente davon; bzw. mit einem Marker markierte HPV 56-DNA oder Fragmente davon und

(ii) mit einem Marker markierte HPV 35-RNA oder Fragmente davon; mit einem Marker markierte HPV 43-RNA oder Fragmente davon; mit einem Marker markierte HPV 44-RNA oder Fragmente davon; bzw. mit einem Marker markierte HPV 56-RNA oder Fragmente davon,

(b) und einer unbekannten Probe von DNA oder RNA, und

(2) Untersuchung des Auftretens einer Kreuzhybridisierung, um HPV 35-DNA oder -RNA; HPV 43-DNA oder -RNA; HPV 44-DNA oder -RNA; bzw. HPV 56-DNA oder -RNA in der Probe nachzuweisen.

**58.** Verfahren nach Anspruch 57, wobei die unbekannte Probe von DNA oder RNA aus einer Genitalläsion, einer Läsion der Kehle, einer oralen Läsion oder einer Hautläsion stammt.

**59.** Verfahren nach Anspruch 58, wobei die unbekannte Probe von DNA oder RNA aus einer Genitalläsion stammt.

**60.** Verfahren nach Anspruch 59, wobei die unbekannte Probe von DNA oder RNA, die aus einer Genitalläsion stammt, durch Biopsie einer Epithelläsion, Ausschaben des Zervix oder Abtupfen des Zervix, um abgeschilferte Zellen zu erhalten, erhalten wird oder es sich um DNA handelt, die aus einer Genitalläsion stammt und in einen Klonierungsvektor kloniert worden ist.

**61.** Verfahren nach Anspruch 57, wobei die Fragmente eine Größe von etwa 15 bis etwa 8000 Basen oder Basenpaaren aufweisen.

**62.** Verfahren nach Anspruch 61, wobei die Fragmente eine Größe von etwa 300 bis etwa 800 Basen oder Basenpaaren aufweisen.

**63.** Verfahren nach Anspruch 57, wobei die HPV 35-DNA, die HPV 43-DNA, die HPV 44-DNA oder die HPV 56-DNA das HPV 35-Genom; das HPV 43-Genom; das HPV 44-Genom; bzw. das HPV 56-Genom umfaßt.

**64.** Verfahren nach Anspruch 57, wobei es sich bei dem Marker um einen radioaktiven Marker handelt.

**65.** Verfahren nach Anspruch 64, wobei es sich bei dem Marker um einen radioaktiven Marker handelt, der aus der aus $^{32}$P, $^{14}$C, $^{3}$H, $^{125}$I und $^{35}$S bestehenden Gruppe ausgewählt ist.

**66.** Verfahren nach Anspruch 57, wobei es sich bei dem Marker um einen nicht-radioaktiven Marker handelt, der aus der aus Biotin, einem Enzym und einem fluoreszierenden Molekül bestehenden Gruppe ausgewählt ist.

**67.** Verfahren nach Anspruch 66, wobei das Enzym aus der aus alkalischer Phosphatase und Meerrettich-Peroxidase bestehenden Gruppe ausgewählt ist.

**68.** Verfahren nach Anspruch 66, wobei das fluoreszierende Molekül aus der aus Fluoreszein und Rhodamin bestehenden Gruppe ausgewählt ist.

**69.** Verfahren nach Anspruch 57, wobei die Kreuzhybridisierung zu DNA-DNA-Hybriden führt.

**70.** Verfahren nach Anspruch 57, wobei die Kreuzhybridisierung zu DNA-RNA-Hybriden führt.

**71.** Verfahren zum Nachweis von HPV 35-DNA oder -RNA; HPV 43-DNA oder -RNA; HPV 44-DNA oder -RNA; oder HPV 56-DNA oder -RNA, umfassend:

(1) Durchführung einer Hybridisierung unter stringenten Bedingungen mit

(a) einer ersten Fraktion von DNA oder RNA, die jeweils aus einer Genitalläsion aus einer Reihe von Genitalläsionen stammt, wobei die Reihe ein epidemiologisches Fortschreiten zu zervikalem Krebs zeigt, und

(b) einem aus der folgenden Gruppe ausgewählten Mitglied:

(i) mit einem Marker markierte HPV 35-DNA oder Fragmente davon; mit einem Marker markierte HPV 43-DNA oder Fragmente davon; mit einem Marker markierte HPV 44-DNA oder Fragmente davon; bzw. mit einem Marker markierte HPV 56-DNA oder Fragmente davon und

(ii) mit einem Marker markierte HPV 35-RNA oder Fragmente davon; mit einem Marker markierte HPV 43-RNA oder Fragmente davon; mit einem Marker markierte HPV 44-RNA oder Fragmente davon; bzw. mit einem Marker markierte HPV 56-RNA oder Fragmente davon;

(2) Durchführung einer Hybridisierung unter stringenten Bedingungen mit

(a) einer zweiten Fraktion von DNA oder RNA, die aus jeder Genitalläsion der Reihe von Genitalläsionen stammt, und

(b) einer unbekannten Probe von DNA oder RNA, die aus einer Genitalläsion stammt, die mit einem Marker markiert ist;

(3) Vergleich der epidemiologischen Verteilung der in Stufe (1) erhaltenen Kreuzhybridisierung mit der in Stufe (2) erhaltenen Kreuzhybridisierung und der Kreuzhybridisierung der DNA aus jeder Läsion, die die epidemiologische Verteilung umfaßt, um HPV 35-DNA oder -RNA; HPV 43-DNA oder -RNA; HPV 44-DNA oder - RNA; bzw. HPV 56-DNA oder -RNA in der Probe nachzuweisen.

72. Verfahren nach Anspruch 71, wobei die Fragmente eine Größe von etwa 15 bis etwa 8000 Basen oder Basenpaaren aufweisen.

73. Verfahren nach Anspruch 72, wobei die Fragmente eine Größe von etwa 300 bis etwa 800 Basen oder Basenpaaren aufweisen.

74. Verfahren nach Anspruch 71, wobei die HPV 35-DNA, die HPV 43-DNA, die HPV 44-DNA oder die HPV 56-DNA das HPV 35-Genom; das HPV 43-Genom; das HPV 44-Genom; bzw. das HPV 56-Genom umfaßt.

75. Verfahren nach Anspruch 71, wobei es sich bei dem Marker um einen radioaktiven Marker handelt.

76. Verfahren nach Anspruch 75, wobei es sich bei dem Marker um einen radioaktiven Marker handelt, der aus der aus $^{32}$P, $^{14}$C, $^{3}$H, $^{125}$I und $^{35}$S bestehenden Gruppe ausgewählt ist.

77. Verfahren nach Anspruch 71, wobei es sich bei dem Marker um einen nicht-radioaktiven Marker handelt, der aus der aus Biotin, einem Enzym und einem fluoreszierenden Molekül bestehenden Gruppe ausgewählt ist.

78. Verfahren nach Anspruch 77, wobei das Enzym aus der aus alkalischer Phosphatase und Meerrettich-Peroxidase bestehenden Gruppe ausgewählt ist.

79. Verfahren nach Anspruch 77, wobei das fluoreszierende Molekül aus der aus Fluoreszein und Rhodamin bestehenden Gruppe ausgewählt ist.

80. Verfahren nach Anspruch 71, wobei die unbekannte Probe von DNA oder RNA, die aus einer Genitalläsion stammt, durch Biopsie einer Epithelläsion, Ausschaben des Zervix oder Abtupfen des Zervix, um abgeschilferte Zellen zu erhalten, erhalten wird oder es sich um DNA handelt, die aus einer Genitalläsion stammt und in einen Klonierungsvektor kloniert worden ist.

81. Verfahren nach Anspruch 71, wobei die Kreuzhybridisierung zu DNA-DNA-Hybriden führt.

82. Verfahren nach Anspruch 71, wobei die Kreuzhybridisierung zu DNA-RNA-Hybriden führt.

**Patentansprüche für folgenden Vertragsstaat : ES**

1. Verfahren zum Nachweis von HPV-DNA oder -RNA, umfassend:

(1) Ausführung einer Hybridisierung unter nichtstringenten Bedingungen mit

(a) einem aus der folgenden Gruppe ausgewählten Mitglied:

(i) mit einem Marker markierte HPV 35-DNA oder Fragmente davon; mit einem Marker markierte HPV 43-DNA oder Fragmente davon; mit einem Marker markierte HPV 44-DNA oder Fragmente davon; oder mit einem Marker markierte HPV 56-DNA oder Fragmente davon und

(ii) mit einem Marker markierte HPV 35-RNA oder Fragmente davon; mit einem Marker markierte HPV 43-RNA oder Fragmente davon; mit einem Marker markierte HPV 44-RNA oder Fragmente davon; oder mit einem Marker markierte HPV 56-RNA oder Fragmente davon,

(b) und einer unbekannten Probe von DNA oder RNA, und

(2) Untersuchung auf das Auftreten von Kreuzhybridisierung, um HPV-DNA oder -RNA in der Probe nachzuweisen.

2. Verfahren nach Anspruch 1, wobei die HPV-DNA die identifizierenden Merkmale der HPV 35-Klone 2A und 2B (ATCC Nr. 40330 bzw. ATCC Nr. 40331); der HPV 43-Klone 2A und 2B (ATCC Nr. 40338 bzw. ATCC Nr. 40339); des HPV 44-Klons 2 (ATCC Nr. 40353) oder der HPV 56-Klone 2A und 2B (ATCC Nr. 40341 bzw. ATCC Nr. 40379) aufweist.

3. Verfahren nach Anspruch 1, wobei es sich bei dem Marker um einen radioaktiven Marker handelt.

4. Verfahren nach Anspruch 3, wobei es sich bei dem Marker um einen radioaktiven Marker handelt, der aus der aus $^{32}$P, $^{14}$C, $^{3}$H, $^{125}$I und $^{35}$S bestehenden Gruppe ausgewählt ist.

5. Verfahren nach Anspruch 1, wobei es sich bei dem Marker um einen nicht-radioaktiven Marker handelt, der aus der aus Biotin, einem Enzym und einem fluoreszierenden Molekül bestehenden Gruppe ausgewählt ist.

6. Verfahren nach Anspruch 5, wobei das Enzym aus der aus alkalischer Phosphatase und Meerrettich-Peroxidase bestehenden Gruppe ausgewählt ist.

7. Verfahren nach Anspruch 5, wobei das fluoreszierende Molekül aus der aus Fluoreszein und Rhodamin bestehenden Gruppe ausgewählt ist.

8. Verfahren nach Anspruch 1, wobei die HPV-DNA oder Fragmente davon biologisch hergestellt werden.

9. Verfahren nach Anspruch 1, wobei die HPV-DNA oder Fragmente davon chemisch hergestellt werden.

10. Verfahren nach Anspruch 1, wobei die unbekannte Probe von DNA oder RNA aus einer Genitalläsion, einer Läsion der Kehle, einer oralen Läsion oder einer Hautläsion stammt.

11. Verfahren nach Anspruch 10, wobei die unbekannte Probe von DNA oder RNA aus einer Genitalläsion stammt.

12. Verfahren nach Anspruch 11, wobei die unbekannte Probe von DNA oder RNA, die aus einer Genitalläsion stammt, durch Biopsie einer Epithelläsion, Ausschaben des Zervix oder Abtupfen des Zervix, um abgeschilferte Zellen zu erhalten, erhalten wird oder es sich um DNA handelt, die aus einer Genitalläsion stammt und in einen Klonierungsvektor kloniert worden ist.

13. Verfahren nach Anspruch 1, wobei die Hybridisierung auch mit

(c) mindestens einem der folgenden Bestandteile durchgeführt wird: einem Mitglied aus der aus (i) mit einem Marker markierter HPV 6-DNA oder Fragmenten davon und (ii) mit einem Marker markierter HPV 6-RNA oder Fragmenten davon bestehenden Gruppe; einem Mitglied aus der aus (i) mit einem Marker markierter HPV 11-DNA oder Fragmenten davon und (ii) mit einem Marker markierter HPV 11-RNA oder Fragmenten davon bestehenden Gruppe; einem Mitglied aus der aus (i) mit einem Marker markierter HPV 16-DNA oder Fragmenten davon und (ii) mit einem Marker markierter HPV 16-RNA oder Fragmenten davon bestehenden Gruppe; und einem Mitglied aus der aus (i) mit einem Marker markierter HPV 18-DNA oder Fragmenten davon und (ii) mit einem Marker markierter HPV 18-RNA oder Fragmenten davon bestehenden Gruppe.

EP 0 294 659 B1

**14.** Verfahren nach Anspruch 13, wobei es sich bei dem weiteren Typ um mindestens ein Mitglied der aus HPV 16 und HPV 18 bestehenden Gruppe handelt.

**15.** Verfahren nach Anspruch 14, wobei es sich bei dem weiteren Typ um HPV 16 und HPV 18.

**16.** Verfahren nach Anspruch 1, wobei die Kreuzhybridisierung zu DNA-DNA-Hybriden führt.

**17.** Verfahren nach Anspruch 1, wobei die Kreuzhybridisierung zu DNA-RNA-Hybriden führt.

**18.** Verfahren zum Nachweis von HPV 35-DNA oder -RNA; HPV 43-DNA oder -RNA; HPV 44-DNA oder -RNA; oder HPV 56-DNA oder -RNA, umfassend:
(1) Durchführung einer Hybridisierung unter stringenten Bedingungen mit
(a) einem aus der folgenden Gruppe ausgewählten Mitglied:
(i) mit einem Marker markierte HPV 35-DNA oder Fragmente davon; mit einem Marker markierte HPV 43-DNA oder Fragmente davon; mit einem Marker markierte HPV 44-DNA oder Fragmente davon; bzw. mit einem Marker markierte HPV 56-DNA oder Fragmente davon und
(ii) mit einem Marker markierte HPV 35-RNA oder Fragmente davon; mit einem Marker markierte HPV 43-RNA oder Fragmente davon; mit einem Marker markierte HPV 44-RNA oder Fragmente davon; bzw. mit einem Marker markierte HPV 56-RNA oder Fragmente davon,
(b) und einer unbekannten Probe von DNA oder RNA, und
(2) Untersuchung des Auftretens einer Kreuzhybridisierung, um HPV 35-DNA oder -RNA; HPV 43-DNA oder -RNA; HPV 44-DNA oder -RNA; bzw. HPV 56-DNA oder -RNA in der Probe nachzuweisen.

**19.** Verfahren nach Anspruch 18, wobei die unbekannte Probe von DNA oder RNA aus einer Genitalläsion, einer Läsion der Kehle, einer oralen Läsion oder einer Hautläsion stammt.

**20.** Verfahren nach Anspruch 19, wobei die unbekannte Probe von DNA oder RNA aus einer Genitalläsion stammt.

**21.** Verfahren nach Anspruch 20, wobei die unbekannte Probe von DNA oder RNA, die aus einer Genitalläsion stammt, durch Biopsie einer EPithelläsion, Ausschaben des Zervix oder Abtupfen des Zervix, um abgeschilferte Zellen zu erhalten, erhalten wird oder es sich um DNA handelt, die aus einer Genitalläsion stammt und in einen Klonierungsvektor kloniert worden ist.

**22.** Verfahren nach Anspruch 18, wobei die Fragmente eine Größe von etwa 15 bis etwa 8000 Basen oder Basenpaaren aufweisen.

**23.** Verfahren nach Anspruch 22, wobei die Fragmente eine Größe von etwa 300 bis etwa 800 Basen oder Basenpaaren aufweisen.

**24.** Verfahren nach Anspruch 18, wobei die HPV 35-DNA, die HPV 43-DNA, die HPV 44-DNA oder die HPV 56-DNA das HPV 35-Genom; das HPV 43-Genom; das HPV 44-Genom; bzw. das HPV 56-Genom umfaßt.

**25.** Verfahren nach Anspruch 18, wobei es sich bei dem Marker um einen radioaktiven Marker handelt.

**26.** Verfahren nach Anspruch 25, wobei es sich bei dem Marker um einen radioaktiven Marker handelt, der aus der aus $^{32}$P, $^{14}$C, $^{3}$H, $^{125}$I und $^{35}$S bestehenden Gruppe ausgewählt ist.

**27.** Verfahren nach Anspruch 18, wobei es sich bei dem Marker um einen nicht-radioaktiven Marker handelt, der aus der aus Biotin, einem Enzym und einem fluoreszierenden Molekül bestehenden Gruppe ausgewählt ist.

**28.** Verfahren nach Anspruch 27, wobei das Enzym aus der aus alkalischer Phosphatase und Meerrettich-Peroxidase bestehenden Gruppe ausgewählt ist.

44

**29.** Verfahren nach Anspruch 27, wobei das fluoreszierende Molekül aus der aus Fluoreszein und Rhodamin bestehenden Gruppe ausgewählt ist.

**30.** Verfahren nach Anspruch 18, wobei die Kreuzhybridisierung zu DNA-DNA-Hybriden führt.

**31.** Verfahren nach Anspruch 18, wobei die Kreuzhybridisierung zu DNA-RNA-Hybriden führt.

**32.** Verfahren zum Nachweis von HPV 35-DNA oder -RNA; HPV 43-DNA oder -RNA; HPV 44-DNA oder -RNA; oder HPV 56-DNA oder -RNA, umfassend:

(1) Durchführung einer Hybridisierung unter stringenten Bedingungen mit

(a) einer ersten Fraktion von DNA oder RNA, die jeweils aus einer Genitalläsion aus einer Reihe von Genitalläsionen stammt, wobei die Reihe ein epidemiologisches Fortschreiten zu zervikalem Krebs zeigt, und

(b) einem aus der folgenden Gruppe ausgewählten Mitglied:

(i) mit einem Marker markierte HPV 35-DNA oder Fragmente davon; mit einem Marker markierte HPV 43-DNA oder Fragmente davon; mit einem Marker markierte HPV 44-DNA oder Fragmente davon; bzw. mit einem Marker markierte HPV 56-DNA oder Fragmente davon und

(ii) mit einem Marker markierte HPV 35-RNA oder Fragmente davon; mit einem Marker markierte HPV 43-RNA oder Fragmente davon; mit einem Marker markierte HPV 44-RNA oder Fragmente davon; bzw. mit einem Marker markierte HPV 56-RNA oder Fragmente davon;

(2) Durchführung einer Hybridisierung unter stringenten Bedingungen mit

(a) einer zweiten Fraktion von DNA oder RNA, die aus jeder Genitalläsion der Reihe von Genitalläsionen stammt, und

(b) einer unbekannten Probe von DNA oder RNA, die aus einer Genitalläsion stammt, die mit einem Marker markiert ist;

(3) Vergleich der epidemiologischen Verteilung der in Stufe (1) erhaltenen Kreuzhybridisierung mit der in Stufe (2) erhaltenen Kreuzhybridisierung und der Kreuzhybridisierung der DNA aus jeder Läsion, die die epidemiologische Verteilung umfaßt, um HPV 35-DNA oder -RNA; HPV 43-DNA oder -RNA; HPV 44-DNA oder - RNA; bzw. HPV 56-DNA oder -RNA in der Probe nachzuweisen.

**33.** Verfahren nach Anspruch 32, wobei die Fragmente eine Größe von etwa 15 bis etwa 8000 Basen oder Basenpaaren aufweisen.

**34.** Verfahren nach Anspruch 33, wobei die Fragmente eine Größe von etwa 300 bis etwa 800 Basen oder Basenpaaren aufweisen.

**35.** Verfahren nach Anspruch 32, wobei die HPV 35-DNA, die HPV 43-DNA, die HPV 44-DNA oder die HPV 56-DNA das HPV 35-Genom; das HPV 43-Genom; das HPV 44-Genom; bzw. das HPV 56-Genom umfaßt.

**36.** Verfahren nach Anspruch 32, wobei es sich bei dem Marker um einen radioaktiven Marker handelt.

**37.** Verfahren nach Anspruch 36, wobei es sich bei dem Marker um einen radioaktiven Marker handelt, der aus der aus $^{32}$P, $^{14}$C, $^{3}$H, $^{125}$I und $^{35}$S bestehenden Gruppe ausgewählt ist.

**38.** Verfahren nach Anspruch 32, wobei es sich bei dem Marker um einen nicht-radioaktiven Marker handelt, der aus der aus Biotin, einem Enzym und einem fluoreszierenden Molekül bestehenden Gruppe ausgewählt ist.

**39.** Verfahren nach Anspruch 38, wobei das Enzym aus der aus alkalischer Phosphatase und Meerrettich-Peroxidase bestehenden Gruppe ausgewählt ist.

**40.** Verfahren nach Anspruch 38, wobei das fluoreszierende Molekül aus der aus Fluoreszein und Rhodamin bestehenden Gruppe ausgewählt ist.

**41.** Verfahren nach Anspruch 32, wobei die unbekannte Probe von DNA oder RNA, die aus einer Genitalläsion stammt, durch Biopsie einer Epithelläsion, Ausschaben des Zervix oder Abtupfen des Zervix, um abgeschilferte Zellen zu erhalten, erhalten wird oder es sich um DNA handelt, die aus einer Genitalläsion stammt und in einen Klonierungsvektor kloniert worden ist.

**42.** Verfahren nach Anspruch 32, wobei die Kreuzhybridisierung zu DNA-DNA-Hybriden führt.

**43.** Verfahren nach Anspruch 32, wobei die Kreuzhybridisierung zu DNA-RNA-Hybriden führt.

**Revendications**
**Revendications pour les Etats contractants suivants : AT, BE, CH, DE, FR, GB, GR, IT, LI, LU, NL, SE**

**1.** ADN recombinant de HPV 35, HPV 43, HPV 44 ou HPV 56 comprenant un vecteur clonant et l'ADN HPV 35 ou ses fragments ; ADN HPV 43 ou ses fragments ; ADN HPV 44 ou ses fragments ou ADN HPV 56 ou ses fragments, respectivement.

**2.** ADN recombinant selon la revendication 1, dans lequel ledit vecreur clonant est sélectionné dans le groupe constitué par pBR322, pUC 11, λ charon, λ L47, le bactériophage dérivé de M13, pZIP-Neo SV (XI), pBKTK-1, pT712 et pT713.

**3.** ADN recombinant selon la revendication 1, dans lequel lesdidts fragments ont une dimension d'environ 15 à environ 8 000 paires de bases.

**4.** ADN recombinant selon la revendication 3, dans lequel lesdidts fragments ont une dimension d'environ 300 à environ 800 paires de bases.

**5.** ADN recombinant selon la revendication 1, dans lequel ledit ADN recombinant a les caractéristiques d'identification des clones 2A et 2B HPV 35 (ATCC n°. 40330 et ATCC n°. 40331, respectivement) ; des clones 2A et 2B HPV 43 (ATCC n°. 40338 et ATCC n°. 40339, respectivement) ; du clone 2 HPV 44 (ATCC n°. 40353) ou des clones 2A et 2B HPV 56 (ATCC n°. 40341 et ATCC n°. 40379, respectivement).

**6.** ADN recombinant selon la revendication 1, dans lequel l'ADN HPV dudit ADN recombinant est marqué avec un marqueur.

**7.** ADN recombinant selon la revendication 6, dans lequel ledit marqueur est un marqueur radioactif.

**8.** ADN recombinant selon la revendication 7, dans lequel ledit marqueur est un marqueur radioactif sélectionné dans le groupe constitué par $^{32}$P, $^{14}$C, $^{3}$H, $^{125}$I, et $^{35}$S.

**9.** ADN recombinant selon la revendication 6, dans lequel ledit marqueur est un marqueur non radioactif sélectionné dans le groupe constitué par la biotine, un enzyme et une molécule flurorescente.

**10.** ADN recombinant selon la revendication 9, dans lequel ledit enzyme est sélectionné dans le groupe constitué par la phosphatase alcaline et la péroxydase raifort.

**11.** ADN recombinant selon la revendication 9, dans lequel ladite molécule fluorescente est sélectionnée dans le groupe constitué par la fluoresceine et la rhodamine.

**12.** ADN HPV 35 pur ou ses fragments ; ADN HPV 43 pur ou ses fragments ; ADN HPV 44 pur ou ses fragments ; ou ADN HPV 56 pur ou ses fragments.

**13.** ADN HPV pur selon la revendication 12, dans lequel ledit ADN HPV ou ses fragments sont produits biologiquement.

**14.** ADN HPV pur selon la revendication 12, dans lequel ledit ADN HPV ou ses fragments sont produits chimiquement.

**15.** ADN HPV selon la revendication 12, dans lequel lesdits fragments ont une dimension de environ 15 à environ 8 000 bases.

**16.** ADN HPV selon la revendication 15, dans lequel lesdits fragments ont une dimension de environ 300 à environ 800 bases.

**17.** ARN HPV 35 pur ou ses fragments ; ARN HPV 43 pur su ses fragments ; ARN HPV 44 pur ou ses fragments ; ou ARN HPV 56 pur ou ses fragments.

**18.** ARN HPV pur selon la revendication 17, dans lequel ledit ARN HPV ou ses fragments sont produits biologiquement.

**19.** ARN HPV pur selon la revendication 17, dans lequel ledit ARN HPV ou ses fragments sont produits chimiquement.

**20.** ARN HPV pur selon la revendication 17, dans lequel lesdits fragments ont une dimension de environ 15 à environ 8 000 bases.

**21.** ARV HPV pur selon la revendication 20, dans lequel lesdits fragments ont une dimension de environ 300 à environ 800 bases.

**22.** Sonde d'hybridation HPV comprenant un chaînon sélectionné dans le groupe constitué par
    (i) ADN HPV 35 ou ses fragments marqués par un marqueur,
    (ii) ARN HPV 35 ou ses fragments marqués par un marqueur,
    (iii) ADN HPV 43 ou ses fragments marqués par un marqueur,
    (iv) ARN HPV 43 ou ses fragments marqués par un marqueur,
    (v) ADN HPV 44 ou ses fragments marqués par un marqueur,
    (vi) ARN HPV 44 ou ses fragments marqués par un marqueur,
    (vii) ADN HPV 56 ou ses fragments marqués par un marqueur, et
    (viii) ARN HPV 56 ou ses fragments marqués par un marqueur.

**23.** Sonde d'hybridation HPV selon la revendication 22, dans laquelle lesdits fragments ont une dimension d'environ 15 à environ 8 000 bases.

**24.** Sonde d'hybridation HPV selon la revendication 23, dans laquelle lesdits fragments ont une dimension d'environ 300 à environ 800 bases.

**25.** Sonde d'hybridation HPV selon la revendication 24, dans laquelle ledit marqueur est un marqueur radioactif.

**26.** Sonde d'hybridation HPV selon la revendication 25, dans laquelle ledit marqueur est un marqueur radioactif sélectionné dans le groupe constitué par $^{32}$P, $^{14}$C, $^{3}$H, $^{125}$I, et $^{35}$S.

**27.** Sonde d'hybridation HPV selon la revendication 22, dans laquelle ledit marqueur est un marqueur non radioactif sélectionné dans le groupe constitué par la biotine, un enzyme et une molécule flurorescente.

**28.** Sonde d'hybridation HPV selon la revendication 27, dans laquelle ledit enzyme est sélectionné dans le groupe constitué par la phospharase alcaline et la péroxydase raifort.

**29.** Sonde d'hybridation HPV selon la revendication 27, dans laquelle ladite molécule fluorescente est sélectionnée dans le groupe constitué par la fluresceine et la rhodamine.

**30.** Composition de sonde d'hybridation HPV comprenant
    (a) un chaînon sélectionné dans le groupe constitué par (i) ADN HPV 35 ou ses fragments, marqué par un marqueur : et (ii) ARN HPV 35 ou ses fragments, marqué par un marqueur, et
    (b) ADN ou ARN ou leurs fragments d'au moins un autre type HPV marqué avec un marqueur, ou
        (a') un chaînon sélectionné dans le coupe constitué par (i) ADN HPV 43 ou ses fragments, marqué par un marqueur ; et (ii) ARN HPV 43 ou ses fragments, marqué par un marqueur, et
        (b') ADN ou ARN ou leurs fragments d'au moins un autre type HPV marqué avec un marqueur, ou

47

(a'') un chaînon sélectionné dans le groupe constitué par (i) ADN HPV 44 ou ses fragments marqué par un marqueur ; et (ii) ARN HPV 44 ou ses fragments, marqué par un marqueur, et
(b'') ADN ou ARN ou leurs fragments d'au moins un autre type HPV marqué avec un marqueur, ou

(a''') un chaînon sélectionné dans le groupe constitué par (i) ADN HPV 56 ou ses fragments, marqué par un marqueur ; et (ii) ARN HPV 56 ou ses fragments marqué par un marqueur, et
(b''') ADN ou ARN ou leurs fragments d'au moins un autre type HPV marqué avec un marqueur.

31. Composition de sonde d'hybridation HPV selon la revendication 30, dans laquelle lesdits fragmenst ont une dimension d'environ 15 à environ 8 000 bases ou paires de bases.

32. Composition de sonde d'hybridation HPV selon la revendication 31, dans laquelle lesdits fragments ont une dimension d'environ 300 à environ 800 bases ou paires de bases.

33. Composition de sonde d'hybridation HPV selon la revendication 30, dans laquelle ledit marqueur est un marqueur radioactif.

34. Composition de sonde d'hybridation HPV selon la revendication 33, dans laquelle ledit marqueur est un marqueur radioactif sélectionné dans le groupe constitué par $^{32}$P, $^{14}$C, $^{3}$H, $^{125}$I, et $^{35}$S.

35. Composition de sonde d'hybridation HPV selon la revendication 30, dans laquelle ledit marqueur est un marqueur non radioactif sélectionné dans le groupe constitué par la biotine, un enzyme et une molécule flurorescente,

36. Composition de sonde d'hybridation HPV selon la revendication 35, dans laquelle ledit enzyme est sélectionné dans le groupe constitué par la phosphatase alcaline et la péroxydase raifort.

37. Composition de sonde d'hybridation HPV selon la revendication 35, dans laquelle ladite molécule fluorescente est sélectionnée dans le groupe constitué par la fluoresceine et la rhodamine.

38. Composition de sonde d'hybridation HPV selon la revendication 30, dans laquelle ledit autre type HPV est au moins un chaînon sélectionné dans le groupe constitué par HPV 6, HPV 11, HPV 16, HPV 18 et HPV31.

39. Composition de sonde d'hybridation HPV selon la revendication 38, dans laquelle ledit autre type HPV est au moins un chaînon sélectionné dans le groupe constitué par HPV 16, HPV 18 et HPV31.

40. Composition de sonde d'hybridation HPV selon la revendication 39, dans laquelle ledit autre type HPV est HPV 16, HPV 18 et HPV31.

41. Procédé pour détecter l'ADN ou l'ARN HPV comprenant :
(1) réaliser l'hybridation, dans des conditions non rigoureuses avec
(a) un chaînon sélectionné dans le groupe consitué par
(i) ADN HPV 35 ou des fragments de celui-ci, marqué avec un marqueur ; ADN HPV 43 ou des fragments de celui-ci, marqué avec un marqueur ; ou ADN HPV 44 ou des fragments de celui-ci, marqué avec un marqueur ; ou ADN HPV 56 ou des fragments de celui-ci, marqué avec un marqueur, et
(ii) ARN HPV 35 ou des fragments de celui-ci, marqué avec un marqueur ; ARN HPV 43 ou des fragments de celui-ci, marqué avec un marqueur ; ou ARN HPV 44 ou des fragments de celui-ci, marqué avec un marqueur ; ou ARN HPV 56 ou des fragments de celui-ci, marqué avec un marqueur,
(b) un échantillon inconnu d'ADN ou ARN, et
(2) titrer la présence d'hybridation croisée de façon à détecter ADN ou ARN HPV dans ledit échantillon.

**42.** Procédé selon la revendication 41, dans lequel ledit échantillon inconnu d'ADN ou d'ARN est dérivé d'une lésion géntitale, de la gorge, buccale ou de la peau.

**43.** Procédé selon la revendication 42, dans lequel ledit échantillon, inconnu d'ADN ou ARN est dérivé d'une lésion génitale.

**44.** Procédé selon la revendication 43, dans lequel ledit échantillon, inconnu d'ADN ou ARN dérivé d'une lésion génitale est obtenu par Biopsie d'une lésion épithéliale, en grattant le cerveau ou en frottant le cerveau pour obtenir des cellules exfoliées, ou l'ADN est dérivé d'une lésion génitale qui a été clonée dans un vecteur clonant.

**45.** Procédé selon la revendication 41, dans lequel lesdits fragments ont une dimension d'environ 15 à 8 000 bases ou paires de bases.

**46.** Procédé selon la revendication 45, dans lequel lesdits fragments ont une dimension d'environ 300 à 800 bases ou paires de bases.

**47.** Procédé selon la revendication 41, dans lequel l'hybridation est aussi réalisée avec
(c) au moins un de ; un chaînon sélectionné dans le groupe constitué par (i) ADN HPV 6 ou ses fragments marqués avec un marqueur, et (ii) ARN HPV 6 ou ses fragments marqués avec un marqueur ; un élément sélectionné dans le groupe constitué par (i) ADN HPV 11 ou ses fragments marqués avec un marqueur et (ii) ARN HPV 11 ou ses fragments marqués avec un marqueur ; un élément sélectionné dans le groupe constitué par (i) ADN HPV 16 ou ses fragments marqués avec un marqueur et (ii) ARN HPV 16 ou ses fragments marqués avec un marqueur ; et un élément sélectionné dans le groupe constitué par (i) ADN HPV 18 ou ses fragments marqués avec un marqueur et (ii) ARN HPV 18 ou ses fragments marqués avec un marqueur.

**48.** Procédé selon la revendication 47, dans lequel ledit autre type est au moins un chaînon sélectionné dans le groupe constitué par HPV 16 et HPV 18.

**49.** Procédé selon la revendication 48, dans lequel dans lequel ledit autre type est HPV 16 et HPV 18.

**50.** Procédé selon la revendication 41, dans lequel le marqueur est un marqueur radioactif.

**51.** Procédé selon la revendication 50, dans lequel ledit marqueur est un marqueur radioactif sélectionné dans le groupe constitué par $^{32}$P, $^{14}$C, $^{3}$H, $^{125}$I, et $^{35}$S.

**52.** Procédé selon la revendication 41, dans lequel ledit marqueur est un marqueur non radioactif sélectionné dans le groupe constitué par la biotine, un enzyme et une molécule flurorescente.

**53.** Procédé selon la revendication 52, dans lequel ledit enzyme est sélectionné dans le groupe constitué par la phosphatase alcaline et la péroxydase raifort.

**54.** Procédé selon la revendication 52, dans lequel ladite molécule fluorescente est sélectionnée dans le groupe constitué par la fluoresceine et la rhodamine.

**55.** Procédé selon la revendication 41, dans lequel l'hybridation croisée produit des hybrides ADN-ADN.

**56.** Procédé selon la revendication 41, dans lequel l'hybridation croisée produit des hybrides ADN-ARN.

**57.** Procédé pour détecter les ADN ou ARN HPV 35 ; ADN ou ARN HPV 43 ; ADN ou ARN HPV 44 ; ADN ou ARN HPV 56, comprenant ;
(1) réaliser l'hybridation, dans des conditions rigoureuses, avec
(a) un chaînon sélectionné dans le groupe constitué par
(i) l'ADN HPV 35 ou des fragments de celui-ci, marqués avec un marqueur ; ADN HPV 43 ou des fragments de celui-ci, marqués avec un marqueur ; ADN HPV 44 ou des fragments de celui-ci, marqués avec un marqueur ; ou ADN HPV 56 ou des fragments de celui-ci marqués avec un marqueur, respectivement, et

49

(ii) l'ARN HPV 35 ou des fragments de celui-ci, marqués avec un marqueur ; ARN HPV 43 ou des fragments de celui-ci, marqués avec un marqueur ; ARN HPV 44 ou des fragments de celui-ci, marqués avec un marqueur ; ou ARN HPV 56 ou des fragments de celui-ci, marqués avec un marqueur, respectivement, et

(b) un échantillon inconnu d'ADN ou d'ARN, et

(2) titrer la présence de l'hybridation croisée de façon à détecter l'ADN ou l'ARN HPV 35 ; ADN ou l'ARN HPV 43 ; ADN ou ARN HPV 44 ; ADN ou ARN HPV 56 respectivement dans ledit échantillon.

58. Procédé selon la revendication 57, dans lequel ledit échantillon inconnu d'ADN ou d'ARN est dérivé d'une lésion génitale, de la gorge, buccale ou de la peau.

59. Procédé selon la revendication 58, dans lequel ledit échantillon inconnu d'ADN ou d'ARN est dérivé d'une lésion génitale.

60. Procédé selon la revendication 59, dans lequel ledit échantillon, inconnu d'ADN ou ARN dérivé d'une lésion génitale est obtenu par Biopsie d'une lésion épithéliale, en grattant le cerveau ou en frottant le cerveau pour obtenir des cellules exfoliées, ou l'ADN est dérivé d'une lésion génitale qui a été clonée dans un vecteur clonant.

61. Procédé selon la revendication 57 dans lequel lesdits fragments ont une dimension d'environ 15 à environ 8 000 bases ou paires de bases.

62. Procédé selon la revendication 61, dans lequel lesdits fragments ont une dimension d'environ 300 à environ 800 bases ou paires de bases.

63. Procédé selon la revendication 57, dans lequel ledit ADN HPV 35, ADN HPV 43, ADN HPV 44 ou ADN HPV 56 comrprennent le génome HPV 35, HPV 43, HPV 44 ou le génome HPV 56, respectivement.

64. Procédé selon la revendication 57, dans lequel le marqueur est un marqueur radioactif.

65. Procédé selon la revendication 64, dans lequel ledit marqueur est un marqueur radioactif sélectionné dans le groupe constitué par $^{32}$P, $^{14}$C, $^{3}$H, $^{125}$I, et $^{35}$S.

66. Procédé selon la revendication 57, dans lequel ledit marqueur est un marqueur non radioactif sélectionné dans le groupe constitué par la biotine, un enzyme et une molécule fluorescente.

67. Procédé selon la revendication 66, dans lequel ledit enzyme est sélectionné dans le groupe constitué par la phosphatase alcaline et la péroxydase raifort.

68. Procédé selon la revendication 66, dans lequel ladite molécule fluorescente est sélectionnée dans le groupe constitué par la fluoresceine et la rhodamine.

69. Procédé selon la revendication 57, dans lequel l'hybridation croisée produit des hybrides ADN-ADN.

70. Procédé selon la revendication 57, dans lequel l'hybridation croisée produit des hybrides ADN-ARN.

71. Procédé pour détecter les ADN ou ARN HPV 35 ; ADN ou ARN HPV 43 ; ADN ou ARN HPV 44 ; ADN ou ARN HPV 56, comprenant :

(1) réaliser l'hybridation, dans des conditions rigoureuses, avec

(a) une première fraction d'ADN ou ARN dérivée de chaque lésion génitale d'un échantillonnage de lésions génitales, lequel échantillonnage présente une progression épidémiologique au cancer du cerveau, et

(b) un chaînon sélectionné dans le groupe consitué par

(i) ADN HPV 35 ou ses fragments marqués avec un marqueur ; ADN HPV 43 ou ses fragments marqués avec un marqueur ; ADN HPV 44 ou ses fragments marqués avec un marqueur ; ou ADN HPV 56 ou ses fragments marqués avec un marqueur, et

(ii) ARN HPV 35 ou ses fragments marqués avec un marqueur ; ARN HPV 43 ou ses fragments marqués avec un marqueur ; ARN HPV 44 ou ses fragments marqués avec un marqueur ; ou ARN HPV 56 ou ses fragments marqués avec un marqueur,

(2) effectuer d'hybridation, dans de conditions rigoureuses, avec

(a) une seconde fraction d'ADN ou ARN dérivée de chaque lésion génitale dudit échantillonage de lésions génitales, et

(b) un échantillon inconnu d'ADN ou ARN dérivé d'une lésion génitale qui a été marquée avec un marqueur ;

(3) comparer la distribution épidémiologique de l'hybridation croisée obtenue dans l'étape (1) avec celle obtenue dans l'étape (2) et l'hybridation croisée de l'ADN de chaque lésion qui comprend ladite distribution épidémiologique de façon à détecter ADN ou ARN HPV 35, ADN ou ARN HPV 43, ADN ou ARN HPV 44 ou ADN ou ARN HPV 56, respectivement dans ledit échantillon.

**72.** Procédé selon la revendication 71, dans lequel lesdits fragments ont une dimension d'environ 15 à environ 8 000 bases ou paires de bases.

**73.** Procédé selon la revendication 72, dans lequel lesdits fragments ont une dimension d'environ 300 à environ 800 bases ou paires de bases.

**74.** Procédé selon la revendication 71, dans lequel ledit ADN HPV 35, ADN HPV 43, ADN HPV 44 ou ADN HPV 56 comprend substantiellement tout du génome HPV 35, HPV 43, HPV 44 ou HPV 56, respectivement.

**75.** Procédé selon la revendication 71, dans lequel ledit marqueur est un marqueur radioactif.

**76.** Procédé selon la revendication 75, dans lequel ledit marqueur est un marqueur radioactif sélectionné dans le groupe constitué par $^{32}$P, $^{14}$C, $^{3}$H, $^{125}$I, et $^{35}$S.

**77.** Procédé selon la revendication 71, dans lequel ledit marqueur est un marqueur non radioactif sélectionné dans le groupe constitué par la biotine, un enzyme et une molécule flurorescente.

**78.** Procédé selon la revendication 77, dans lequel ledit enzyme est sélectionné dans le groupe constitué par la phosphatase alcaline et la péroxydase raifort.

**79.** Procédé selon la revendication 77, dans lequel ladite molécule fluorescente est sélectionnée dans le groupe constitué par la fluoresceine et la rhodamine.

**80.** Procédé selon la revendication 71, dans lequel ledit échantillon, inconnu d'ADN ou ARN dérivé d'une lésion génitale est obtenu par Biopsie d'une lésion épithéliale, en grattant le cerveau ou en frottant le cerveau pour obtenir des cellules exfoliées, ou l'ADN est dérivé d'une lésion génitale qui a été clonée dans un vecteur clonant.

**81.** Procédé selon la revendication 71, dans lequel l'hybridation croisée produit des hybrides ADN-ADN.

**82.** Procédé selon la revendication 71, dans lequel l'hybridation croisée produit des hybrides ADN-ARN.

**Revendications pour l'Etat contractant suivant : ES**

**1.** Procédé pour déctecter l'ADN ou l'ARN comprenant :

(1) réaliser l'hybridation, dans des conditions non rigoureuses avec

(a) un chaînon sélectionné dans le groupe constitué par

(i) ADN HPV 35 ou des fragments de celui-ci, marqué avec un marqueur ; ADN HPV 43 ou des fragments de celui-ci, marqué avec un marqueur ; ou ADN HPV 44 ou des fragments de celui-ci, marqué avec un marqueur ; ou ADN HPV 56 ou des fragments de celui-ci, marqué avec un marqueur, et

(ii) ARN HPV 35 ou des fragments de celui-ci, marqué avec un marqueur ; ARN HPV 43 ou des fragments de celui-ci, marqué avec un marqueur ; ou ARN HPV 44 ou des fragments de celui-ci, marqué avec un marqueur ; ou ARN HPV 56 ou des fragments de celui-ci, marqué avec un marqueur,

(b) un échantillon inconnu d'ADN ou ARN, et

(2) titrer la présence d'hybridation croisée de façon à détecter ADN ou ARN HPV.

2. Procédé selon la revendication 1, dans lequel ledit ADN HPV a les caractéristiques d'identification des clones 2A et 2B HPV 35 (ATCC n°. 40330 et ATCC n°. 40331, respectivement) ; des clones 2A et 2B HPV 43 (ATCC n°. 40338 et ATCC n°. 40339, respectivement) ; du clone 2 HPV 44 (ATCC n°. 40353) ; ou des clones 2A et 2B HPV 56 (ATCC n°. 40341 et ATCC n°. 40379, respectivement).

3. Procédé selon la revendication 1, dans lequel le marqueur est un marqueur radioactif.

4. Procédé selon la revendication 3, dans lequel ledit marqueur est un marqueur radioactif sélectionné dans le groupe constitué par $^{32}$P, $^{14}$C, $^{3}$H, $^{125}$I, et $^{35}$S.

5. Procédé selon la revendication 1, dans lequel ledit marqueur est un marqueur non radioactif sélectionné dans le groupe constitué par la biotine, un enzyme et une molécule flurorescente.

6. Procédé selon la revendication 5, dans lequel ledit enzyme est sélectionné dans le groupe constitué par la phosphatase alcaline et la péroxydase raifort.

7. Procédé selon la revendication 5, dans lequel ladite molécule fluorescente est sélectionnée dans le groupe constitué par la fluoresceine et la rhodamine.

8. Procédé selon la revendication 1, dans lequel l'ADN HPV ou ses fragments sont produits par voie biologique.

9. Procédé selon la revendication 1, dans lequel l'ADN HPV ou ses fragments sont produits par voie chimique.

10. Procédé selon la revendication 1, dans lequel ledit échantillon inconnu d'ADN ou d'ARN est dérivé d'une lésion génitale, de la gorge, buccale ou de la peau.

11. Procédé selon la revendication 10, dans lequel ledit échantillon, inconnu d'ADN ou ARN est dérivé d'une lésion génitale.

12. Procédé selon la revendication 10, dans lequel ledit échantillon, inconnu d'ADN ou ARN dérivé d'une lésion génitale est obtenu par Biopsie d'une lésion épithéliale, en grattant le cerveau ou en frottant le cerveau pour obtenir des cellules exfoliées, ou l'ADN est dérivé d'une lésion génitale qui a été clonée dans un vecteur clonant.

13. Procédé selon la revendication 1, dans lequel l'hybridation est aussi réalisée avec
(c) au mains un de : un chaînon sélectionné dans le groupe constitué par (i) ADN HPV 6 ou ses fragments marqués avec un marqueur, et (ii) ARN HPV 6 ou ses fragments marqués avec un marqueur ; un élément sélectionné dans le groupe constitué par (i) ADN HPV 11 ou ses fragments marqués avec un marqueur et (ii) ARN HPV 11 ou ses fragments marqués avec un marqueur ; un élément sélectionné dans le groupe constitué par (i) ADN HPV 16 ou ses fragments marqués avec un marqueur et (ii) ARN HPV 16 ou ses fragments marqués avec un marquer ; et un élément sélectionné dans le groupe constitué par (i) ADN HPV 18 ou ses fragments marqués avec un marqueur et (ii) ARN HPV 18 ou ses fragments marqués avec un marqueur.

14. Procédé selon la revendication 13, dans lequel ledit autre type est au moins un chaînon sélectionné dans le groupe constitué par HPV 16 et HPV 18.

15. Procédé selon la revendication 14, dans lequel ledit autre type est HPV 16 et HPV 18.

16. Procédé selon la revendication 1, dans lequel l'hybridation croisée produit des hybrides ADN-ADN.

17. Procédé selon la revendication 1, dans lequel l'hybridation croisée produit des hybrides ADN-ARN.

**18.** Procédé pour détecter les ADN ou ARN HPV 35 ; ADN ou ARN HPV 43 ; ADN ou ARN HPV 44 ; ADN ou ARN HPV 56, comprenant :
    (1) réaliser l'hybridation, dans des conditions rigoureuses, avec
        (a) un chaînon sélectionné dans le groupe constitué par
            (i) l'ADN HPV 35 ou des fragments de celui-ci, marqués avec un marqueur ; ADN HPV 43 ou des fragments de celui-ci, marqués avec un marqueur ; ADN HPV 44 ou des fragments de celui-ci, marqués avec un marqueur ; ou ADN HPV 56 ou des fragments de celui-ci marqués avec un marqueur, respectivement, et
            (ii) l'ARN HPV 35 ou des fragments de celui-ci, marqués avec un marqueur ; ARN HPV 43 ou des fragments de celui-ci, marqués avec un marqueur ; ARN HPV 44 ou des fragments de celui-ci, marqués avec un marqueur ; ou ARN HPV 56 ou des fragments de celui-ci, marqués avec un marqueur, respectivement, et
        (b) un échantillon inconnu d'ADN ou d'ARN, et
    (2) titrer la présence de l'hybridation croisée de façon à détecter l'ADN ou l'ARN HPV 35 ; ADN ou l'ARN HPV 43 ; ADN ou ARN HPV 44 ; ADV ou ARN HPV 56 respectivement dans ledit échantillon.

**19.** Procédé selon la revendication 18, dans lequel ledit échantillon inconnu d'ADN ou d'ARN est dérivé d'une lésion génitale, de la gorge, buccale ou de la peau.

**20.** Procédé selon la revendication 19, dans lequel ledit éhantillon inconnu d'ADN ou d'ARN est dérivé d'une lésion génitale.

**21.** Procédé selon la revendication 20, dans lequel ledit échantillon, inconnu d'ADN ou ARN dérivé d'une lésion génitale est obtenu par Biopsie d'une lésion épithéliale, en grattant le cerveau ou en frottant le cerveau pour obtenir des cellules exfoliées, ou l'ADN est dérivé d'une lésion génitale qui a été clonée dans un vecteur clonant.

**22.** Procédé selon la revendication 18, dans lequel lesdits fragments ont une dimension d'environ 15 à environ 8 000 bases ou paires de bases.

**23.** Procédé selon la revendication 22, dans lequel lesdits fragments ont une dimension d'environ 300 à environ 800 bases ou paires de bases.

**24.** Procédé selon la revendication 18, dans lequel ledit ADN HPV 35, ADN HPV 43, ADN HPV 44 ou ADN HPV 56 comprennent le génome HPV 35, HPV 43, HPV 44 ou le génome HPV 56, respectivement.

**25.** Procédé selon la revendication 18, dans lequel le marqueur est un marqueur radioactif.

**26.** Procédé selon la revendication 25, dans lequel ledit marqueur est un marqueur radioactif sélectionné dans le groupe constitué par $^{32}$P, $^{14}$C, $^{3}$H, $^{125}$I, et $^{35}$S.

**27.** Procédé selon la revendication 18, dans lequel ledit marquer est un marqueur non radioactif sélectionné dans le groupe constitué par la biotine, un enzyme et une molécule flurorescente.

**28.** Procédé selon la revendication 27, dans lequel ledit enzyme est sélectionné dans le groupe constitué par la phosphatase alcaline et la péroxydase raifort.

**29.** Procédé selon la revendication 27, dans lequel ladite molécule fluorescente est sélectionnée dans le groupe constitué par la fluoresceine et la rhodamine.

**30.** Procédé selon la revendication 18, dans lequel l'hybridation croisée produit des hybrides ADN-ADN.

**31.** Procédé selon la revendication 18, dans lequel l'hybridation croisée produit des hybrides ADN-ARN.

**32.** Procédé pour détecter les ADN ou ARN HPV 35 ; ADN ou ARN HPV 43 ; ADN ou ARN HPV 44 ; ADN ou ARN HPV 56, comprenant :
    (1) réaliser l'hybridation, dans des conditions rigoureuses, avec

(a) une première fraction d'ADN ou ARN dérivée de chaque lésion génitale d'un échantillonnage de lésions génitales, lequel échantillonage présente une progression épidémiologique au cancer du cerveau, et

(b) un chaînon sélectionné dans le groupe consitué par

(i) ADN HPV 35 ou ses fragments marqués avec un marqueur ; ADN HPV 43 ou ses fragments marqués avec un marqueur ; ADN HPV 44 ou ses fragments marqués avec un marqueur ; ou ADN HPV 56 ou ses fragments marqués avec un marqueur, et

(ii) ARN HPV 35 ou ses fragments marqués avec un marqueur ; ARN HPV 43 ou ses fragments marqués avec un marqueur ; ARN HPV 44 ou ses fragments marqués avec un marqueur ; ou ARN HPV 56 ou ses fragments marqués avec un marqueur,

(2) effectuer d'hybridation, dans de conditions rigoureuses, avec

(a) une seconde fraction d'ADN ou ARN dérivée de chaque lésion génitale dudit échantillonage de lésions génitales, et

(b) un échantillon inconnu d'ADN ou ARN dérivé d'une lésion génitale qui a été marquée avec un marqueur ;

(3) comparer la distribution épidémiologique de l'hybridation croisée obtenue dans l'étape (1) avec celle obtenue dans l'étape (2) et l'hybridation croisée de l'ADN de chaque lésion qui comprend ladite distribution épidémiologique de façon à détecter ADN ou ARN HPV 35, ADN ou ARN HPV 43, ADN ou ARN HPV 44 ou ADN ou ARN HPV 56, respectivement dans ledit échantillon.

33. Procédé selon la revendication 32, dans lequel lesdits fragments ont une dimension d'environ 15 à environ 8 000 bases ou paires de bases.

34. Procédé selon la revendication 32, dans lequel lesdits fragments ont une dimension d'environ 300 à environ 800 bases ou paires de bases.

35. Procédé selon la revendication 32, dans lequel ledit ADN HPV 35, ADN HPV 43, ADN HPV 44 ou ADN HPV 56 comprend substantiellement tout du génome HPV 35, HPV 43, HPV 44 ou HPV 56, respectivement.

36. Procédé selon la revendication 32, dans lequel ledit marqueur est un marqueur radioactif.

37. Procédé selon la revendication 36, dans lequel ledit marqueur est un marqueur radioactif sélectionné dans le groupe constitué par $^{32}$P, $^{14}$C, $^{3}$H, $^{125}$I, et $^{35}$S.

38. Procédé selon la revendication 32, dans lequel ledit marqueur est un marqueur non radioactif sélectionné dans le groupe constitué par la biotine, un enzyme et une molécule flurorescente.

39. Procédé selon la revendication 38, dans lequel ledit enzyme est sélectionné dans le groupe constitué par la phosphatase alcaline et la péroxydase raifort.

40. Procédé selon la revendication 38, dans lequel ladite molécule fluorescente est sélectionnée dans le groupe constitué par la fluoresceine et la rhodamine.

41. Procédé selon la revendication 32, dans lequel ledit échantillon, inconnu d'ADN ou ARN dérivé d'une lésion génitale est obtenu par Biopsie d'une lésion épithéliale, en grattant le cerveau ou en frottant le cerveau pour obtenir des cellules exfoliées, ou l'ADN est dérivé d'une lésion génitale qui a été clonée dans un vecteur clonant.

42. Procédé selon la revendication 32, dans lequel l'hybridation croisée produit des hybrides ADN-ADN.

43. Procédé selon la revendication 32, dans lequel l'hybridation croisée produit des hybrides ADN-ARN.

FIG.1

CLONE 2A
CONT.

CLONE 2B (4.1Kb)

CLONE 2A (3.75Kb)

PI
SI
P2
PI
BI
P2
E5
PI
PI
BI
PI
PI
H3

FIG.2

OPEN READING FRAMES OF HPV6

E6
E7
E1
E4
E2
E5a
E5b
L2
L1

HPV6

E3
PI
BI
PI
PI

HPV35

PI
BI
P2
PI
PI
BI
PI
PI

MAP
UNITS
0        1        2        3        4        5        6        7        8

KILOBASES

FIG. 3

FIG.4

CLONE 2B

CLONE 2A

REGION OF OVERLAP

Kb

EP 0 294 659 B1

# FIG.5

OPEN READING FRAMES OF HPV6

CLONE 2B
CLONE 2A

OVERLAP

MAP
UNITS   0    1    2    3    4    5    6    7    8

KILOBASES

EP 0 294 659 B1

FIG.6

FIG.7

Pst I
Nco I
Xba I
Eco RI
Pvu II
Hpa I
Ava I

0  1  2  3  4  5  6  7  8

Kb

OPEN READING FRAMES OF HPV6

E6
E7
E4
E2
E5a
L1
E1
E5b
L2

FIG.8

Eco RI   Pst I   Bam HI   Pst I   Pst I

HPV 6

Nco I   Nco I   BamHI   Nco I   Nco I

HPV 44

60

FIG. 9

% HPV POSITIVE LESIONS

NORMAL SQUAMOUS EPITHELIUM — METAPLASTIC SQUAMOUS EPITHELIUM — CIN I — CIN II — CIN III — SQUAMOUS CARCINOMA — ENDOCERVICAL ADENOCARCINOMA

HPV 6 + HPV 11 · HPV 16 · HPV 18 · HPV 31 · HPV 44

FIG.10

FIG.11

FIG. 12